(19) Europäisches Patentamt
European Patent Office
Office européen des brevets

(11) EP 0 710 667 B1

(12) **EUROPÄISCHE PATENTSCHRIFT**

(45) Veröffentlichungstag und Bekanntmachung des Hinweises auf die Patenterteilung:
**14.12.2011 Patentblatt 2011/50**

(51) Int Cl.:
***C07H 21/00*** *(2006.01)* ***C07H 19/14*** *(2006.01)*
***A61K 31/70*** *(2006.01)*

(21) Anmeldenummer: **95117058.8**

(22) Anmeldetag: **30.10.1995**

(54) **Modifizierte Oligonukleotide, deren Herstellung sowie deren Verwendung**

Modified oligonucleotides, their preparation and their use

Oligonucléotides modifiés, leur préparation et leur utilisation

(84) Benannte Vertragsstaaten:
**AT BE CH DE DK ES FR GB GR IE IT LI LU NL PT SE**

(30) Priorität: **04.11.1994 DE 4438918**

(43) Veröffentlichungstag der Anmeldung:
**08.05.1996 Patentblatt 1996/19**

(73) Patentinhaber: **Roche Diagnostics GmbH**
**82377 Penzberg (DE)**

(72) Erfinder:
- **Seela, Frank Prof. Dr.**
  **D-49076 Osnabrück (DE)**
- **Thomas, Horst, Dr.**
  **D-27801 Dötlingen/Neerstedt (DE)**

(74) Vertreter: **Hildebrandt, Martin K. E.**
**Roche Diagnostics GmbH**
**Patent Department (TR-E)**
**P.O.Box 11 52**
**82372 Penzberg (DE)**

(56) Entgegenhaltungen:
**EP-A- 0 251 786 EP-A- 0 252 683**
**EP-A- 0 286 028 WO-A-92/06219**
**WO-A-93/09127 WO-A-93/12130**
**WO-A-94/22892 WO-A-94/24144**
**WO-A-95/04747 DE-A- 3 529 478**

- **BIOORG. MED. CHEM. LETT., Bd. 4, Nr. 8, 1994, Seiten 971-6, XP002021199 F. SEELA: "3-Deaza and 7-Deazapurines: Duplex Stability of Oligonucleotides Containing Modified Adenine and Guanine Bases"**
- **NUCLEIC ACIDS SYMP. SER., 9.November 1994, Seiten 151-2, XP002021200 F. SEELA: "Oligonucleotides with Unnatural Bases or an Altered Sugar-Phosphate Backbone"**
- **NUCLEIC ACIDS RES., Bd. 20, 1992, Seiten 4831-7, XP002021270 K.J. LIVAK EZ AL.: "Detection of Single Base Differences Using Biotinylated Nucleotides With Very Long Linker Arms"**
- **CHEMICAL ABSTRACTS, vol. 98, no. 21, 1983 Columbus, Ohio, US; abstract no. 175428, B.G. HUGHES ET AL.: "2',5'-Oligoadenylates and Related 2',5'-Oligonucleotide Analogs" Seite 322; XP002021271 & BIOCHEMISTRY, Bd. 22, 1983, Seiten 2116-26,**
- **CHEMICAL ABSTRACTS, vol. 123, no. 23, 1995 Columbus, Ohio, US; abstract no. 314367, F. SEELA ET AL.: "Duplex Stabilization of DNA" Seite 946; XP002021272 & HELV. CHIM. ACTA, Bd. 78, Nr. 1, 1995, Seiten 94-108,**
- **J. MED. CHEM., Bd. 28, 1985, Seiten 1461-7, XP000655163 H.B. COTTAM ET AL.: "Synthesis and Biological Activity of Certain 6-Substituted and 2,6-Disubstituted 2'-Deoxytubercidins"**
- **NUCLEOSIDES, NUCLEOTIDES, Bd. 8, 1989, Seiten 587-97, XP000655157 F. SEELA ET AL.: "Assignement of Anomeric Configuration of D-Ribo-, Arabino-, 2'-Deoxyribo-, and 2',3'-Dideoxyribonucleosides by NOE Difference Spectroscopy"**
- **HELV. CHIM. ACTA, Bd. 77, Nr. 4, 1994, Seiten 897-903, XP000655155 F. SEELA UND H. THOMAS: "84. Synthesis of Certain 5-Substituted 2'-Deoxytubercidin Derivatives"**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **J. HETEROCYCLIC CHEM., Bd. 27, 1990, Seiten 2069-75, XP000655185 K. EGER AT AL.: "Synthesis of Pyrrolo(2,3-d)pyrimidine Ribosides and thei Potential in Chemotherapeutics"**
- **HELV. CHIM. ACTA, Bd. 78, 1995, Seiten 1083-90, XP000655172 F. SEELA ET AL.: "88. 7-Substituted 7-Deaza-2'-deoxyguanosines"**
- **SCIENCE, Bd. 238, 1987, Seiten 336-341, XP000604017 J.M. PROBER ET AL.: "A System for Rapid DNA Sequencing with Fluorescent Chain-Terminating Dideoxynucleotides"**
- **PATENT ABSTRACTS OF JAPAN vol. 013, no. 047 & JP 63 241356 A (SEKISUI CHEM CO LTD), 6.Oktober 1988,**
- **LIEB. ANN. CHEM., 1984, Seiten 708-21, XP000652359 F. SEELA UND H. WINKLER: "Synthese und Furanosid/ Pyranosid-Isomerisierung von 7-Desaza-2'-desoxy-7-methylguanosin"**


Bemerkungen:

Die Akte enthält technische Angaben, die nach dem Eingang der Anmeldung eingereicht wurden und die nicht in dieser Patentschrift enthalten sind.

## Beschreibung

Die vorliegende Erfindung betrifft neue, modifizierte Basen enthaltende Oligonucleotide mit wertvollen physikalischen, biologischen und pharmakologischen Eigenschaften , ein Verfahren zu deren Herstellung sowie deren Verwendung als Inhibitoren der Genexpression (Antisense Oligonukleotide, Ribozyme, Sense Oligonukleotide und Triplex Forming Oligonukleotide), als Sonden zum Nachweis von Nucleinsäuren, als Hilfsmittel in der Molekularbiologie und als Arzneimittel oder Diagnostikum.

Aus der Literatur sind zahlreiche chemische Modifikationen von Oligonukleotiden bekannt. Diese Modifikation können das Zucker-Phosphat-Gerüst oder die Nucleobasen betreffen. Eine Übersicht über den Stand der Technik geben beispielsweise Uhlmann & Peyman, Chem. Rev. 1990, 90, 543 und Milligan et al., J. Med. Chem. 1993, 36, 1923].

Eine chemische Modifikation von Oligonukleotiden ist in der Regel erforderlich, da unmodifizierte Oligonukleotide sehr rasch durch nukleolytische Aktivitäten sowohl in Zellen als auch im Zellkulturmedium abgebaut werden. Die Stabilisierung gegen nucleolytischen Abbau kann durch Ersatz des Zucker-Phosphat-Rückgrats oder durch Modifikation der Phosphatbrücke, des Zuckerbausteins oder der Nucleobase erfolgen [ Milligan et al., supra und Uhlmann & Peyman, supra].

Neben Modifikationen, die zu Oligonukleotiden mit einer erhöhten Stabilität gegenüber einem nukleolytischen Abbau führen, sind Modifikationen von Interesse, die das Hybridisierungsverhalten der modifizierten Oligonukleotide dahingehend verändern, daß diese z.B. stabilere Hybridisierungskomplexe (Duplexe) mit intrazellulären Nukleinsäuren (sogenannte Target-Nukleinsäuren) bilden können. Eine Veränderung der Hybridisierungseigenschaften von Oligonukleotiden ist z.B. durch Modifikation der Basen möglich. Die veränderten Hybridisierungseigenschaften solch modifizierter Oligonukleotide sind z.B. an der - im Vergleich zu den unmodifizierten Oligonukleotiden - veränderten Schmelztemperatur ($T_m$-Wert) der Duplexe zu erkennen.

So bilden Oligonukleotide, die z.B. 5-Bromuracil enthalten, stabilere Hybridisierungskomplexe mit den komplementären Nucleinsäuren als Oligonukleotide, die die entsprechenden unsubstituierten Basen (Uracil) enthalten [G.D. Fasman, CRC Handbook of Biochemistry and Molecular Biology, 3rd edition, 1975, Nucleic Acids, Vol. 1, 58-585]. Aus der PCT-Anmeldung WO 93/10820 sind ferner Oligonukleotide bekannt, die modifizierte Uracil- bzw. Cytosinbasen enthalten und im Vergleich zu den nicht modifizierten Oligonukleotiden stabilere Duplex- oder Triplexstrukturen mit den Targetnukleinsäuren ausbilden können. Verbesserte Hybridisierungseigenschaften wurden auch für Oligonukleotide beschrieben, die das Basenanalogon 2-Aminoadenin enthalten [Chollet et al, (1988), Nucleic Acid Research, 16, 305-317]. Aus der Deutschen Patentanmeldung P4415370.8 ist bekannt, daß der Einbau von 8-Azapurinbasen in Oligonukleotide die Stabilität der entsprechenden Hybridisierungskomplexe mit den Targetnukleinsäuren erhöht. Aus WO 93/09127 sind ferner Oligonukleotide bekannt, die substituierte oder unsubstituierte 7-Desazapurinbasen enthalten und die dadurch leichter Triplexstrukturen mit den Targetmolekülen (Doppelstrang-DNA) bilden können. Oligonukleotide, die an der 7-Position substituierte 7-Desazapurinbasen enthalten, sind ferner aus WO 94/22892 und WO 94/24144 bekannt.

Allerdings läßt sich nicht vorhersagen, welche Basenmodifikationen zu einer Erhöhung der Duplexstabilität führen. So sind auch zahlreiche Beispiele von Basenmodifikationen bekannt, die die Duplexstabilität verringeren. So werden in der PCT-Anmeldung WO 92/002258 pyrimidin-modifizierte Oligonukleotide beschrieben, die eine verminderte Bindungsaffinität zu der Targetnukleinsäuren aufweisen. Auch veringern Methyl- oder Brom-Substituenten, die an die 8-Position des Purinringes eingeführt werden, die Stabilität der entprechenden Duplexe [E. N. Kanaya et al, Biochemistry (1987) 26 7159, und Biochemistry, 1984, 23, 4219]. 7-Desazaadenin enthaltende Oligonukleotide bilden deutlich weniger stabile Duplexe mit komplementären Oligonukleotiden als Adenin enthaltenden Oligonukleotide [Seela et al., Nucleic Acid Research (1982) 10, 1389].

Aufgabe der vorliegenden Erfindung ist es daher, neue Oligonukleotide mit vorteilhaften Eigenschaften zur Verfügung zu stellen.

Es wurde nun überraschend gefunden, daß Oligonukleotide, die mindestens eine substituierte 7-Desazapurinbase aufweisen, deutlich stabilere Hybridisierungskomplexe mit den Targetnukleinsäuren bilden als vergleichbare Oligonukleotide, die unsubstituierte 7-Desazapurinbasen aufweisen.

Die Erfindung betrifft somit Oligonukleotide der Formel I

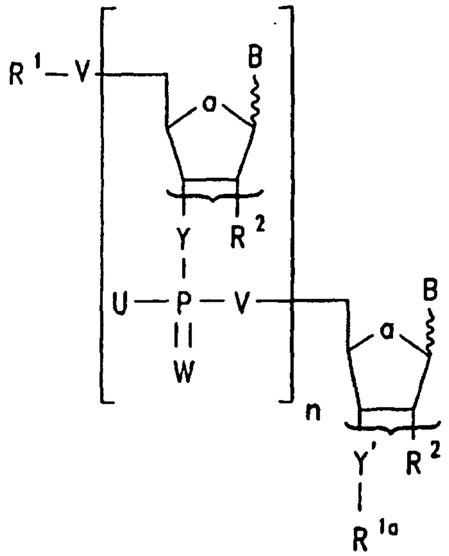

( I )

sowie deren physiologisch verträglichen Salze worin

B unabhängig voneinander eine in der Nukleotidchemie übliche Base bedeuten und mindestens ein B eine Base der Formel II bedeutet

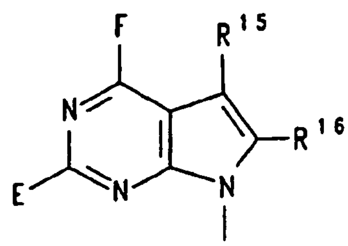

( I I )

worin

$R^{16}$ Wasserstoff und $R^{15}$ $(C_2\text{-}C_{10})$-Alkinyl E=H oder $NH_2$ und F = $NH_z$ oder OH bedeutet, wobei wenn E=H ist, F=$NH_2$ bedeutet und wenn E=$NH_2$ ist, F=OH bedeutet

$R^1$ Wasserstoff einen Rest der Formel IIIa

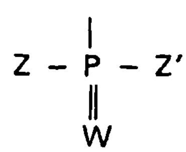

(IIIa)

bedeutet;

$R^{1a}$ Wasserstoff, oder einen Rest der Formel IIIb

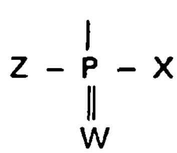

(IIIb)

bedeutet;

| | |
|---|---|
| $R^2$ | Wasserstoff, Hydroxy, bedeutet; |
| a | für Oxy steht; |
| n | eine ganze Zahl ≥ 1 bedeutet; |
| W | Oxo, bedeutet; |
| V | Oxy, bedeutet; |
| Y | Oxy, bedeutet; |
| Y' | Oxy, Sulfandiyl, ist,; |
| X | Hydroxy bedeutet; |
| U | Hydroxy, bedeutet, |
| Z und Z' | sind. Hydroxy, |

die geschweifte Klammer andeutet, daß sich $R^2$ und der benachbarte Phosphoryl-Rest bzw. -Y'-$R^{10}$-Rest in 2'- und 3'-Stellung oder auch umgekehrt in 3'- und 2'-Stellung befinden können,
wobei jedes Nucleotid in seiner D- bzw. L-Konfiguration vorliegen kann und sich die Base B in α- bzw. β-Stellung befinden kann.

Unter in der Nukleotidchemie übliche Basen sind beispielsweise natürliche Basen wie Adenin, Cytosin, Thymin, Guanin, Uracil, Hypoxanthin oder unnatürliche Basen wie beispielsweise Purin, 8-Azapurin, 2.6-Diaminopurin, 7-Deazaadenin, 7-Deazaguanin, $N^4$,$N^4$-Ethanocytosin, $N^6N^6$-Ethano-2,6-diaminopurin, Pseudoisocytosin, 5-Methylcytosin, 5-Fluoruracil, 5-($C_3$-$C_6$)-Alkinyluracil, 5-($C_3$-$C_6$)-Alkinylcytosin oder deren Prodrugformen zu verstehen.

Besonders bevorzugt sind Oligonukleotide der Formel 1, die mindestens eine an der 7-Position substituierte 7-Desazaadeninbase und gegebenfalls zusätzlich eine oder mehrere an der 7-Position substituierte 7-Desazaguanin-Basen aufweisen.

Bevorzugt sind auch Oligonukleotide der Formel I, bei denen sich die Base am Zucker in β-Stellung befindet, die Nucleotide in der D-Konfiguration vorliegen, $R^2$ sich in 2'-Stellung befindet.

Unter der 7-Position des Azapurinringsystems ist die Position zu verstehen, an der der Substituent $R^{15}$ sitzt. An der 8-Position sitzt dementsprechend der Substituent $R^{16}$.

Die erfindungsgemäßen Oligonukleotide weisen im Vergleich zu den natürlichen Oligonukleotiden bei der Anlagerung an komplementäre Nukleinsäuren (Targetnukleinsäuren) eine verbesserte Bindungsaffinität auf.

Ein weiterer Vorteil der erfindungsgemäßen Oligonukleotide ist deren erhöhte Säure- Und Nucleasestabilität in Vergleich zu Oligonukleotiden, die natürliche Purinbasen enthalten.

Für die therapeutische Nutzung dieser Oligonukleotide ist es vorteilhaft, wenn in diese zusätzliche Modifikationen, beispielsweise des Phosphatrückgrats, der Ribose-Einheit oder der Oligonukleotid-Enden eingeführt werden [J.S. Cohen, Topics in Molecular and Structural Biology 12 (1989) Macmillan Press, E. Uhlmann et al., supra].
Durch ansich bekannte Modifikationen des Zucker-Phosphat-Rückrats werden die erfindungsgemäßen Oligonukleotide beispielsweise noch besser gegen den Angriff von Nukleasen geschützt, was vorteilhaft ist.

Bevorzugt sind daher auch Verbindungen der Formel 1, worin V, Y , Y' und W die Bedeutung von Thioxo, Selenoxo, Oxy, Oxo, Sulfandiyl, Imino oder Methylen haben und U die Bedeutung von Hydroxy, Mercapto oder Methyl hat. Ganz besonders bevorzugt sind diese , falls $R^2$ zusätzlich Hydroxy oder Wasserstoff, insbesondere Wasserstoff bedeutet.

Unter den in der Nukleotidchemie üblichen Schutzgruppen sind beispielsweise Amino-, Hydroxyl- oder andere Schutzgruppen zu verstehen, wie sie in [E.Sonveaux, 1986. Bioorganic Chemistry, 14, 274-325 oder S.L. Beaucage et al., 1992, Tetrahedron, 48, 2223-2311] beschrieben werden.

Alkyl, Alkenyl und Alkinyl können geradkettig oder verzweigt sein. Entsprechendes gilt auch für davon abgeleitete Reste, wie Alkanoyl oder Alkoxy.

Als ($C_1$-$C_{10}$)-Alkyl gelten insbesondere Methyl, Ethyl, Propyl, Isopropyl, Butyl, Isobutyl, Pentyl, Hexyl, und Heptyl. Beispiele für halogenierte ($C_1$-$C_{10}$)-Alkyle sind $CHF_2$, $CF_3$, $CH_2F$, $CF_3$-$CH_2$-$CH_2$-, $CF_3$-$CF_2$-$CH_2$, $CF_3(CF_2)_6$-$CH_2$,

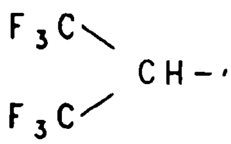, H-$(CF_2)_6$-$CH_2$-, $CF_3(CF_2)_7$-$CH_2$-$CH_2$-.

($C_2$-$C_{10}$)-Alkenyl ist beispielsweise Vinyl (-CH=$CH_2$), 1-Propenyl (-CH=CH-$CH_3$), 2-Methyl-1-propenyl (-CH=C($CH_3$)-$CH_3$), 1-Butenyl (-CH = CH-$CH_2$-$CH_3$), 1-Pentenyl, 1-Hexenyl, 1-Heptenyl, 1-Octenyl. Beispiele für halogenierte ($C_2$-$C_{10}$)-Alkenyle sind -CH=$CF_2$, -CH=CH-$CF_3$, -CF=CF-$CF_3$.

($C_2$-$C_{10}$)-Alkinyl ist beispielsweise Ethinyl (-C≡CH), 1-Propinyl (-C≡C-$CH_3$), 1-Butinyl (-C≡C-$CH_2$-$CH_3$), 3-Methyl-butinyl (-C≡C-CH($CH_3$)-$CH_3$), 3,3-Dimethyl-butinyl (-C≡C-C($CH_3$)$_3$), 1-Pentinyl, 1,3-Pentadiniyl (-C≡C-C≡C-$CH_3$), 1-Hexinyl, 1-Heptinyl. Beispiele für halogenierte ($C_2$-$C_{10}$)-Alkinyle sind -C≡C-$CH_2$F, -C≡C-$CF_3$, -C≡C-($CH_2$)$_3$-$CF_3$, -C≡C-($CF_2$)$_3$-$CF_3$,

Unter Cycloalkyl werden auch Alkyl-substituierte Ringe verstanden.

($C_6$-$C_{20}$)-Aryl ist beispielsweise Phenyl, Naphthyl oder Biphenyl, vorzugsweise Phenyl.

Unter Halogen ist Jod, Brom, Chlor oder Fluor zu verstehen.

Unter Heteroaryl werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliederigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Beispielhaft für Gruppen $NR^3R^4$, in denen $R^3$ und $R^4$ zusammen mit dem sie tragenden Stickstoffatom einen 5- bis 6-gliedrigen heterocyclischen Ring bilden, der zusätzlich ein weiteres Heteroatom enthält, seien der Morpholinyl- und der Imidazolidinyl-Rest genannt.

Unter physiologisch verträglichen Salzen von Verbindungen der Formel (I) versteht man sowohl anorganische als auch organische Salze, wie sie in Remington's Pharmaceutical Sciences (17. Auflage, Seite 1418 (1985)) beschrieben sind.

Aufgrund der physikalischen und chemischen Stabilität und der Löslichkeit sind für saure Gruppen unter anderem Natrium-, Kalium-, Calcium- und Ammoniumsalze bevorzugt.

Die offenbarung ist nicht auf α- und β-D- bzw. L-Ribofuranoside, α- und β-D- bzw. L- Desoxyribofuranoside und entsprechende carbocyclische Fünfringanaloga beschränkt, sondern gilt auch für Oligonucleotidanaloga, die aus anderen Zucker-Bausteinen aufgebaut sind, beispielsweise Xylo- und Arabinofuranose Derivate, ringerweiterte und ringverengte Zucker, acyclische, ringverbrückte oder geeignete andersartige Zucker-Derivate. Die Erfindung ist ferner nicht auf die in Formel I beispielhaft aufgeführten Derivate des Phosphat-Restes beschränkt, sondern bezieht sich auch auf die bekannten Dephospho-Derivate.

Die offenbarten Oligonukleotide können also in vielfältiger Weise von der natürlichen Struktur abgewandelt sein. Solche Modifikationen, die nach an sich bekannten Methoden eingeführt werden, sind beispielsweise:

a) Modifikationen der Phosphatbrücke
Beispielhaft seien genannt: Phosphorothioate, Phosphorodithioate, Methylphosphonate, Phosphoramidate, Boranophosphate, Phosphatmethylester, Phosphatethylester, Phenylphosphonate. Bevorzugte Modifikationen der Phosphatbrücke sind Phosphorothioate, Phosphorodithioate und Methylphosphonate.

b) Ersatz der Phosphatbrücke
Beispielhaft seien genannt: Ersatz durch Acetamid, Formacetal, 3'-Thioformacetal, Methylhydroxylamin, Oxim, Methylendimethylhydrazo, Dimethylensulfon, Silylgruppen. Bevorzugt ist der Ersatz durch Acetamid, Formacetale und 3'-Thioformacetale.

c) Modifikationen des Zuckers
Beispielhaft seien genannt: α-anomere Zucker, 2'-O-Methylribose, 2'-O-Butylribose, 2'-O-Allylribose, 2'-Fluoro-2'-desoxyribose, 2'-Amino-2'-desoxyribose, α-Arabinofuranose, Carbocyclische Zuckeranaloge. Bevorzugte Modifikation ist die durch 2'-O-Methylribose und 2'-O-n-Butylribose. Ganz besonders bevorzugt sind solche Modifikationen, bei denen die 2'- und 3'-Kohlenstoffatome der O-Ribose über eine Doppelbindung verknüpft sind und jeweils ein Wasserstoffatom als Substituenten tragen.

d) Modifikationen des Zuckers und der Phosphat-Brücke
Beispielhaft seien genannt die Peptid-Nukleinsäuren (PNA's), bei denen das Zucker/Phosphat-Rückgrat durch ein Aminoethylglycin-Rückgrat ersetzt ist, (s. Deutsche Patentanmeldung P4408531.1) sowie die carbamatverbrückten Morpholino-Oligomeren. Die PNA's können auch mit Nukleinsäuren verknüpft sein, wie in der Deutschen Patentanmeldung P4408528.1 beschrieben.

e) Andere Modifikationen der Basen, insbesondere der Pyrimidinbasen Beispielhaft seien genannt: 5-Propinyl-2'-desoxyuridin, 5-Propinyl-2'-desoxycytidin, 5-Hexinyl-2'-desoxyuridin, 5-Hexinyl-2'-desoxycytidin, 5-Fluoro-2'-desoxycytidin, 5-Fluor-2'-desoxyuridin, 5-Hydroxymethyl-2'-desoxyuridin, 5-Methyl-2'-desoxycytidin, 5-Brom-2'-desoxycytidin. Bevorzugte Modifikationen sind 5-Propinyl-2'-desoxyuridin, 5-Hexinyl-2'-desoxyuridin, 5-Hexinyl-2'-desoxycytidin und 5-Propinyl-2'-desoxycytidin.

f) 3'-3'- und 5'-5'-Inversionen [z.B. M. Koga et al., J. Org. Chem. 56 (1991) 3757]

g) 5'- und 3'-Konjugate.
Beispielhaft für Gruppen, die die intrazelluläre Aufnahme begünstigen, sind verschiedene lipophile Reste wie $-O-(CH_2)_x CH_3$, worin x eine ganze Zahl von 6 bis 18 bedeutet, $-O-(CH_2)_n-CH = CH-(CH_2)_m-CH_3$, worin n und m unabhängig voneinander eine ganze Zahl von 6 bis 12 bedeuten, $-O-(CH_2CH_2O)_4-(CH_2)_9-CH_3$, $-O-(CH_2CH_2O)_8-(CH_2)_{13}-CH_3$ und $-O-(CH_2CH_2O)_7-(CH_2)_{15}-CH_3$, aber auch Steroid-Reste wie Cholesteryl oder Vitamin-Reste wie Vitamin E, Vitamin A oder Vitamin D und andere Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor). Unter Markierungs-Gruppen sind fluoreszierende Gruppen beispielsweise von Dansyl-( = N-Dimethyl-1-aminonaphthyl-5-sulfonyl-), Fluorescein- oder Coumarin-Derivaten oder chemilumineszierende Gruppen beispielsweise von Acridin-Derivaten zu verstehen sowie das über ELISA nachweisbare Digoxygenin-System, die über das Biotin/Avidin-System nachweisbare Biotin-Gruppe oder aber Linker-Arme mit funktionellen Gruppen, die eine nachträgliche Derivatisierung mit nachweisbaren Reporter-Gruppen gestatten, beispielsweise ein Aminoalkyl-Linker, der mit einem Acridinium-Aktivester zur Chemilumineszenz-Probe umgesetzt wird. Dem Fachmann sind weitere geeignete Linker aus den veröffentlichten Patentanmeldungen EP 251786 und WO 93/09217 bekannt.

h) Konjugation über 7- und/oder 8-Position am 7-Desazapurin Über die 7- und/oder 8-Position des 7-Desazapurins können ebenfalls Gruppen konjugiert sein, die als Markierung einer DNA- oder RNA-Sonde dienen oder die intrazelluläre Aufnahme begünstigen. 7-Desazapurinnucleoside, an die über die 7-Stellung des 7-Desazapurins Biotin- bzw. Iminobiotinreste über einen spezielle Verbindungsgruppe konjugiert sind, sind aus EP 63 879 bekannt.

Unter Markierungs-Gruppen für eine DNA- oder RNA-Sonde sind fluoreszierende Gruppen beispielsweise von Dansyl- (=N-Dimethyl-1-aminonaphthyl-5-sulfonyl-), Fluorescein- oder Coumarin-Derivaten oder chemilumineszierende Gruppen beispielsweise von Acridin-Derivaten zu verstehen sowie das über ELISA nachweisbare Digoxygenin-System oder die über das Biotin/Avidin-System nachweisbare Biotin-Gruppe sowie die unter g) bereits aufgeführte Interkalatoren und chemisch aktive Gruppen (siehe auch Beaucage et al., Tetrah. (1993) Vol 49, No. 10, 1925-1963).
Beispielhaft für Gruppen, die die intrazelluläre Aufnahme begünstigen, sind Steroid-Reste wie Cholesteryl oder Vitamin-Reste wie Vitamin E, Vitamin A oder Vitamin D und andere Konjugate, die natürliche Carriersysteme ausnutzen wie Gallensäure, Folsäure, 2-(N-Alkyl, N-Alkoxy)-Aminoanthrachinon und Konjugate der Mannose und Peptide der entsprechenden Rezeptoren, die zur rezeptorvermittelten Endozytose der Oligonucleotide führen wie EGF (Epidermal Growth Factor), Bradykinin und PDGF (Platelet Derived Growth Factor).

Allgemein können die beschriebenen Gruppen sowohl auf der Ebene der Oligonukleotide (beispielsweise über SH-Gruppen) als auch auf der Ebene der Monomeren (Phosphonate, Phosphoamidite oder Triphosphate) eingeführt werden. Bei den Monomeren, insbesondere bei den Triphosphaten, ist es vorteilhaft, die Seitenketten, an die eine Reporter- oder Interkalatorgruppe eingeführt werden soll, geschützt zu lassen, und erst nach der Phosphorylierung die Seitenkettenschutzgruppen abzuspalten und mit einem gegebenenfalls aktivierten Derivat der entsprechenden Reporter- oder Interkalatorgruppe umzusetzen.

Typische Markierungsgruppen sind:

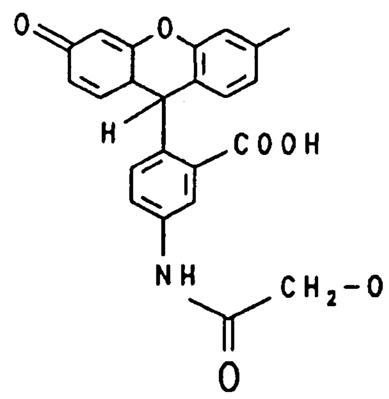

Fluorescein-Derivat

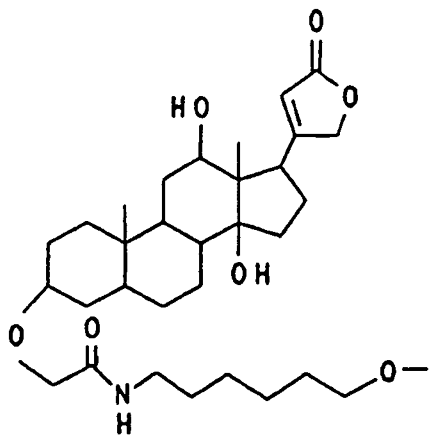

Digoxgeninkonjugat

Oligonucleotidanaloga, die an Nucleinsäuren binden bzw. interkalieren und/oder spalten oder quervernetzen, enthalten z.B. Acridin-, Psoralen-, Phenanthridin, Naphtochinon-, Daunomycin- oder Chlorethylaminoaryl-Konjugate. Typische interkalierende und quervernetzende Reste sind:

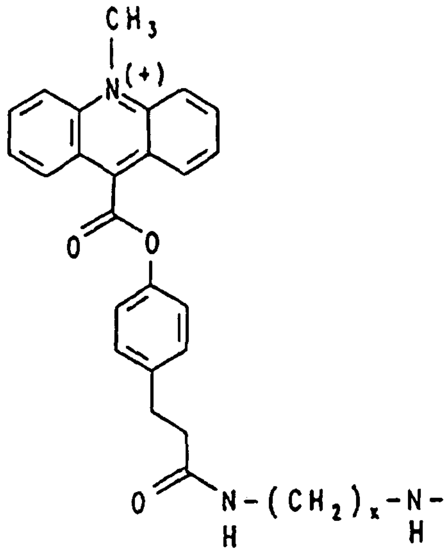

Acridinium-Ester

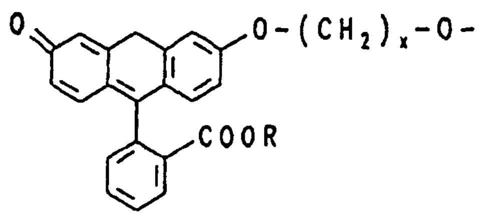

x = 2-18 bevorzugt 4

R = H oder $C_1$-$C_4$-Alkyl

(= "Fluorescein" fuer x = 4 und R = $CH_3$)

- Fluorescein-Derivat

R = H oder Aminoschutzgruppe

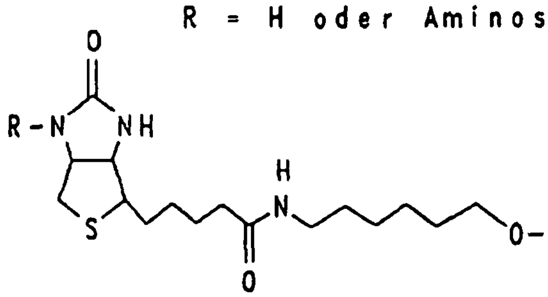

Biotinkonjugat (= "Biotin" fuer R = Fmoc)

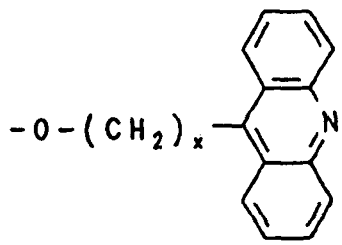

Acridinderivat x = 2-12, bevorzugt 4

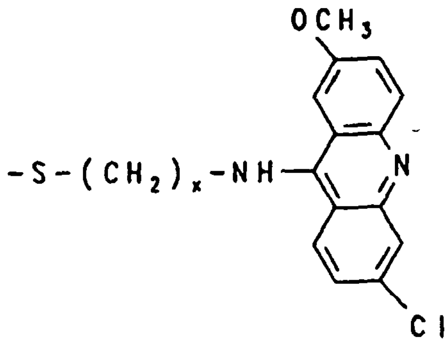

x = 2-12, bevorzugt 4

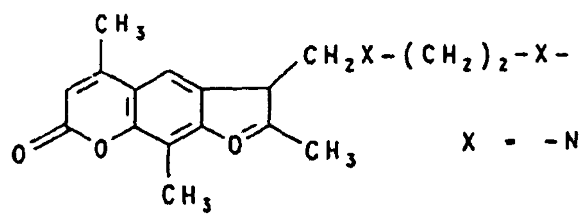

X = -NH oder -O-

Trimethylpsoralen-konjugat (= "Psoralen" fuer X = O)

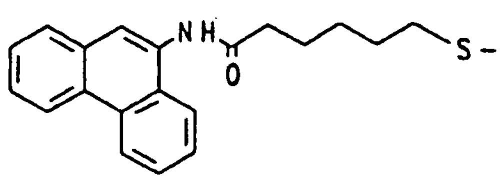

Phenanthrolinkonjugat

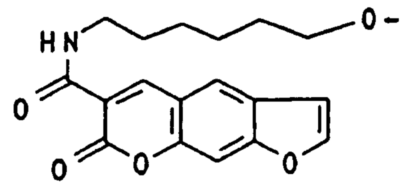

Psoralenkonjugat

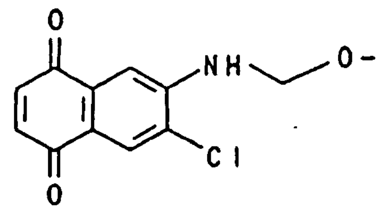

Naphthochinonkonjugat

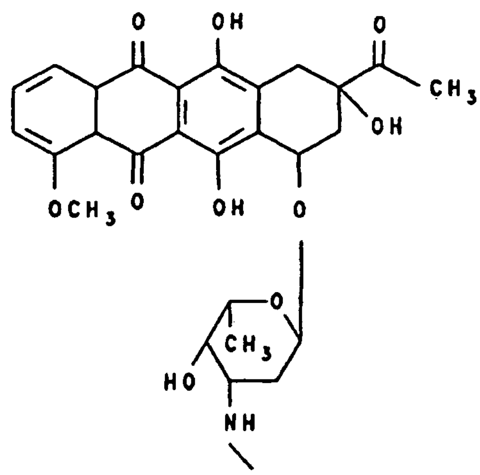

Daunomycinderivat

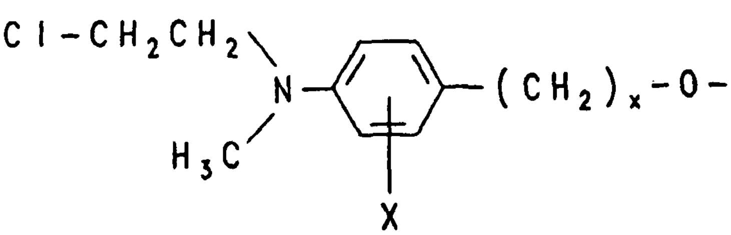

x - 1-18, X - Alkyl, Halogen. $NO_2$, CN,

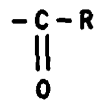

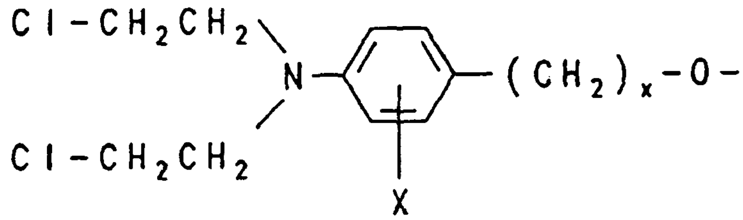

x = 1-18, X - Alkyl, Halogen, $NO_2$, CN,

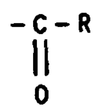

Gegenstand der offenbarung ist weiterhin die Verbindung der Formel V

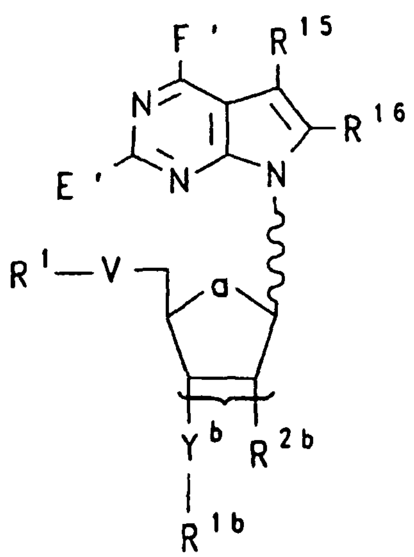

(V)

worin

| | |
|---|---|
| V | Oxy, Sulfandiyl oder Imino ist; |
| $Y^b$ | Oxy, Sulfandiyl, Imino oder Methylen ist; |
| a | für Oxy, Sulfanidyl oder Methylen steht; |
| $R^{2b}$ | Wasserstoff, $OR^{12}$, $C_1$-$C_{18}$-Alkoxy, $C_1$-$C_6$-Alkenyloxy, insbesondere Allyloxy, Halogen, Azido oder $NR^{10}R^{11}$ bedeutet; |

$R^1$ eine in der Nukleotidchemie übliche Schutzgruppe bedeutet;

$R^{1b}$ ein Succinylrest ist, der über eine Amid- oder Methylimidbindung mit einem amino- oder methylaminofunktionalisierten Träger verknüpft ist, oder einen Rest der Formel IIIc oder IIId bedeutet

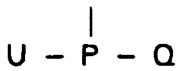 (IIIc) 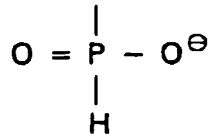 (IIId)

worin

U $(C_1\text{-}C_{18})$-Alkoxy, $(C_1\text{-}C_{18})$-Alkyl, $(C_6\text{-}C_{20})$-Aryl, $(C_6\text{-}C_{14})$-Aryl-$(C_1\text{-}C_8)$- Alkyl,O-$R^7$, S-$R^7$ oder einen Rest der Formel IV

$$(OCH_2CH_2)_pO(CH_2)_qCH_2R^5 \qquad (IV)$$

worin $R^5$ gleich H ist, bedeutet;

Q einen Rest $-NR^8R^9$ bedeutet

$R^7$ $-(CH_2)_2$-CN bedeutet;

$R^8$ und $R^9$ gleich oder verschieden sind und $C_1$-$C_6$-Alkyl, insbesondere Isopropyl oder Ethyl bedeuten oder zusammen mit dem sie tragenden Stickstoffatom einen 5-9-gliedrigen heterozyklischen Ring bedeuten, der zusätzlich ein weiteres Heteroatom aus der Reihe O, S, N enthalten kann, insbesondere

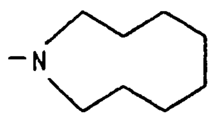 , 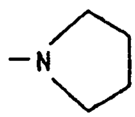 oder 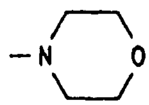 ;

E' und F' unabhängig voneinander H, $OR^{12}$, $NR^{10}R^{11}$ bedeuten,

$R^{10}$ und $R^{11}$ gleich oder verschieden sind und Wasserstoff oder eine in der Nukleotidchemie übliche Aminoschutzgruppe bedeuten oder $R^{10}$ und $R^{11}$ gemeinsam eine in der Nukleotidchemie übliche Aminoschutzgruppe bilden,

$R^{12}$ Wasserstoff oder eine in der Nukleotidchemie übliche Hydroxylschutzgruppe, wie beispielsweise t-Butyldimethyl-silyl, Dimethoxytriphenylmethyl (DMT), Triisopropyl-silyl, o-nitro-Benzyl, p-nitro- Benzyl, iBu, 2-Fluorphenyl-4-methoxypiperidin-4-yl (FPMP), oder Methyl bedeutet,

$R^{15}$ und $R^{16}$ unabhängig voneinander

1. Wasserstoff
2. Halogen,
3. $(C_1\text{-}C_{10})$-Alkyl,
4. $(C_2\text{-}C_{10})$-Alkenyl,
5. $(C_2\text{-}C_{10})$-Alkinyl,
6. $NO_2$,
7. $NH_2$,
8. Cyano,
9. -S-$(C_1\text{-}C_6)$-Alkyl,
10. $(C_1\text{-}C_6)$-Alkoxy
11. $(C_6\text{-}C_{20})$-Aryloxy
12. $SiH_3$,
13.

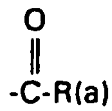

14. einen wie unter 3., 4., oder 5. definierten Rest, der mit einem oder mehreren Resten aus der Gruppe SH, S-($C_1$-$C_6$)-Alkyl, ($C_1$-$C_6$)-Alkoxy, OH, -NR(c)R(d), -CO-R(b), -NH-CO-NR(c)R(d), -NR(c)R(g), -NR (e)R(f), -NR(e)R(g) oder mit einem Polyalkylenglykol-Rest der Formel -[O-$(CH_2)_r]_s$-NR(c)R(d), wobei r und s unabhängig voneinander eine ganze Zahl zwischen 1-18, bevorzugt 1-6, bedeuten, substituiert ist wobei funktionelle Gruppen wie OH, SH, -CO-R(b), -NH-CO-NR(c)R(d), -NR(c)R(d), -NR(e)R(f), -NR(e)R(g) oder -NR(c)R(g) eine in der Nukleotidchemie übliche Schutzgruppe tragen oder mit einer oder mehreren Gruppen, gegebenenfalls über einen weiteren Linker, verknüpft sein können, die die intrazelluläre Aufnahme begünstigen oder als Markierung einer DNA-oder RNA-Sonde dienen oder bei der Hybridisierung des Oligonucleotidanalogons an die Target-Nucleinsäure diese unter Bindung, Quervernetzung oder Spaltung angreifen, oder

15. einen wie unter 3., 4. oder 5. definierten Rest, worin ein bis alle H-Atome durch Halogen, vorzugsweise Fluor, substituiert sind, bedeutet.

R(a) OH, ($C_1$-$C_6$)-Alkoxy, ($C_6$-$C_{20}$)-Aryloxy, $NH_2$ oder NH-T bedeutet, wobei T eine Alkylcarboxy- oder Alkylaminogruppe darstellt, die mit einer oder mehreren Gruppen, gegebenenfalls über einen weiteren Linker, verknüpft ist, die die intrazelluläre Aufnahme begünstigen oder als Markierung einer DNA- oder RNA-Sonde dienen oder bei der Hybridisierung des Oligonucleotidanalogons an die Target-Nucleinsäure diese unter Bindung, Quervernetzung oder Spaltung angreifen,

R(b) Hydroxyl, ($C_1$-$C_6$)-Alkoxy, oder -N(R(c)R(d) bedeutet,

R(c) und R(d) unabhängig voneinander H, ($C_1$-$C_6$)-Alkyl unsubstituiert oder substituiert mit -NR(e)R(f) oder -NR(e) R(g) bedeuten,

R(e) und R(f) unabhängig voneinander H oder ($C_1$-$C_6$)-Alkyl bedeuten,

R(g) ($C_1$-$C_6$)-Alkyl-COOH bedeutet

mit der Maßgabe, daß $R^{15}$ und $R^{16}$ nicht jeweils gleichzeitig Wasserstoff, $NO_2$, $NH_2$, Cyano oder $SiH_3$ sein können, wobei funktionelle Gruppen wie OH, $NH_2$ oder COOH gegebenenfalls mit einer in der Nukleotidchemie üblichen Schutzgruppe geschützt sind,

die geschweifte Klammer andeutet, daß sich $R^{2b}$ und der benachbarte $y^b$-$R^{1b}$-Rest in 2'- und 3'-Stellung oder auch umgekehrt in 3'- und 2'-Stellung befinden können,

Offenbart sind weiterhin Verbindungen der Formel (V) dar, in denen V, $Y^b$ und a für Oxy stehen, $R^{2b}$ Wasserstoff oder $OR^{12}$, insbesondere Wasserstoff und $R^{1b}$ einen Rest der Formel (IIIc) oder (IIId) bedeutet, wobei U O-$(CH_2)_2$-CN bedeutet und $R^8$ und $R^9$ gleich oder verschieden sind und Isopropyl oder Ethyl bedeuten, oder zusammen mit dem sie tragenden N-Atom einen aliphatischen Heterocyclus, bevorzugt Pyrrolidino, bilden.

Ganz besonders bevorzugt sind diese, falls sich zusätzlich die Base am Zucker in β-Stellung und $R^{2b}$ in 2'-Stellung befindet.

Bevorzugt sind auch Verbindungen der Formel (V), in denen E gleich $NR^{10}R^{11}$ und F gleich H ist und ganz allgemein solche Verbindungen der Formel (V), die für die Herstellung bevorzugte Oligonukleotide der Formel 1 eingesetzt werden können.

Bevorzugte Aminoschutzgruppen sind beispielsweise Acyl- oder AmidinSchutzgruppen.

Unter dem üblicherweise als Salz vorliegenden Rest der Formel (IIId) sind anorganische oder organische Salze, beispielsweise Alkali-, Erdalkali- oder Ammoniumsalze, die z.B. in Remington's Pharmaceutical Sciences (17. Auflage, Seite 1418 (1985) beschrieben sind, zu verstehen. Beispielshaft seien Triethylammonium- und Pyridiniumsalze genannt.

Die Offenbarung umfaßt aber auch Verbindungen der allgemeinen Formel (V), in denen der Rest der Formel (IIId) als freie Säure vorliegt.

Die Verbindungen der Formel V können als Bausteine für die Herstellung der erfindungsgemäßen Oligonukleotide der Formel I eingesetzt werden.

Aus EP 251 786 sind 7-Desazapurin-Nukleotide, bzw. deren Mono-, Di- oder Triphosphate bekannt, die eine Alkinylaminogruppe an der 7-Purin Position aufweisen. Die Alkinylaminogruppe dient als "Linker", über den fluoreszierende Markermoleküle an das Nukleotid angekoppelt werden können. Die mit einem Fluoreszenzmarker versehenen Didesoxynukleotide können dann als Kettenabbruchmoleküle ("chain terminatoren") für die Didesoxysequenzierung nach Sanger verwendet und direkt fluoreszenspektoskopisch detektiert werden. 7-Desazapurinnukleotide, die einen detektierbaren Rest am 7-Desazapurin tragen, sind aus U.S. 5,241,060 bekannt.

Die Offenbarung betrifft auch Verbindungen der Formel VI

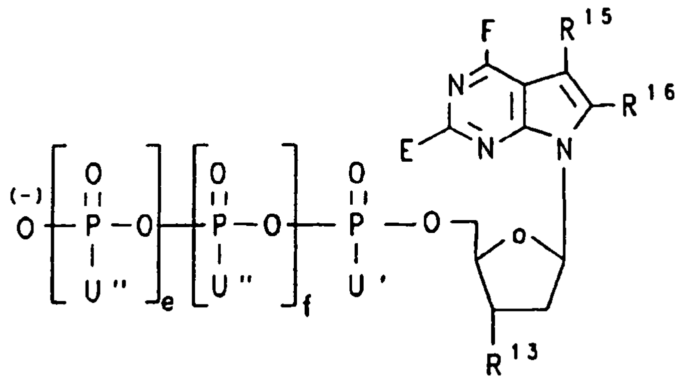

(VI)

worin unabhängig voneinander

| | |
|---|---|
| U' = U'' - U''' | gleich Hydroxyl oder Mercapto ist und U' zusätzlich $BH_3$ sein kann, |
| e und f | 0 oder 1 bedeuten; |
| $R^{13}$ | Wasserstoff, OH, $C_1$-$C_{18}$-Alkoxy, $C_1$-$C_6$-Alkenyloxy, insbesondere Allyloxy, bedeutet; |
| E und F | unabhängig voneinander H, OH oder $NH_2$ bedeuten; und |
| $R^{15}$ und $R^{16}$ | unabhängig voneinander |

1. Wasserstoff
2. Halogen,
3. ($C_1$-$C_{10}$)-Alkyl,
4. ($C_2$-$C_{10}$)-Alkenyl,
5. ($C_2$-$C_{10}$)-Alkinyl,
6. $NO_2$,
7. $NH_2$,
8. Cyano,
9. -S-($C_1$-$C_6$)-Alkyl,
10. ($C_1$-$C_6$)-Alkoxy
11. ($C_6$-$C_{20}$)-Aryloxy
12. $SiH_3$,
13.

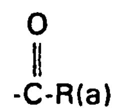

14. einen wie unter 3., 4., oder 5. definierten Rest, der mit einem oder mehreren Resten aus der Gruppe SH, S-($C_1$-$C_6$)-Alkyl, ($C_1$-$C_6$)-Alkoxy, OH, -NR(c)R(d), -CO-R(b), -NH-CO-NR(c)R(d), -NR(c)R(g), -NR(e)R(f), -NR(e)R(g) oder mit einem Polyalkylenglykol-Rest der Formel $-[O-(CH_2)_r]_s-NR(c)R(d)$, wobei r und s unabhängig voneinander eine ganze Zahl zwischen 1-18, bevorzugt 1-6, bedeuten, substituiert ist wobei funktionelle Gruppen wie OH, SH, -CO-R(b), -NH-CO-NR(c)R(d), -NR(c)R(d), -NR(e)R(f), -NR(e)R(g) oder -NR(c)R(g) zusätzlich mit einer oder mehreren Gruppen, gegebenenfalls über einen weiteren Linker, verknüpft sein können, die die intrazelluläre Aufnahme begünstigen oder als Markierung einer DNA-oder RNA-Sonde dienen oder bei der Hybridisierung des Oligonucleotidanalogons an die Target-Nucleinsäure diese unter Bindung, Quervernetzung oder Spaltung angreifen, oder
15. einen wie unter 3.,4. oder 5. definierten Rest, worin ein bis alle H-Atome durch Halogen, vorzugsweise Fluor, substituiert sind, bedeutet.

| | |
|---|---|
| R(a) | OH, ($C_1$-$C_6$)-Alkoxy, ($C_6$-$C_{20}$)-Aryloxy, $NH_2$ oder NH-T bedeutet, wobei T eine Alkylcarboxy- oder Alkylaminogruppe darstellt, die mit einer oder mehreren Gruppen, gegebenenfalls über einen weiteren |

Linker, verknüpft ist, die die intrazelluläre Aufnahme begünstigen oder als Markierung einer DNA- oder RNA-Sonde dienen oder bei der Hybridisierung des Oligonucleotidanalogons an die Target-Nucleinsäure diese unter Bindung, Quervernetzung oder Spaltung angreifen,

| | |
|---|---|
| R(b) | Hydroxyl, ($C_1$-$C_6$)-Alkoxy, oder -N(R(c)R(d) bedeutet, |
| R(c) und R(d) | unabhängig voneinander H, ($C_1$-$C_6$)-Alkyl unsubstituiert oder substituiert mit -NR(e)R(f) oder -NR(e) R(g) bedeuten, |
| R(e) und R(f) | unabhängig voneinander H oder ($C_1$-$C_6$)-Alkyl bedeuten, |
| R(g) | ($C_1$-$C_6$)-Alkyl-COOH bedeutet |

mit der Maßgabe, daß $R^{15}$ und $R^{16}$ nicht jeweils gleichzeitig Wasserstoff, $NO_2$, $NH_2$, Cyano oder $SiH_3$ sein können, wobei Verbindungen der Formel VI ausgenommen sind in denen $R^{16}$ gleich H ist und $R^{15}$ ($C_2$-$C_{10}$)-Alkinyl, substituiert mit -NR(c)R(d) oder -NR(e)R(f) bedeutet; und der weiteren Maßgabe, daß e und f nicht O sind, wenn E = OH oder $NH_2$ und F = OH ist, $R^{16}$ Wasserstoff ist und $R^{15}$ Br, Cl, F, Cyano, ($C_1$-$C_4$)-Alkyl, ($C_2$-$C_4$)-Alkenyl oder ($C_2$-$C_4$)-Alkinyl bedeutet.

Die offenbarung umfaßt auch Verbindungen der allgemeinen Formel VI, die in allgemein üblicher Weise mit einem radioaktiver Markierung versehen sind (z.B. αP-Atom ist gleich $^{32}P$; U'ist gleich $^{35}S$).

Bevorzugt sind Verbindungen der Formel VI in denen U' Hydroxyl oder Mercapto bedeutet, U" = U"' Hydroxyl bedeutet und e und/oder f gleich 1 ist.

Besonders bevorzugt sind die Verbindungen der Formel VI, wenn e und f gleich 1 ist.

Die überlicherweise als Salz vorliegende Verbindungen der Formel VI umfassen anorganische oder organische Salze, beispielsweise Alkali-, Erdalkali- oder Ammoniumsalze [Remington's Pharmaceutical Sciences (17. Auflage, Seite 1418 (1985)]. Beispielshaft seien Triethylammonium und Pyridinumsalze genannt.
Die Verbindung VI umfassen auch solche Verbindungen, in denen die Phosphatgruppe als freie Säure vorliegt.

Die Verbindungen der Formel VI können allgemein als Hilfsmittel in der Molekularbiologie eingesetzt werden, beispielsweise in PCR-Reaktionen (e = f =1, $R^{13}$ = OH) oder zum Sequenzieren (e = f = 1; $R^{13}$ = H oder OH). Verbindungen der Formel VI sind besonders geeignet in PCR-Reaktionen, wenn $R^{16}$ = H und $R^{15}$ = Halogen ist.

Die Verwendung der erfindungsgemäßen 7-Desazapurinnucleotide für die Sequenzierung von Nukleinsäuren ist aus mehreren Gründen vorteilhaft.
So wird bei der Sequenzierungsmethode nach Sanger (Didesoxytechnik) die für GC-reiche Nukleotidbereiche oft zu beobachtende Kompression der Banden, die eine korrekte Bestimmung der Nukleotidsequenz erschwert, eliminiert oder zumindest verringert. Zusätzlich werden die während der Sequenzierung durch DNA- bzw. RNA-Polymerasen synthetisierten doppelsträngigen Nucleinsäuren durch den Einbau 7-, 8- oder 7,8-substituierter 7-Desazapurinbasen stabilisiert. Die Verwendung substituierter 7-Desazapurinnukleotide ist somit gegenüber der Verwendung von unsubstituierten 7-Desazaguanosinnukleotiden, die üblicherweise bei der Nukleinsäuresequenzierung zur Eliminierung von Bandenkompressionen GC-reicher DNA-Abschnitte eingesetzt werden (EP 212536), von Vorteil. Ein weiterer Vorteil in der Verwendung substituierter 7-Desazapurinnukleotiden bei der Sequenzierung ist, daß sich an den Substituenten in einer Reihe von Folgereaktionen fluoreszierende Reste in Form von Reportergruppen einführen lassen, die die fluoreszenzspektroskopische Detektion der während der Sequenzierungsreaktion synthetisierten Nucleinsäurermoleküle ermöglicht.
Des weiteren ermöglicht der Einbau von eigenfluoreszierenden, substituierten 7-Desazapurinbasen in Oligonukleotide, diese direkt über die Eigenfluoreszenz der substituierten 7-Desazapurinbasen nachzuweisen. So werden z.B. die in unsubstituierter Form nicht fluoresziernden 7-Desazapurinbasen fluoreszierend, wenn an die 7-Position eine Alkinylgruppe, z.B. Hexinyl eingeführt wird. Die Eigenfluoreszenz dieser Verbindungen lässt sich nach Anregung mit Licht der Wellenlänge 280 nm (Exitation) bei 350 nm (Emission) messen.

Die Darstellung der Verbindungen der Formel VI ist ausgehend von den entsprechenden substituierten 7-Desazapurin-Nucleosiden möglich und erfolgt nach allgemein bekannten Methoden. Bevorzugt können die Verbindungen der Formel VI nach einem verkürzten Eintopfverfahren von Ludwig in Gegenwart von 1,8-Bis(dimethylamino) naphathalin und Trimethylphosphat hergestellt werden [J. Ludwig et al., (1981) Acta Biochem. Biophys. Sci. Hung., 16, 131].

Die Erfindung betrifft auch Verbindungen der allgemeinen Formel VII

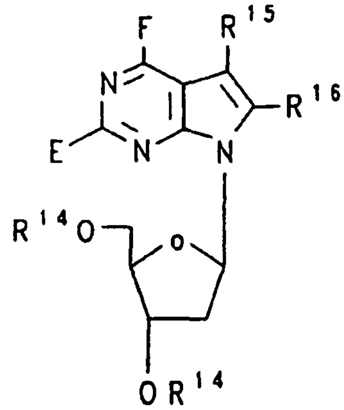

(VII)

worin

| | |
|---|---|
| E und F | unabhängig voneinander H, OH oder $NH_2$ bedeuten und OH und $NH_2$ gegebenenfalls mit einer in der Nukleotidchemie üblichen Schutzgruppe geschützt sind; |
| $R^{15}$ und $R^{16}$ | unabhängig voneinander Wasserstoff, $(C_1$-$C_{10})$-Alkyl, $(C_2$-$C_{10})$- Alkenyl, $(C_2$-$C_{10})$-Alkinyl, J, Cl, Br, F, Cyano oder $(C_1$-$C_{10})$-Alkyl, $(C_2$-$C_{10})$-Alkenyl oder $(C_2$-$C_{10})$-Alkinyl, worin ein bis alle H-Atome durch Halogen, vorzugsweise Fluor, substituiert sind, bedeuten, wobei $R^{15}$ und $R^{16}$ nicht gleichzeitig Wasserstoff und Cyano sein können, und der weiteren Maßgabe, daß $R^{15}$ nicht J ist, wenn $R^{16}$ Wasserstoff, E $NH_2$ und F OH bedeutet, |
| $R^{14}$ | unabhängig voneinander H oder eine in der Nukleotidchemie übliche Schutzgruppe bedeutet. |

Die Erfindung umfaßt auch alle tautomeren Formen der Verbindungen der Formel 1, V und VI, VII, insbesondere alle tautomeren Formen der 7-Desazapurinbasen der Formel 11.

Ganz allgemein sind auch solche Verbindungen der Formel V, VI und VII bevorzugt, die als Ausgangs- bzw. Zwischenverbindungen für die Herstellung bevorzugter Oligonukleotide der Formel I verwendet werden können.

Gegenstand der offenbarung ist weiterhin ein Verfahren zur Herstellung der erfindungsgemäßen Oligonukleotide der Formel I.

Zur Herstellung der erfindungsgemäßen, substituierte 7-Desazapurine enthaltende Oligonukleotide können die in der chemischen Synthese von Oligonucleotiden üblichen Standardbedingungen angewandt werden.

Die Darstellung der erfindungsgemäßen Oligonukleotide der Formel I erfolgt in Lösung oder vorzugsweise an fester Phase, gegebenenfalls unter Zuhilfenahme eines automatischen Synthesegeräts. Der Aufbau der Oligomere der Formel I kann schrittweise erfolgen, indem sukzessive eine Mononucleotid mit jeweils einer Nucleobase an einen entsprechend derivatisierten Träger oder an eine wachsende Oligomerkette ankondensiert werden.

Alternativ kann der Aufbau der Oligonukleotide der Formel I durch Zusammenfügen von Di- oder auch Trinukleotiden erfolgen [S. Beaucage et al, Tetrah. Vol. 48, No. 12, 2223-2311, (1992); und Tetrah. Vol. 48, No. 28, 6123-6194, (1993)]. Dies ist besonders bei der Synthese von Oligonukleotiden, die modifizierte Phosphatbrücken aufweisen, von Vorteil.

Der Aufbau des Oligonucleotide erfolgt nach den dem Fachmann bekannten Verfahren wie der Triester-Methode, der H-Phosphonat-Methode oder Phosphoramidit-Methode, [E. Sonveaux, (1986), Bioorganic Chemistry, 14, 274-325; S.L. Beaucage et al., (1992), Tetrathedron, 48, 2223-2311]. Zur Einführung der 7-Desazapurinderivate werden bevorzugt die Nukleotid-Monomerbausteine der Formel V, insbesonders bevorzugt die der Formel V in der E' gleich $NR^{10}R^{11}$ und F' gleich $OR^{12}$ ist oder F' gleich $NR^{10}NR^{11}$ ist und E' gleich H ist, eingesetzt.

Die Bereitstellung der Verbindungen der Formel V als Bausteine für die Oligonucleotid-Festphasensynthese kann ausgehend von den entsprechenden 7-Desazapurin-Nucleosiden erfolgen. Die Einführung von Substituenten an die 7-Position des 7-Desazapurinringsystems ist nach allgemein bekannten Methoden möglich. Die Herstellung von an der 7-Position mit Halogen oder Methyl substituierten 7-Desazapurinnucleoside wird beispielsweise von Seela et al. [Helvetica Chimica Acta, (1994) 77, 897-9031 beschrieben. Alkenyl- bzw. Alkinyl-substituierte 7-Desazapurinderivate der Formel V lassen sich ausgehend von dem bekannten 5-Jodtubercidin ( = 7-J-7-Desazaadenosin, siehe Seela et al., supra) herstellen, indem mittels einer Kreuzkupplungsreaktion in Gegenwart von Tetrakis(triphenyl-phosphin)-palladium (O) Alkenyl- oder Alkinylgruppen an die 7-Position des 7-Desazapurinringssystems gekoppelt werden. Elektrophile Substituenten (z.B. Halogene) können in die 8-Position des 7-Desazapuringsystems eingeführt werden, wenn als Ausgangsverbindungen Nukleoside eingesetzt werden, die einen elektronenliefernden Substituenten (beispielsweise eine Aminogruppe) an der 2-Position des 7-Desazapurins aufweisen. Ist die 2-Aminogruppe beispielsweise acetyliert, so

wird der elektrophile Substituent in die 7-Position dirigiert. Die vorliegende Offenbarung betrifft somit auch ein Vefahren zur regioselektiven Einführung von elektrophilen Substituenten (beispielsweise Halogenen) in die 7- oder 8- Position von 7-Desazanukleosiden. Die halogenierten Nukleoside können dann als Ausgangsverbindung für die Einführung anderer Substituenten, beispielsweise von Alkyl, Alkenyl- oder Alkinylgruppen über die oben beschriebene palladiumkatalysierte Kreuzkupplungsreaktion, dienen. Alkoxy- oder substituierte Aminderivate können durch nukleophile Substitution, Nitrogruppen durch elektrophile Substitution eingeführt werden.
Im Falle von 7,8 bis-alkinylierten 7-Desazapurinen läßt sich durch Bergman-Reaktionen und auch photochemisch ein Diradikal erzeugen, bzw. ein weiteres Ringsystem annelieren [N. Turro et al., Tetrahydron Letters, 1994, Vol. 35, 8089).
Nach Einführung geeigneter Schutzgruppen für die Aminogruppen der 7-Desazapurinbasen sowie für die freie 5'-Hydroxylgruppe des Zuckers werden die Monomere in die entsprechenden Phosphonat- bzw. Phosphoramidit-Derivate übergeführt. Die Einführung geeigneter Aminoschutzgruppen, z.B. in Form einer Formamidin-Schutzgruppe ((Dimethylamino)methyliden) oder Acylschutzgruppen (z.B. Benzoyl oder Phenoxyacetyl) erfolgt nach allgemein bekannten Methoden [L.J. Mc Bride et al, (1983) Tetrahedron Lett., 24, 2953, G.S. Ti et al, (1982) J. Am. Chem. Soc., 104, 1316; H. Schaller et al. (1963), J. Am. Chem. Soc., 85, 3821], wobei im Falle der Acylierung der Aminogruppe die Anwendung der Peracylierungsmethode nach Schaller vorteilhaft ist.
Eine geeignete Schutzgruppe für die freie 5'-OH-Gruppe des Zuckers ist z.B. 4,4'-Dimethoxytrityl, deren Einführung ebenfalls nach bekannten Methoden erfolgt [C.B. Reese (1978), Tetrahedron, 34, 3143; D. Flockerzi et al., (1981), Liebigs Ann. Chem.,1568]. Die solchermaßen geschützten Monomere können entsprechend einer Vorschrift von Froehler et al zu den entsprechenden Phosphonaten umgesetzt werden [B. C. Froehler et al., (1986), Nucl. Acid Res., 14, 5399]. Die Herstellung von Cyanoethyl-Phosphoramidit-Derivaten kann beispielsweise durch Umsetzung der Monomere mit Chlor-β-cyanoethoxy-(N,N-diisopropylamino)phosphan in wasserfreien Dichlormethan erfolgen [N. D. Sinha et al., (1984) Nucl. Acid Res., 12, 4539].

Die Synthese von Verbindungen der Formel I, deren Oligonucleotid-Teil am 3'- und/oder am 5'-Ende modifiziert ist, erfolgt bezüglich dieser Modifikationen nach den in EP-A 0 552 766 beschriebenen Verfahren.

Gegenstand der Erfindung sind weiterhin die Verwendung der erfindungsgemäßen Oligonukleotide der Formel I zur Herstellung eines Arzneimittels sowie ein Verfahren zur Herstellung eines Arzneimittels, das dadurch gekennzeichnet ist, daß man die erfindungsgemäßen Oligonucleotide mit einem physiologisch annehmbaren Träger sowie gegebenenfalls geeigneten Zusatz- und/oder Hilfsstoffen vermischt.

Ganz generell erstreckt sich die vorliegende Erfindung auf die Verwendung von Oligonukleotiden der Formel I als therapeutisch wirksame Bestandteile eines Arzneimittels. Als therapeutisch wirksame Oligonucleotid-Derivate versteht man im allgemeinen Antisense Oligonucleotide, Tripelhelix-bildende-Oligonucleotide, Aptamere oder Ribozyme, insbesondere Antisense-Oligonucleotide.

Darüberhinaus ist ein weiterer Gegenstand der vorliegenden Erfindung die Verwendung von Oligonukleotiden mit mindestens einem substituierten 7-Desazapurin, vorzugsweise 7-Desazaadenin oder 7-Desazaguanin, als Diagnostikum, beispielsweise zur Detektion der An- oder Abwesendheit oder der Menge eines spezifischen doppelsträngigen oder einzelsträngigen Nucleinsäuremoleküls in einer biologischen Probe.

Die Oligonukleotide haben für die erfindungsgemäße Verwendung eine Länge von 4 bis 100, vorzugsweise von ca. 5-40, insbesondere von ca. 6-30 Nucleotiden. Ansonsten gelten auch hier die oben beschriebenen Vorzugsbereiche, Modifikationen bzw. Konjugationen.

Die Arzneimittel der vorliegenden Erfindung können beispielsweise zur Behandlung von Erkrankungen, die durch Viren hervorgerufen werden, beispielsweise durch HIV, HSV-1, HSV-2, Influenza, VSV, Hepatitis B oder Papilloma Viren, verwendet werden.

Erfindungsgemäße Antisense Oligonucleotide-Derivate, also Antisense Oligonucleotide, in denen mindestens eine Purinbase durch eine substituierte 7-Desazapurinbase ersetzt ist, und die gegen solche Targets wirksam sind, haben beispielsweise folgende Basensequenzen:

a) gegen HIV, z.B.
5'-ACACCCAATTCTGAAAATGG-3' (I) oder
5'-AGGTCCCTGTTCGGGCGCCA-3' (II) oder
5'-GTCGACACCCAATTCTGAAAATGGATAA-3' (III) oder
5'-GCTATGTCGACACCCAATTCTGAAA-3' (IV) oder
5'-TCGTCGCTGTCTCCGCTTCTTCTTCCTGCCA (VI) oder

b) gegen HSV-1, z.B.
5'-GCGGGGCTCCATGGGGGTCG-3' (VII)

Die Arzneimittel der vorliegenden Erfindung eignen sich beispielsweise auch zur Behandlung von Krebs. Beispielsweise können dabei Oligonucleotid-Sequenzen zum Einsatz kommen, die gegen Targets gerichtet sind, die für

Krebsentstehung bzw. Krebswachstum verantwortlich sind. Solche Targets sind beispielsweise:

1) Nucleare Onkoproteine wie beispielsweise c-myc, N-myc, c-myb, c-fos, c-fos/jun, PCNA, p120

2) Cytoplasmische/Membran-assoziierte Onkoproteine wie beispielsweise EJ-ras, c-Ha-ras, N-ras, rrg, bcl-2, cdc-2, c-raf-1, c-mos, c-src, c-abl

3) Zelluläre Rezeptoren wie beispielsweise EGF-Rezeptor, c-erbA, Retinoid-Rezeptoren, Protein-Kinase regulatorische Untereinheit, c-fms

4) Cytokine, Wachstumsfaktoren, Extrazelluläre Matrix wie beispielsweise CSF-1, IL-6, IL-1a, IL-1b, IL-2, IL-4, bFGF, Myeloblastin, Fibronectin.

Erfindungsgemäße Antisense-Oligonucleotide der Formel I, die gegen solche Targets wirksam sind, haben beispielsweise folgende Basen-Sequenz:

a) gegen c-Ha-ras, z.B.
5'-CAGCTGCAACCCAGC-3' (VIII)

c) c-myc, z.B.
5'-GGCTGCTGGAGCGGGGCACAC-3' (IX)
5'-AACGTTGAGGGGCAT-3' (X)

d) c-myb, z.B.
5'-GTGCCGGGGTCTTCGGGC-3' (XI)

e) c-fos, z.B.
5'-GGAGAACATCATGGTCGAAAG-3' (XII)
5'-CCCGAGAACATCATGGTCGAAG-3' (XIII)
5'-GGGGAAAGCCCGGCAAGGGG-3' (XIV)

f) p120, z.B.
5'-CACCCGCCTTGGCCTCCCAC-3' (XV)

g) EGF-Rezeptor, z.B.
5'-GGGACTCCGGCGCAGCGC-3' (XVI)
5'-GGCAAACTTTCTTTTCCTCC-3' (XVII)

h) p53 Tumorsuppressor, z.B.
5'-GGGAAGGAGGAGGATGAGG-3' (XVIII)
5'-GGCAGTCATCCAGCTTCGGAG-3'r (XIX)

Die Arzneimittel der vorliegenden Erfindung eignen sich beispielsweise ferner zur Behandlung von Erkrankungen, die durch Integrine oder Zell-Zell-Adhäsionsrezeptoren beeinflußt werden, beispielsweise durch VLA-4, VLA-2, ICAM, VCAM oder ELAM.

Erfindungsgemäße Antisense-Oligonucleotid-Derivate, die gegen solche Targets wirksam sind, haben beispielsweise folgende Basen-Sequenz:

a) VLA-4, z.B.
5'-GCAGTAAGCATCCATATC-3' (XX)oder

b) ICAM, z.B.
5'-CCCCCACCACTTCCCCTCTC-3' (XXI)
5'-CTCCCCCACCACTTCCCCTC-3' (XXII)
5'-GCTGGGAGCCATAGCGAGG-3' (XXIII)

c) ELAM-1, z.B.
5'-ACTGCTGCCTCTTGTCTCAGG-3' (XXIV)

5'-CAATCAATGACTTCAAGAGTTC-3' (XXV)

Die Arzneimittel der vorliegenden Erfindung eignen sich beispielsweise auch zur Verhinderung der Restenose. Beispielsweise können dabei Oligonucleotid-Sequenzen zum Einsatz kommen, die gegen Targets gerichtet sind, die für Proliferation oder Migration verantwortlich sind. Solche Targets sind beispielsweise:

1) Nucleare Transaktivator-Proteine und Cycline wie beispielsweise c-myc, c-myb, c-fos, c-fos/jun, Cycline und cdc2-Kinase

2) Mitogene oder Wachstumsfaktoren wie beispielsweise PDGF, bFGF, EGF, HB-EGF und TGF-β.

3) Zelluläre Rezeptoren wie beispielsweise bFGF-Rezeptor, EGF-Rezeptor und PDGF-Rezeptor.

Erfindungsgemäße Oligonucleotide der Formel I, die gegen solche Targets wirksam sind, haben beispielsweise folgende Basen-Sequenz:

a) c-myb
5'-GTGTCGGGGTCTCCGGGC-3' (XXVI)

b) c-myc
5'-CACGTTGAGGGGCAT-3' (XXVII)

c) cdc2-Kinase
5'-GTCTTCCATAGTTACTCA-3' (XXVIII)

d) PCNA (proliferating cell nuclear antigen of rat)
5'-GATCAGGCGTGCCTCAAA-3' (XXIX)

Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Der Einschluß der Arzneimittel in Liposomen, die gegebenenfalls weitere Komponenten wie Proteine enthalten, ist eine ebenfalls geeignete Applikationsform. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talg und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Bevorzugte Verabreichungsformen sind topische Applikationen, lokale Applikationen wie beispielsweise mit Hilfe eines Katheters oder aber auch Injektionen. Zur Injektion werden die Antisense-Oligonucleotid-derivate in einer flüssigen Lösung, vorzugsweise in einem physiologisch annehmbaren Puffer, wie z.B. Hank's Lösung oder Ringer's Lösung, formuliert. Die Antisense-Oligonucleotide können aber auch in fester Form formuliert werden und vor dem Gebrauch gelöst oder suspendiert werden. Die für die systemische Verabreichung bevorzugten Dosierungen betragen ca. 0,01 mg/kg bis ca. 50 mg/kg Körpergewicht und Tag.

Ganz generell erstreckt sich die Erfindung auf die Verwendung von Verbindungen der Formel I als DNA-Sonden oder Primer in der DNA-Diagnostik und allgemein als Hilfsmittel in der Molekularbiologie

Einzelne DNA-Moleküle können elektronenmikroskopisch, z.B. im Tunnelrastermikroskop, sichtbar gemacht werden. Während Pyrimidinbasen sich aufgrund der Methylgruppe an der 5-Position elektronenmikroskopisch unterscheiden lassen ist dies bei den Purinbasen Adenin und Guanin nicht möglich. Es ist daher nicht ohne weiteres möglich, die Basensequenz von Nukleinsäurenmolekülen elektronenmikroskopisch zu entschlüsseln. Enthält das zu untersuchende Nukleinsäuremolekül nun aber z.B. substituierte 7-Desazaguaninderivate statt der unmodifizierten Guaninbasen, so lassen sich die substituierten 7-Desazaguaninbasen von unsubstituierten Adeninbasen im Elektronenmikroskop unterscheiden (und umgekehrt Guaninbasen von substituierten 7-Desazaadeninbasen). Die Basensequenz von Nukleinsäuren, die 7-substituierte 7-Desazapurinbasen enthalten, kann somit mit Hilfe der Elektronenmikroskopie entschlüsselt werden.

Beispiele:

Die in den Beispielen genannten Verbindungen (1)-(25) weisen folgende Strukturformeln auf.

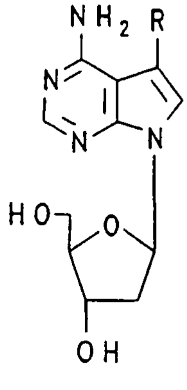

| | R |
|---|---|
| (1) | Cl |
| (2) | Br |
| (3) | $CH_3$ |

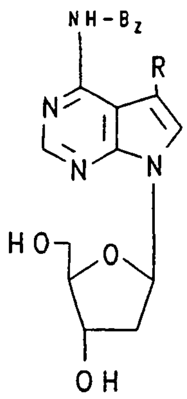

| | R |
|---|---|
| (4) | Cl |
| (5) | Br |
| (6) | $CH_3$ |

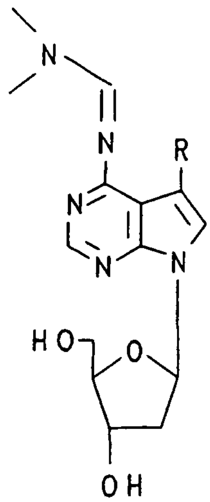

| | R |
|---|---|
| (7) | Br |

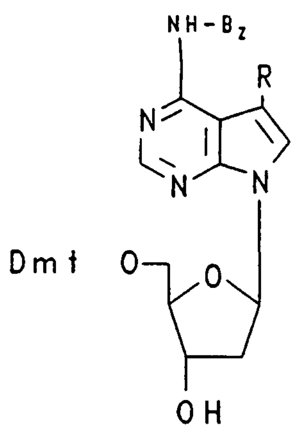

| | R |
|---|---|
| (8) | Cl |
| (9) | Br |
| (10) | $CH_3$ |

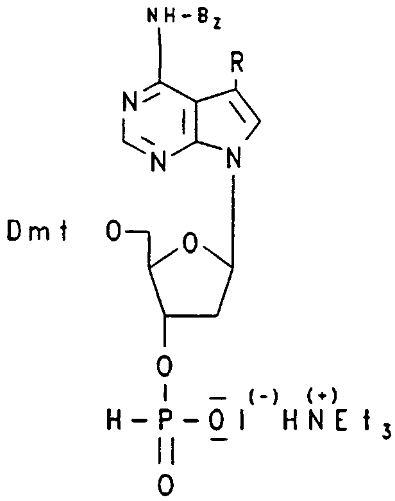

| | R |
|---|---|
| (11) | Cl |
| (12) | Br |
| (13) | $CH_3$ |
| (15) | $-C\equiv C-CH_2NHC-CF_3$ |
| (16) | $-C\equiv C-(CH)_3NHC-CF_3$ |

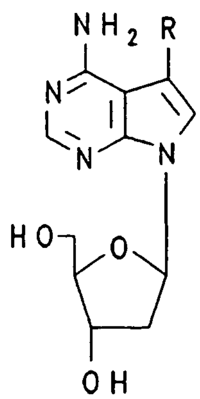

| | R |
|---|---|
| (14) | J |
| (17) | $-HC=CHCO_2Me$ |
| (23) | $-C\equiv C-Si(Me)_3$ |
| (24) | $-C\equiv CH$ |
| (25) | $-C\equiv C-(CH_2)_3Me$ |

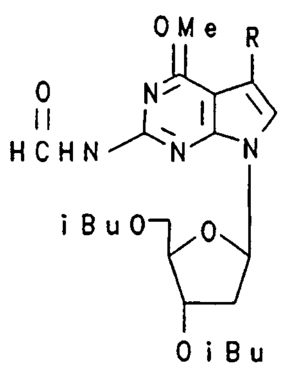

| | R |
|---|---|
| (18) | H |
| (19) | Br |

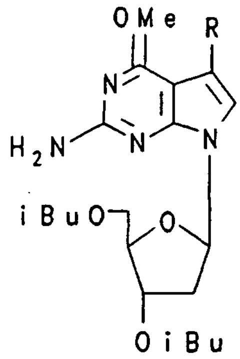

| | R |
|---|---|
| (20) | Cl |

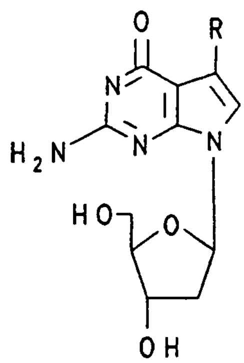

| | R |
|---|---|
| (21) | Br |
| (22) | Cl |

Die Herstellung der Desoxytubercidinderivate (1)-(3) (Tubercidin = 7-Desazaadenosin) erfolgt nach dem von Seela et al. [Helvetica Chimica Acta, 1994, 77, 897-903) beschriebenen Verfahren.

Bei Verwendung eines Tubercidinderivats als Ausgangsverbindung können analog zu den folgenden Beispielen die entsprechenden Ribonukleosidderivate hergestellt werden.

Beispiel 1:

4-Benzoylamino-5-chlor-7-(2-desoxy-β-D-erythro-pentofuranosyl)-7H-pyrrolo[2,3-d]pyrimidin (4)

1.14 g (4.0 mmol) 5-Chlordesoxytubercidin (1) werden zweimal mit trockenem Pyridin abgedampft, in 10 ml trockenem Pyridin gelöst und anschließend mit 5.2 ml (40.6 mmol) Trimethylchlorsilan 2 h bei Raumtemperatur gerührt. Dann wird mit 520 µl (4.1 mmol) frisch destilliertem Benzoylchlorid versetzt und weitere 2 h bei Raumtemperatur gerührt. Unter Eiskühlung werden 4 ml Wasser und nach weiteren 5 min 8 ml 25%-iges wäßriges Ammoniak zugetropft. Der Ansatz wird 30 min bei Raumtemperatur gerührt und anschließend bis zur Trockene eingedampft. Der Rückstand wird in 20 ml Wasser aufgenommen und dreimal mit je 30 ml Ethylacetat extrahiert. Die organischen Phasen werden über $Na_2SO_4$ getrocknet, eingedampft und an Kieselgel chromatographiert (Säule 20 • 5 cm, Dichlormethan/ Methanol 9:1). Aus der langsamer wandernden Hauptfraktion erhält man nach Eindampfen des Lösungsmittels und Umkristallisation aus Methanol/ Wasser 930 mg (2.4 mmol, 60%) der Verbindung (4) als farblose Kristalle: Schmp. 190˚C.
DC (Kieselgel, Dichlormethan/ Methanol 9:1): $R_f$ = 0.4.
UV (MeOH): $\lambda_{max}$ = 274 nm (5300), 305 nm (5600).
$^1$H-NMR ([D$_6$]DMSO): δ = 2.31 (m, 2'α-H), 2.57 (m, 2'β-H), 3.58 (m, 2H, 5'-H), 3.89 (m, 4'-H), 4.41 (m, 3'-H), 5.00 (t, J = 5.0 Hz, 5'-OH), 5.33 (d, J = 5.3 Hz, 3'-OH), 6.72 (pt, J = 6.75 Hz, 1'-H), 7.44-7.65 (m, 3H, meta- und para-$H_{Bz}$), 8.00 (s, 6-H), 8.05 (d, 2H, ortho-$H_{Bz}$), 8.72 (s, 2-H), 11.2 (br, 4-NH).

| | | | | |
|---|---|---|---|---|
| $C_{18}H_{17}ClN_4O_4$ (388.8) | Ber. | C 55.61 | H 4.41 | N 14.41 |
| | Gef. | C 55.71 | H 4.54 | N 14.30 |

Beispiel 2:

4-Benzoylamino-5-brom-7-(2-desoxy-β-D-erythro-pentofuranosyl)-7H-pyrrolo[2,3-d]pyrimidin (5)

1.31 g (4.0 mmol) 5-Bromdesoxytubercidin (2) werden zweimal mit trockenem Pyridin abgedampft, in 10 ml trockenem Pyridin gelöst und anschließend mit 5.2 ml (40.6 mmol) Trimethylchlorsilan 2 h bei Raumtemperatur gerührt. Dann wird mit 520 µl (4.1 mmol) frisch destilliertem Benzoylchlorid versetzt und weitere 2 h bei Raumtemperatur gerührt. Unter Eiskühlung werden 4 ml Wasser und nach weiteren 5 min 8 ml 25%-iges wäßriges Ammoniak zugetropft. Der Ansatz wird 30 min. bei Raumtemperatur gerührt und anschließend analog zu Verbindung 14b aufgearbeitet. Nach Chromatographie an Kieselgel (Säule 20 • 5 cm, Dichlormethan/ Methanol 9:1) erhält man nach Eindampfen des Lösungsmittels und Umkristallisation aus Methanol/ Wasser 1.2 g (2.8 mmol, 70%) farblose Kristalle vom Schmp. 198˚C.
DC (Kieselgel, Dichlormethan/ Methanol 9:1): $R_f$ = 0.4.
UV (MeOH): $\lambda_{max}$ = 276 nm (4600), 308 nm (4500).
$^1$H-NMR ([$D_6$]DMSO): δ = 2.27 (m, 2'α-H), 2.50 (m, 2'β-H, überlagert von DMSO), 3.56 (m, 2H, 5'-H), 3.86 (m, 4'-H), 4.38 (m, 3'-H), 5.01 (t, J = 5.0 Hz, 5'-OH), 5.34 (d, J = 5.3 Hz, 3'-OH), 6.69 (pt, J = 6.7 Hz, 1'-H), 7.52-7.64 (m, 3H, meta- und para-$H_{Bz}$), 8.04 (d, 2H, ortho-$H_{Bz}$), 8.04 (s, 6-H), 8.72 (s, 2-H), 11.0 (br, 4-NH).

| | | | | |
|---|---|---|---|---|
| $C_{18}H_{17}BrN_4O_4$ (433.3) | Ber. | C 49.90 | H 3.96 | N 12.93 |
| | Gef. | C 50.04 | H 4.10 | N 13.05 |

Beispiel 3:

4-Benzoylamino-7-(2-desoxy-β-D-erythro-pentofuranosyl)- 5-methyl-7H-pyrrolo[2,3-d]pyrimidin (6)

1.06 g (4.0 mmol) 5-Methyldesoxytubercidin (3) werden zweimal mit je 20 ml absolutem Pyridin nachgedampft, in 10 ml trockenem Pyridin gelöst und mit 5.2 ml (40.6 mmol) Trimethylchlorsilan 2 h bei Raumtemperatur gerührt.
Anschließend wird mit 520 µl (4.1 mmol) frisch destilliertem Benzoylchlorid versetzt und weitere 2 h bei Raumtemperatur gerührt. Die Aufarbeitung erfolgt analog zu Verbindung (4), anschließend wird an Kieselgel chromatographiert (Säule 20 • 5 cm, Dichlormethan/ Methanol 9:1). Aus der langsamer wandernden Hauptfraktion erhält man nach Eindampfen des Lösungsmittels und Umkristallisation aus Methanol/ Wasser 1.1 g (2.9 mmol, 73%) farblose Kristalle (Verbindung 6) vom Schmp. 196˚C.
DC (Kieselgel, Dichlormethan/ Methanol 9:1): $R_f$ = 0.3.
UV (MeOH): $\lambda_{max}$ = 274 nm (7050), 309 nm (5500).
$^1$H-NMR ([$D_6$]DMSO): δ = 2.09 (m, 2'α-H), 2.21 (s, 5-$CH_3$), 2.50 (m, 2'β-H, überlagert von DMSO), 3.53 (m, 2H, 5'-H), 3.83 (m, 4'-H), 4.36 (m, 3'-H), 4.97 (t, J = 5.0 Hz, 5'-OH), 5.32 (d, J = 5.3 Hz, 3'-OH), 6.65 (pt, J = 6.7 Hz, 1'-H), 7.53 (s, 6-H), 7.53-7.66 (m, 3H, meta- und para-$H_{Bz}$), 8.05 (d, 2H, ortho-$H_{Bz}$), 8.60 (s, 2-H), 10.95 (br, 4-NH).

| | | | | |
|---|---|---|---|---|
| $C_{19}H_{20}N_4O_4$ (368.4) | Ber. | C 61.95 | H 5.47 | N 15.21 |
| | Gef. | C 62.08 | H 5.65 | N 15.00 |

Beispiel 4:

5-Brom-4-[(1-dimethylamino)methylen]amino-7-(2-desoxy-β-D-erythro-pentofuranosyl)-7H-pyrrolo[2,3-d]pyrimidin (7)

Zu einer Lösung von 200 mg (0.61 mmol) der Verbindung (2) in 15 ml Dimethylformamid gibt man 1.5 ml (8.75 mmol) N,N-Dimethylformamiddiethylacetal und läßt 2 h bei Raumtemperatur rühren. Anschließend engt man die Reaktionslösung bis zur Trockene ein und dampft den öligen Rückstand je zweimal mit Toluol und Aceton nach. Das Rohprodukt wird an Kieselgel adsorbiert und durch Säulenchromatographie gereinigt (Säule 20 • 5 cm, Dichlormethan/ Methanol 9:1). Nach Einengen der Hauptfraktion und Umkristallisation aus Aceton/ Methanol 9:1 erhält man Verbindung (7) als farblose Plättchen (150 mg, 0.4 mmol, 65%): Schmp. 177˚C.
DC (Kieselgel, Dichlormethan/ Methanol 9:1): $R_f$ = 0.65.
$^1$H-NMR ([$D_6$]DMSO): δ = 2.20 (m, 2'α-H), 2.50 (m, 2'β-H, überlagert von DMSO), 3.18, 3.19 (2s, 2 N-$CH_3$), 3.54 (m,

2H, 5'-H), 3.86 (m, 4'-H), 4.35 (m, 3'-H), 5.01 (t, J = 5.5 Hz, 5'-OH), 5.26 (d, J = 5.0 Hz, 3'-OH), 6.57 (pt, J = 6.9 Hz, 1'-H), 7.70 (s, 6-H), 8.34 (s, 2-H), 8.82 (s, N=CH).

| | | | | |
|---|---|---|---|---|
| $C_{14}H_{18}BrN_5O_3$ (384.2) | Ber. | C 43.77 | H 4.72 | N 18.23 |
| | Gef. | C 43.92 | H 4.80 | N 18.11 |

Beispiel 5:

4-Benzoylamino-5-chlor-7-[(2-desoxy-β-D-erythro-pentofuranosyl)-5'-O-(4,4'-dimethoxytriphenylmethyl)]-7H-pyrrolo [2,3-d]pyrimidin (8)

500 mg (1.28 mmol) von Verbindung (4) werden zweimal mit trockenem Pyridin abgedampft und anschließend in 20 ml absolutem Pyridin gelöst. Man versetzt mit 650 mg (1.95 mmol) Dimethoxytritylchlorid und rührt 1 h bei Raumtemperatur. Der Ansatz wird mit 10 ml 5%-iger wäßriger $NaHCO_3$-Lösung hydrolysiert und zweimal mit je 25 ml Dichlormethan extrahiert. Nach Trocknen der vereinigten organischen Phasen über $Na_2SO_4$ wird an Kieselgel chromatographiert (Säule 20 • 5 cm, Dichlormethan/ Methanol 9:1). Der nach Eindampfen der Hauptzone erhaltene Rückstand liefert nach Abdampfen mit Aceton 680 mg (0.99 mmol, 77%) gelblichen Schaum. Zur Aufreinigung wird die Substanz in wenig Dichlormethan gelöst und langsam unter starkem Rühren in einen 200fachen Überschuß n-Hexan getropft. Verbindung (8) wird als weißer, amorpher Feststoff isoliert.
DC (Kieselgel, Dichlormethan/ Methanol 9:1): $R_f$ = 0.5.
$^1$H-NMR ([$D_6$]DMSO): δ = 2.30 (m, 2'α-H), 2.50 (m, 2',β-H, überlagert von DMSO), 3.15 (m, 2H, 5'-H), 3.73 (s, 6H, 2 $OCH_3$), 3.98 (m, 4'-H), 4.42 (m, 3'-H), 5.40 (d, J = 5.0 Hz, 3'-OH), 6.69 (pt, J = 6.7 Hz, 1'-H), 6.84 (m, 4H, DMT), 7.2-7.8 (m, 12H, aromatische Protonen), 7.87 (s, 6-H), 8.06 (d, 2H, ortho-$H_{Br}$), 8.70 (s, 2-H), 11.0 (br, 4-NH).

| | | | | |
|---|---|---|---|---|
| $C_{39}H_{35}ClN_4O_6$ (691.2) | Ber. | C 67.77 | H 5.10 | N 8.11 |
| | Gef. | C 67.70 | H 5.05 | N 8.19 |

Beispiel 6:

4-Benzoylamino-5-brom-7-[(2-desoxy-β-D-erythro-pentofuranosyl)-5'-O-(4,4'-dimethoxytriphenylmethyl)]-7H-pyrrolo [2,3-d]pyrimidin (9)

500 mg (1.15 mmol) von Verbindung (5) werden zweimal mit trockenem Pyridin abgedampft und anschließend in 20 ml absolutem Pyridin gelöst. Man versetzt mit 585 mg (1.75 mmol) Dimethoxytritylchlorid und rührt 1 h bei Raumtemperatur. Der Ansatz wird mit 10 ml 5%-iger wäßriger $NaHCO_3$-Lösung hydrolysiert und zweimal mit je 25 ml Dichlormethan extrahiert. Nach Trocknen der vereinigten organischen Phasen über $Na_2SO_4$ wird an Kieselgel chromatographiert (Säule 20 • 5 cm, Dichlormethan/ Methanol 9:1). Der nach Eindampfen der Hauptzone erhaltene Rückstand liefert nach Abdampfen mit Aceton 620 mg (0.93 mmol, 80%) gelblichen Schaum. Zur Aufreinigung wird die Substanz in wenig Dichlormethan gelöst und langsam unter starkem Rühren in einen 200fachen Überschuß n-Hexan getropft. Verbindung (9) fällt als weißer, amorpher Feststoff aus und wird abgesaugt.
DC (Kieselgel, Dichlormethan/ Methanol 9:1): $R_f$ = 0.55.
$^1$H-NMR ([$D_6$]DMSO): δ = 2.30 (m, 2'α-H), 2.50 (m, 2'β-H, überlagert von DMSO), 3.15 (m, 2H, 5'-H), 3.73 (s, 6H, 2 $OCH_3$), 3.98 (m, 4'-H), 4.42 (m, 3'-H), 5.40 (d, J = 5.0 Hz, 3'-OH), 6.69 (pt, J = 6.7 Hz, 1'-H), 6.84 (m, 4H, DMT), 7.2-7.8 (m, 12H, aromatische Protonen), 7.87 (s, 6-H), 8.06 (d, 2H, ortho-$H_{Bz}$), 8.70 (s, 2-H), 11.0 (br, 4-NH).

| | | | | |
|---|---|---|---|---|
| $C_{39}H_{35}BrN_4O_6$ (735.6) | Ber. | C 63.68 | H 4.79 | N 7.62 |
| | Gef. | C 63.85 | H 4.67 | N 7.52 |

Beispiel 7:

4-Benzoylamino-7-[(2-desoxy-β-D-erythro-pentofuranosyl)-5'-O-(4,4'-dimethoxytriphenylmethyl)]-5-methyl-7H-pyrrolo [2,3-d]pyrimidin (10)

500 mg (1.36 mmol) von Verbindung (6) werden zweimal mit je 20 ml trockenem Pyridin abgedampft, in 20 ml absolutem Pyridin gelöst und mit 690 mg (2.1 mmol) Dimethoxytritylchlorid 1 h bei Raumtemperatur gerührt. Der Ansatz wird analog zu Verbindung (8) aufgearbeitet und an Kieselgel chromatographiert (Säule 20 • 5 cm, Dichlormethan/

Methanol 9:1). Aus der Hauptzone erhält man 720 mg (1.05 mmol, 77%) der vollgeschützten Verbindung (10) als gelblichen Schaum. Die Aufreinigung durch Umfällen aus n-Hexan liefert einen farblosen, amorphen Feststoff.
DC (Kieselgel, Dichlormethan/ Methanol 9:1): $R_f$ = 0.5.
$^1$H-NMR ([$D_6$]DMSO): δ = 2.08 (s, 5-$CH_3$), 2.30 (m, 2'α-H), 2.50 (m, 2'β-H, überlagert von DMSO), 3.10 (m, 2H, 5'-H), 3.73 (s, 6H, 2 $OCH_3$), 3.97 (m, 4'-H), 4.44 (m, 3'-H), 5.39 (d, J = 5.0 Hz, 3'-OH), 6.67 (pt, J = 6.7 Hz, 1'-H), 6.85 (m, 4H, DMT), 7.2-7.8 (m, 12H, aromatische Protonen), 7.58 (s, 6-H), 8.06 (d, 2H, ortho-$H_{Bz}$), 8.60 (s, 2-H), 10.95 (br, 4-NH).

| | | | | |
|---|---|---|---|---|
| $C_{40}H_{38}N_4O_6$ (670.8) | Ber. | C 71.63 | H 5.71 | N 8.35 |
| | Gef. | C 71.48 | H 5.71 | N 8.36 |

Beispiel 8:

4-Benzoylamino-5-chlor-7-[(2-desoxy-β-D-erythro-pentofuranosyl)-5'-O-(4,4'-dimethoxytriphenylmethyl)]-7H-pyrrolo [2,3-d]pyrimidin 3'-(Triethylammonium Phosphonat) (11)

Unter Argonatmosphäre wird zu einer Lösung von 315 μl (3.7 mmol) Phosphortrichlorid und 4.1 ml (37.0 mmol) N-Methylmorpholin in 40 ml absolutem Dichlormethan bei Raumtemperatur 840 mg (12.0 mmol) 1,2,4-1H-Triazol gegeben. Nach 30-minütigem Rühren wird auf 0˚C gekühlt und innerhalb von 10 min eine Lösung von 500 mg (0.74 mmol) des vollgeschützten Nucleosids (8) in 10 ml trockenem Dichlormethan zugetropft. Man läßt weitere 10 min bei Raumtemperatur rühren und fügt anschließend 30 ml 1M Triethylammoniumbicarbonatpuffer (TBK, pH = 7.5) hinzu. Die Phasen werden getrennt, die wäßrige Phase mehrmals mit $CH_2Cl_2$ extrahiert und die vereinigten organischen Phasen über $Na_2SO_4$ getrocknet. Das Lösungsmittel wird abgedampft und der verbleibende Schaum an Kieselgel chromatographiert (Säule 20 • 5 cm, 0.5 l Dichlormethan/ $Et_3N$ 98:2, danach Dichlormethan/ Methanol/ $Et_3N$ 88:10:2). Nach Einengen der Hauptzone wird der Rückstand in 50 ml Dichlormethan aufgenommen und fünfmal mit je 25 ml 0.1 M TBK-Puffer extrahiert. Nach Trocknung der organischen Phase über $Na_2SO_4$ und Abdampfen des Lösungsmittels erhält man 445 mg (0.52 mmol, 70%) des Phosphonats (11) als farblosen Schaum. Zur weiteren Aufreinigung wird dieser Schaum analog zum vollgeschützten Nucleosid (8) aus n-Hexan umgefällt.
DC (Kieselgel, Dichlormethan/ Methanol 9:1): $R_f$ = 0.6.
$^1$H-NMR ([$D_6$)DMSO): δ = 1.16 (t, 9H, $(CH_3CH_2)_3N$), 2.50 (m, 2'α-H, überlagert von DMSO), 2.74 (m, 2',β-H), 3.00 (q, 6H, $(CH_3CH_2)_3N$), 3.33 (m, 2H, 5'-H), 3.72 (s, 6H, 2 $OCH_3$), 4.15 (m, 4'-H), 4.78 (m, 3'-H), 6.66 (d, J = 585.8 Hz, P-H), 6.69 (pt, J = 7.8 Hz, 1'-H), 6.84 (m, 4H, DMT), 7.2-7.7 (m, 12H, aromatische Protonen), 7.79 (s, 6-H), 8.04 (d, 2H, ortho-$H_{Bz}$), 8.69 (s, 2-H), 10.6 (br, 4-NH). $^{31}$P-NMR ([$D_6$]DMSO): δ = 1.16 ppm (dd, $^1$J(PH) = 588 Hz, $^3$J(PH) = 8.6 Hz.
$C_{45}H_{51}ClN_5O_8P$ (900.8)

Beispiel 9:

4-Benzoylamino-5-brom-7-[(2-desoxy-β-D-erythro-pentofuranosyl)-5'-O-(4,4'-dimethoxytriphenylmethyl)]-7H-pyrrolo [2,3-d]pyrimidin 3'-(Triethylammonium Phosphonat) (12)

Unter Argonatmosphäre wird zu einer Lösung von 290 μl (3.4 mmol) Phosphortrichlorid und 3.8 ml (34.0 mmol) N-Methylmorpholin in 30 ml absolutem Dichlormethan bei Raumtemperatur 770 mg (11.0 mmol) 1,2,4-1H-Triazol gegeben. Nach 30-minütigem Rühren wird auf 0˚C gekühlt und innerhalb von 10 min eine Lösung von 500 mg (0.68 mmol) des vollgeschützten Nucleosids (9) in 10 ml trockenem Dichlormethan zugetropft. Man läßt weitere 10 min bei Raumtemperatur rühren und fügt anschließend 30 ml 1M Triethylammoniumbicarbonatpuffer (TBK, pH = 7.5) hinzu. Die Phasen werden getrennt, die wäßrige Phase mehrmals mit $CH_2Cl_2$ extrahiert und die vereinigten organischen Phasen über $Na_2SO_4$ getrocknet. Das Lösungsmittel wird abgedampft und der verbleibende Schaum an Kieselgel chromatographiert (Säule 20 • 5 cm, 0.5 l Dichlormethan/ $Et_3N$ 98:2, danach Dichlormethan/ Methanol/ $Et_3N$ 88:10:2). Nach Einengen der Hauptzone wird der Rückstand in 50 ml Dichlormethan aufgenommen und fünfmal mit je 25 ml 0.1 M TBK-Puffer extrahiert. Nach Trocknung der organischen Phase über $Na_2SO_4$ und Abdampfen des Lösungsmittels erhält man 410 mg (0.46 mmol, 67%) des Phosphonats (12) als farblosen Schaum. Zur weiteren Aufreinigung kann dieser Schaum analog zum vollgeschützten Nucleosid (9) aus n-Hexan umgefällt werden.
DC (Kieselgel, Dichlormethan/ Methanol 9:1): $R_f$ = 0.7.
$^1$H-NMR ([$D_6$]DMSO): δ = 1.16 (t, 9H, $(CH_3CH_2)_3N$), 2.50 (m, 2'a-H, überlagert von DMSO), 2.78 (m, 2'β-H), 3.00 (q, 6H, $(CH_3CH_2)_3N$), 3.22 (m, 2H, 5'-H), 3.73 (s, 6H, 2 $OCH_3$), 4.17 (m, 4'-H), 4.82 (m, 3'-H), 6.68 (d, J = 588.5 Hz, P-H), 6.69 (pt, J = 6.7 Hz, 1'-H), 6.90 (m, 4H, DMT), 7.2-7.8 (m, 12H, aromatische Protonen), 7.86 (s, 6-H), 8.07 (d, 2H, ortho-$H_{Bz}$), 8.70 (s, 2-H), 11.05 (br, 4-NH). $^{31}$P-NMR ([$D_6$]DMSO): δ = 1.16 ppm (dd, $^1$J(PH) = 588 Hz, $^3$J(PH) = 8.6 Hz).
$C_{45}H_{51}BrN_5O_8P$ (900.8)

Beispiel 10:

4-Benzoylamino-7-[(2-desoxy-β-D-erythropentofuranosyl)-5'-O-(4,4'-dimethoxytriphenylmethyl)]-5-methyl-7H-pyrrolo [2,3-d]pyrimidin 3'-(Triethylammonium Phosphonat) (13)

Unter Argonatmosphäre werden zu einer Lösung von 25 ml absolutem Dichlormethan, 290 µl (3.4 mmol) Phosphortrichlorid und 3.44 g (34.0 mmol) N-Methylmorpholin bei Raumtemperatur 0.78 g (11.3 mmol) 1,2,4-H-Triazol gegeben. Nach 30-minütigem Rühren wird die Reaktionslösung auf 0°C abgekühlt und innerhalb von 10 min mit einer Lösung von 500 mg (0.75 mmol) des vollgeschützten Nucleosids (10) in 15 ml Dichlormethan versetzt. Man läßt weitere 20 min bei Raumtemperatur rühren und hydrolysiert anschließend mit 1 M Triethylammoniumbicarbonat-Puffer (TBK, pH = 7.5). Nach Phasentrennung, dreimaliger Extraktion der wäßrigen Phase mit je 20 ml Dichlormethan, Trocknen und Eindampfen der organischen Phase wird an Kieselgel chromatographiert (Säule 20 • 5 cm, 0.5 l Dichlormethan/ $Et_3N$ 98:2, danach Dichlormethan/ Methanol/ $Et_3N$ 88:10:2). Die Hauptzone wird eingedampft, in 50 ml Dichlormethan aufgenommen und mehrmals mit 0.1 M TBK-Puffer extrahiert. Nach Trocknung der organischen Phase über $Na_2SO_4$ und Abdampfen des Lösungsmittels erhält man 440 mg (0.53 mmol, 70%) der Verbindung (13) als farblosen Schaum.
DC (Kieselgel, Dichlormethan/ Methanol 9:1): $R_f$ = 0.65.
$^1$H-NMR ([$D_6$]DMSO): δ = 1.16 (t, 9H, $(CH_3CH_2)_3N$), 2.09 (s, 5-$CH_3$), 2.24 (m, 2'a-H), 2.67 (m, 2' β-H), 3.00 (q, 6H, $(CH_3CH_2)_3N$), 3.20 (m, 2H, 5'-H), 3.73 (s, 6H, 2 $OCH_3$), 4.13 (m, 4'-H), 4.83 (m, 3'-H), 6.65 (pt, J = 6.5 Hz, 1'-H), 6.68 (d, J = 588.5 Hz, P-H), 6.85 (m, 4H, DMT), 7.2-7.6 (m, 12H, aromatische Protonen), 7.58 (s, 6-H), 8.05 (d, 2H, ortho-$H_{Bz}$), 8.60 (s, 2-H), 10.98 (br, 4-NH). $^{31}$P-NMR ([$D_6$]DMSO): δ = 1.08 ppm (dd, $^1J(PH)$ = 577 Hz, $^3J(PH)$ = 8.9 Hz). $C_{46}H_{54}N_5O_8P$ (835.8)

Beispiel 11:

4-Amino-5-brom-7-(2-desoxy-β-D-erythro-pentofuranosyl)-7H-pyrrolo[2,3-d]pyrimidin 5'-O-Triphosphat, Triethylammoniumsalz

Verbindung (2) (33 mg, 0.1 mmol) wird zusammen mit 1,8-Bis(dimethylamino)naphthalin (33 mg , 0.15 mmol) in Trimetylphosphat (0.25 ml) unter leichtem Erwärmen in Lösung gebracht. Nach Abkühlen auf 0°C erfolgt eine Zugabe von frisch destilliertem $POCl_3$ (12 µl, 0.13 mmol). Das Reaktionsgemisch wird für 4 h bei 4°C gehalten und anschließend eine Lösung aus Tri-n-butylammoniumdiphosphat (0.5 m in DMF, 1 ml) und Tri-n-butylamin (100 µl, 0.42 mmol) zugegeben. Nach 3 min Rühren bei 0°C wird die Mischung mit 1 M TBK-Puffer (10 ml) versetzt und zur Trockene eingedampft. Der Rückstand wird an DEAD Sephadex (Säule 1.5 • 20 cm, $HCO_3^-$-Form) chromatographiert. Nach Waschen mit ca. 500 ml $H_2O$ wurde mit einem linearen Gradienten von $H_2O$/ 0.9 M TBK-Puffer (je 1 l) chromatographiert. Dabei erhält man das Triphosphat (0.019 mM, 20%) bei ca. 0.5 M TBK-Puffer. DC (Kieselgel, i-Propanol/$H_2O$/$NH_3$, 3:1:1): $R_f$ = 0.2. UV ($H_2O$): $\lambda_{max}$ = 269 nm.
$^{31}$P-NMR (0.1 M Tris-HCl, pH 8.0, 100 mM EDTA/$D_2O$): -11.87 (d, J = 20.2, $P_\gamma$); -10.98 (td, J = 20.0 und 6.0, $P_\alpha$); -23.06 (t, J = 20.2, $P_\beta$).

Beispiel 12:

4-Amino-7-(2-desoxy-β-D-erythro-pentofuranosyl)-5-jod-7H- pyrrolo[2,3-d]pyrimidin (14) (5-Jod-2'-desoxytubercidin).

Zu 1.0 g (2.5 mmol) 4-Chlor-7-[2-desoxy-3,5-di-O-(4-toluoyl)-β-D-erythro-pentofuranosyl]-5-jod-7H-pyrrolo [2,3-d]pyrimidin, gelöst in 80 ml Dioxan, wird 25%iges wäßriges Ammoniak hinzugegeben. Der Ansatz wird 48 h bei 110°C in einer Stahlbombe gerührt. Nach Abdampfen wird der eingeengte Rest auf Kieselgel chromatographiert (Säule 20x5 cm, Lösungsmittel B). Farblose kristalle von MeOH (0.75 g, 2.0 mmol, 45%). M.p. 194°C. TLC: $R_f$ 0.4 ($CH_2Cl_2$/ MeOH 9:1). UV (MeOH) 283 nm (5 800).
1H-NMR ($D_6$-DMSO): 2.16 (m, H-2'α), 2.46 (m, H-$2'_\beta$, überlagert von DMSO), 3.54 (m, 2-H, H-5'), 3.81 (m, H-4'), 4.33 (m, H-3'), 5.00 (t, J = 5.1 Hz, 5'-OH), 5.23 (d, J = 5.1 Hz, 3'-OH), 6.49 (pt, J = 6.7 Hz, H-1'), 6.65 (br, $NH_2$), 7.65 (s, H-6), 8.10 (s, H-2).
13C-NMR ($D_6$-DMSO) 157.3 (C-4), 152.0 (C-2), 149.8 (C-7a), 126.9 (C-6), 103.2 (C-4a), 87.5 (C-4'), 83.0 (C-1'), 71.0 (C-3'), 62.0 (C-5'), 51.9 (C-5), 39.8 (C-2').
Anal. berechnet für $C_{11}H_{13}IN_4O_3$: C 35.13, H 3.48, N 14.90; gefunden: C 35.33, H 3.69, N 15.01.

Beispiel 13:

Kreuzkupplungsreaktion von 5-Jod-2'-Desoxytubercidin (14)

5-Jod-2'-desoxytubercidin (14) (200 mg, 0.532 mmol) und Kupfer(I)Jodid (10 mg, 10 mol%) werden in 3 ml trockenem, mit Argon vorgespülten DMF suspendiert und mit Alkin (6-15 eq.), trockenem Trietylamin (108 mg, 1.06 mmol, 2 eq.) und Tetrakis(triphenylphosphin)palladium (0) (30.75 mg, 0.027 mmol, 5 mol%) versetzt.
Innerhalb einiger Stunden entsteht aus der Mischung eine klare gelbe Lösung. Die Reaktion wird solange fortgeführt, bis die Ausgangsprodukte verbraucht sind (Überprüfung mittels Dünnschichtchromatographie). Das Reaktionsgemisch wird dann mit 5 ml Methanol-Dichlormethan (1:1) verdünnt und mit Dowex 1x8 (100-200 mesh; 500 mg, Bicarbonatform) versetzt. Nachdem nach 15 Minuten Rühren die Gasbildung zum Stillstand kommt wird noch für weitere 30 Minuten gerührt. Anschließend wird das Reaktionsgemisch filtriert und die Matrix mit Methanol-Dichlormethan (1:1) gewaschen. Die Filtrate werden vereinigt und getrocknet. Der getrocknete Rückstand wird sofort über eine Kieselgelsäule (25 g) unter Verwendung von Dichlormethan mit zunehmenden Gehalt von Methanol (10, 15, 20%) chromatographiert. Nach Eindampfen der Hauptfraktion erhält man das substituierte 2'-Desoxytubercidinderivat.

Beispiel 14:

4-Benzoylamino-5-(1-propinyl-3-trifluoracetamid)-7-[(2-desoxy-β-D-erythro-pentofuranosyl)-5'-O-(4,4'-dimethoxytriphenylmethyl)]-7H-pyrrolo[2,3-d]pyrimidin 3'-(Triethylammonium Phosphonat) (15)

a) 7-Desaza-2'-desoxy-7-(1-propinyl-3-trifluoracetamid)-adenosin

5-Jod-2'-desoxytubercidin (14) aus Beispiel 12 wird unter den in Beispiel 13 beschriebenen Bedingungen über 9 h an N-Propargyltrifluoracetamid gekoppelt. Folgende Mengen werden eingesetzt: 5-Jod-2'desoxytubercidin (14) (200 mg, 0.532 mmol), Kupfer(I)jodid (5.0 mg, 0.0236 mmol, 5 mol% ), DMF (3 ml), N-Propargyltrifluoracetamid (482 mg, 3.2 mmol, 6 eq.), Triethylamin (108 mg, 1.06 mmol, 2 eq.), Tetrakis(triphenylphosphin)palladium (0) (61.5 mg, 0.0532 mmol, 10 mol%).
Nach Chromatographie wird der Feststoff aus Ethylactetat umkristallisiert: Blaßgelbe Kristalle (70 mg, 0.176 mmol, 33%). M.p. 187-188˚C. TLC: $R_f$ 0.30 ($CH_2Cl_2$/MeOH 9:1). UV (MeOH) 237 (14 400), 279 (14 200).
$^1$H-NMR ($D_6$-DMSO) 10.07 (s, 1H, NHTFA), 8.12 (s, 1H, H-2), 7.76 (s, 1H, H-6), 6.79 (broad s, 2H, $NH_2$), 6.49 (pt, 1H, H-1', J = 6.6 Hz), 5.25 (d, 1H, 3'-OH, J = 3.0 Hz), 5.05 (t, 1H, 5'-OH, J = 4.5 Hz), 4.35 (m, 1H, H-3'), 4.32 (d, 2H, $CH_2$, J = 4.2 Hz), 3.84 (m, 1H, H-4'), 3.56 (m, 2H, H-5'), 2.47 (m, 1H, H-2'β), 2.19 (m, 1H, H-2'α).
$^{13}$C-NMR ($D_6$-DMSO): 157.4 (C-4), 156.4 und 156.1 (C=O), 152.7 (C-2), 149.2 (C-7a), 126.5 (C-6), 116.8 und 114.6 ($CF_3$), 102.2 (C-4a), 94.0 (C-5), 87.5 (C-4'), 86.7 and 76.2 (C=C), 83.2 (C-1'), 70.9 (C-3'), 61.8 (C-5'), 39.6 (C-2', überlagert von DMSO), 29.9 ($CH_2$).
Anal. berechnet für $C_{16}H_{16}F_3N_5O_4$: C 48.13, H 4.04, N 17.54; gefunden: C 48.26, H 4.13, N 17.58.

b) 4-Benzoylamino-5-(1-propinyl-3-trifluoracetamid)-7-[(2-desoxy-β-D-erythro-pentofuranosyl)-7H-pyrrolo[2,3-d]pyrimidin

Die Einführung der Benzoylaminoschutzgruppe in 7-Desaza-2'-desoxy-7-(1-propinyl-3-trifluoracetamid)-adenosin erfolgt analog zu Beispiel 1.

c) 4-Benzoylamino-5-(1-propinyl-3-trifluoracetamid)-7-[(2-desoxy-β-D-erythro-pentofuranosyl)-5'-O-(4,4'-dimethoxytriphenylmethyl)]-7H-pyrrolo[2,3-d]pyrimidin

Die Einführung der Dmt-Hydroxylschutzgruppe in 4-Benzoylamino-5-(1-propinyl-3-trifluoracetamid)-7-[(2-desoxy-β-D-erythro-pentofuranosyl)-7H-pyrrolo[2,3-d]pyrimidin erfolgt analog zu Beispiel 5.

d) Die Titelverbindung (15) wird analog zu Beispiel 8 aus 4-Benzoylamino-5-(1-propinyl-3-trifluoracetamid)-7-[(2-desoxy-β-D-erythro-pentofuranosyl)-5'-O-(4,4'-dimethoxytriphenylmethyl)]-7H-pyrrolo[2,3-d]pyrimidin hergestellt.

Beispiel 15:

4-Benzoylamino-5-(1-pentinyl-trifluoracetamid)-7-[(2-desoxy-β-D-erythro-pentofuranosyl)-5'-O-14,4'-dimethoxytriphenylmethyl)]-7H-pyrrolo[2,3-d]pyrimidin 3'-(Triethylammonium Phosphonat) (16)

a) 7-Desaza-2'-desoxy-7-(1-pentinyl-trifluoracetamid)-adenosin

5-Jod-2'-desoxytubercidin (14) aus Beispiel 12 wird unter den in Bespiel 13 beschrieben Bedingungen über 48 h an 5-Trifluoracetamid-1-pentin gekoppelt. Folgende Mengen werden eingesetzt: 5-Jod-2'-Desoxytubercidin (200 mg, 0.532 mmol), Kupfer(I)jodid (5 mg, 0.0236 mmol, 5 mol%), DMF (3 ml), 5-Trifluoracetamid-1-pentin (953 mg, 5.32 mmol, 10 eq.), Triethylamin (108 mg, 1.06 mmol, 2 eq.), Tetrakis(triphenylphosphin)-palladium (0) (61.5 mg, 0.0532 mmol, 10 mol%). Nach Chromatographie wird durch Kristallisation aus der Flüssigkeit ein schwach gelblicher öliger Rückstand (84.1 mg, 0.197 mmol, 37%) erhalten. M.p. 51-52°C. TLC: $R_f$ 0.35 ($CH_2Cl_2$/MeOH 9:1). UV (MeOH) max= 239 (14 300), 280 (10 900). $^1$H-NMR ($D_6$-DMSO): 8.08 (s, 1H, H-2), 7.64 (s, 1H, H-6), 6.46 (pt, 1H, H-1', J = 6.9 Hz), 4.32 (m, 1H, H-3'), 3.80 (m, 1H, H-4'), 3.59-3.28 (mehrere m, 5H, H-5',C$\underline{H}_2$-CH$_2$-C$\underline{H}_2$-N), 2.47 (m, 1H, H-2'ß), 2.17 (m, 1H, H-2'a), 1.76 (Quintett, 2H, CH$_2$-C$\underline{H}_2$-CH$_2$-N). $^{13}$C-NMR ($D_6$-DMSO): 157.6 (C-4), 156.5 and 156.2 (C=O), 152.7 (C-2), 149.2 (C-7a), 125.7 (C-6), 118.5 and 114.0 ($CF_3$), 102.3 (C-4a), 95.5 (C-5), 91.6 (C=C, 1"), 87.6 (C-4'), 83.2 (C-1'), 74.0 (C=C, 2"), 71.0 (C-3'), 62.0 (C-5'), 39.6 und 38.6 (C-2'und $CH_2$, überlagert durch DMSO), 27.7 ($CH_2$), 16.6 ($CH_2$). Anal. berechnet für $C_{18}H_{20}N_5O_4F_3$: C 50.59, H 4.72, N 16.39; gefunden: C 50.65, H 4.82, N 16.32.
Die Titelverbindung (16) wird aus 7-Desaza-2'-desoxy-7-(1-pentinyl-trifluoracetamid)-adenosin analog zu Beispiel 14b), 14c) und 14d) erhalten.

Beispiel 16:

2'-Desoxy-7-desaza-7-(2-carboxyethenyl)-adenosin (17).

5-Jod-2'-desoxytubercidin (14) aus Beispiel 12 wird unter den in Bespiel 13 beschrieben Bedingungen über 65 h an Methylacylat gekoppelt. Folgende Mengen werden eingesetzt: 5-Jod-2'-Desoxytubercidin (200 mg, 0.532 mmol), Kupfer(I)jodid (5 mg, 0.0236 mmol, 5 mol%), DMF (3 ml), Triethylamin (108 mg, 1.06 mmol, 2 eq.), Methylacrylat (686 mg, 8.0 mmol, 15 eq.), Tetrakis(triphenylphosphin)palladium (0) (61.5 mg, 0.0532 mmol, 10 mol%). Nach chromatographischer Aufreinigung wird ein blaßgelber Schaum erhalten und nach Waschen mit Dichlormethan werden 71.1 mg Festsubstanz erhalten (40%). M.p. 101-102°C. TLC: $R_f$ 0.40 ($CH_2Cl_2$/MeOH 9:1). UV (MeOH) Max= 268.0 (13 500), 324.8 ( 11 900).
$^1$H-NMR ($D_6$-DMSO): 8.11 (2s, 2H, H-2 and H-6), 7.94 (d, 1H, H-1", J = 15.6 Hz), 6.86 (s,2H, $NH_2$), 6.51 (pt, 1H, H-1', J = 6.6 Hz), 6.4 (d, 1H, H-2", J = 15.6 Hz), 5.26 (d, 1H, 3'-OH, J = 3.6 Hz), 5.04 (t, 1H, 5'-OH, J = 5.1 Hz), 4.36 (m, 1H, H-3'), 3.83 (m, 1H, H-4'), 3.70 (s, 3H, $OCH_3$), 3.55 (m, 1H, H-5'), 2.45 (m, 1H, H-2'ß), 2.22 (m, 1H, H-2'α).
$^{13}$C-NMR ($D_6$-DMSO): 166.9 (C=O), 158.0 (C-4), 152.1 (C-2), 151.2 (C-7a), 137.4 ($\underline{C}$H-C = O), 123.7 (C-6), 115.5 ($\underline{C}$H = CH-C = O), 111.5 (C-5), 101.0 (C-4a), 87.6 (C-4'), 83.2 (C-1'), 70.9 (C-3'), 62.0 (C-5'), 51.2 ($OCH_3$), 39.6 (C-2', überlagert durch DMSO).
Anal. berechnet für $C_{15}H_{18}N_4O_5$: C 53.89, H 5.43, N 16.76; gefunden: C 53.79, H 5.56, N 16.74.

Beispiel 17:

7-[2-Desoxy-3,5-di-O-(2-methylpropionyl)-β-D-erythro-pentofuranosyl]-4-methoxy-2-[(formyl)amino]-7H-pyrrolo[2,3-d] pyrimidin (18).

7-(2-Desoxy-β-D-erythro-pentofuranosyl)-4-methoxy-2-[(formyl)amino]-7H-pyrrolo[2,3-d]pyrimidin (1.0 g, 3.3 mmol) [F. Seela, H. Driller, Nucleosides, Nucleotides 1989, 8, 1-21] in Acetonitril (20 ml) wurde bei Raumtemperatur mit Isobuttersäureanhydrid (33 mmol) in Gegenwart von Triethylamin (23 mmol) 15 Stunden lang gerührt. Das Lösungsmittel wird abgedampft und mit Methanol nachgedampft. Der Rückstand wird im Laufmittel Methylenchlorid/Aceton (95: 5) chromatographiert, die Hauptzone isoliert und der Inhaltsstoff aus Cyclohexan umkristallisiert. 1.26 g (89 %) farbloser Feststoff.
$^1$H NMR ([$D_6$]DMSO), δ: 1.05 - 1.14 (m, 4$CH_3$), 2.60, 2.90 (m, CH und 2'-Ha,b), 4.02 (S, $OCH_3$), 4.16 (m, 5'-H), 4.26 (m, 4'-H), 5.35 (m, 3'-H), 6.47 (m, 1'-H), 6.52 (d, 5-H), 7.39 (d, 6-H). 9.44 (d, NH), 10.71 (d, HCO).

Beispiel 18:

5-Brom-7-[2-desoxy-3,5-di-O-(2-methylpropionyl)-β-D-erythro-pentofuranosyl]-4-methoxy-2-[(formyl)-amino]-7H-pyrrolo[2,3-d]pyrimidin (19).

Eine Lösung von Verbindung 18 (10.1 mmol) in Dimethylformamid wurde mit H-Bromsuccinimid (10.1 mmol) bei Raumtemperatur 1 Stunde gerührt. Zum Abpuffern wird die Lösung mit einigen Tropfen 5 %igem wässrigem $HaHCO_3$ versetzt und dann Methylenchlorid hinzugefügt. Die organische Phase wird mit Wasser gegengeschüttelt, separiert, über Natriumsulphat getrocknet und abgedampft. Die Chromatographie des Rückstandes an einer Kieselgelsäule im Laufmittel Dichlormethan/Aceton (95:5) führt zu zwei Zonen. Der Abdampfrückstand der langsam wandernden Hauptzone liefert farbloses (19) (75 %) als Feststoff.
$^1$H NMR ([$D_6$]DMSO), δ: 1.05 - 1.14 (m, $4CH_3$), 2.60, 2.88 (m, CH und 2'-Ha,b), 4.04 (s, $OCH_3$), 4.16 (m, 5'-H), 4.22 (m, 4'-H), 5.34 (m, 3'-H), 6.46 (m, 1'-H), 7.60 (s, 6-H), 9.43 (s, NH), 10.86 (s, HCO).

Aus der schnell wandernden Nebenzone der oben genannten Reaktion wird ein farbloser Feststoff erhalten der als 5,6-Dibrom-7-[2-desoxy-3,5-di-O-(2-methylpropionyl)-β-D-erythro-pentofuranosyl]-4-methoxy-2-[(formyl)amino]-7H-pyrrolo[2,3-d]pyrimidin charakterisiert wurde.
$^1$H NMR ([$D_6$]DMSO), δ. 0.99 - 1.13 (m, $4CH_3$), 2.59, 3.58 (m, CH und 2'-Ha,b), 4.03 (s, $OCH_3$), 4.16 (m, 5'-H), 4.30 (m, 4'-H), 5.56 (m, 3'-H), 6.39 (m, 1'-H), 9.42 (s, NH), 10.91 (s, HCO).

Beispiel 19:

5-Chlor-7-[2-desoxy-3,5-di-O-(2-methylpropionyl)-β-D-erythro-pentofuranosyl]-4-methoxy-2-amino-7H-pyrrolo[2,3-d] pyrimidin (20).

Die Substanz wurde unter Verwendung von N-Chlorsuccinimid analog der Verbindung (19) dargestellt und aufgearbeitet. Die Reaktionszeit der Halogenierung betrug 8 Stunden. Als Lösungsmittel wurde Acetonitril/DMF (4:1) verwandt. Farbloser Feststoff (70 %).
$^1$H NMR ([$D_6$]DMSO), δ: 1.07 - 1.13 (m, $4CH_3$), 2.59, 2.74 (m, CH und 2'-Ha,b), 3.93 (s, $OCH_3$), 4.15 (m, 5'-H), 4.21 (m, 4'-H), 5.25 (m, 3'-H), 6.39 (m, 1'-H), 6.47 (5, $NH_2$), 7.20 (s, 6-H).

Als Nebenprodukt entstand als farbloser Feststoff die Verbindung 5,6-Dichlor-7-[2-desoxy-3,5-di-O-(2-methylpropionyl)-β-D-erythro-pentofuranosyl]-4-methoxy-2-amino-7H-pyrrolo[2,3-d]pyrimidin.
$^1$H NMR ([$D_6$]DMSO), δ: 1.03 - 1.13 (m, $4CH_3$), 2.58, 3.40 (m, CH und 2'-Ha,b), 3.93 (s, $OCH_3$), 4.16 (m, 5'-H), 4.37 (m, 4'-H), 5.44 (m, 3'-H), 6.37 (m, 1'-H), 6.58 (s, $NH_2$).

Beispiel 20:

2-Amino-7-(2'desoxy-β-erythro-pentofuranosyl)-3,7-dihydro-5-brom-4-pyrrolo[2,3-d]pyrimidin-4-on (21)

Die Verbindung (19) wird nach einem bekannten Verfahren umgesetzt [F.Seela, B. Westermann, U. Bindig, J. Chem. Soc. Perkin Trans I 1988, 699]. 500 mg der Verbindung (19) wird in 200 ml 2N NaOH 3 Stunden unter Rückfluß erhitzt. Die abgekühlte Lösung wird mit Eisessig neutralisiert, der anorganische Rückstand wird abfiltriert und die wässerige Phase abgedampft. Umkristallisation aus Wasser liefert farblose Kristalle von (21).

Beispiel 21:

2-Amino-7-(2'desoxy-β-erythro-pentofuranosyl)-3,7-dihydro-5-chlor-4-pyrrolo[2,3-d]pyrimidin-4-on (22)

Die Verbindung (22) wird ausgehend von der Verbindung (20) analog zu dem im Beispiel 20 beschriebenen Verfahren hergestellt. Umkristallisation aus Wasser liefert farblose Kristalle von (22).

Beispiel 22:

4-Amino-7-[2-desoxy-β-D-erythro-pentofuranosyl]-5-trimethylsilylethinyl-7H-pyrrolo[2,3-d]pyrimidin (23)

Verbindung (23) wird nach der allgemeinen Vorschrift der Kreuzkupplungsreaktion in Beispiel 13 aus Trimethylsilylacetylen dargestellt. Farbloser Feststoff. Ausbeute 54%

Ber. C 55.47, H 6.40, N 16.17. Gef. C 55.57, H 6.53, N 16.20.
$^1$H NMR (DMSO): 8.12 (s, 1H, H-2), 7.80 (s, 1H, H-6), 6.76 (breit, 2H, $NH_2$), 6.47 ("t",1H, H-1', J = 6.7 Hz), 5.23 (d, 1H,

3'-OH, J = 3.3 Hz), 5.07 (t, 1H, 5'-OH), 4.33 (m, 1H, H-3')" 3.87 (m, 1H, H-4'), 3.54 (m, 2H, H-5'), 2.46 (m, 1H, H-2'), 2.17 (m, 1H, H-2'), 0.73 (s, 9H,Me).

Beispiel 23:

4-Amino-7-[2-desoxy-β-D-erythro-pentofuranosyl]-5-ethinyl-7H-pyrrolo[2,3-d]pyrimidin (24)

200 mg der Verbindung (23) werden in 20 ml MeOH gelöst. Zugabe von 8mg $K_2CO_3$ führt nach 1 h Rühren zur Hydrolyse. Nach Einrotieren der Lösung wird der Rückstand an Kieselgel im Laufmittel Methylenchlorid/MeOH (8:1) chromatographiert. Umkristallisationn aus MeOH führt zu farblosen Kristallen (73%).
Ber. C 56.93, H 5.15, N 20.43. Gef. C 56.77, H5.71, N 20.42.
$^1$H NMR (DMSO): 8.13 (s, 1H, H-2), 7.81 (s, 1H, H-6), 6.65 (breit, 2H, $NH_2$), 6.49 (t, 1H, H-1')" 5.25 (m, 1H, 3'-OH), 5.05 (m, 1H, 5'-OH), 4.36 (m, 1H, H-3'), 4.26 (s, 1H, Ethin), 3.84 (s, 1H, H-4'), 3.56 (m, 2H, H-5'), 2.47 (m, 1H, H-2'), 2.21 (m, 1H, H-2').

Beispiel 24

4-Amino-7-[2-desoxy-β-D-erythro-pentofuranosyl]-5-hexinyl-7H-pyrrolo[2,3-d]pyrimidin (25)

Verbindung (25) wird nach der allgemeinen Vorschrift der Kreuzkupplungsreaktion (Bsp. 13) unter Verwendung von 1-Hexin hergestellt. Umkristallisation aus MeOH führt zu farblosen Kristallen (Ausbeute: 48%).
Ber. C 61.80, H 6. 71, N 19.96; Gef. C 61.68, H 6.60, N 16.90
$^1$ H NMR (DMSO) 8.31 (s, 1H, H-2), 7.65 (s, 1H, H-6), 6.65 (breit, 2H, $NH_2$), 6.49 ("t", 1H, H-1'), 5.24 (m, !H, 3'-OH), 5.05 (m, 1H, 5'-OH), 4.50 (m, 1H, 3'-H), 3.84 (m, 1H, H-4'), 3.56 (m, 2H, H-5'), 2.48 (m, 2H, $CH_2C$=), 2.46 (m, 1H, H-2'), 2.18 (m, 1H, H-2') 1.54 (m, 2H, $CH_2$), 1.43 (m, 2H, $CH_2$), = 0.93 (m, 2H, $CH_3$).

Beispiel 25:

2-Amino-6-chlor-7[2-desoxy-3, 5-di-O-acetyl-β-D-erythropentofuranosyl]-4-methoxy-7H-pyrrolo[2,3-d]pyrimidin.

Eine Lösung von 50 mg (0.14 mmol) 2-Amino-7(2-desoxy-3,5-di-O-acetyl-β-D-erythro-pentofuranosyl)-4-methoxy-7H-pyrrolo[2,3-d]pyrimidin in 3 ml Dichlormethan werden mit 36.6 mg (0.27 mmol) N-Chlorsuccinimid versetzt und 12 h bei Raumtemperatur gerührt. Das Lösungsmittel wird abgezogen und der Rückstand an Kieselgel in $CH_2Cl_2$/Aceton (9:1) chromatographiert.
Aus der langsam wandernden Hauptzone erhält man 30 mg (54 %) farblosen Schaum.
$^1$H-NMR $D_6$ (DMSO): 1.99 (s, 3H, $CH_3$), 2.09 (s, 3H, $CH_3$), 2.37 (m, 1H, H-2'b), 3.93 (s, 3H, $OCH_3$), 4.18 (m, 2H, H-5'), 4.43 (m, 1H, H-4), 5.44 (m, 1H, H-3'), 6.38 (m, 1H, H-1'), 6.42 (s, 1H, H-5).
$^{13}$C-NMR (DMSO): 20.48 ($CH_3$), 20.76 ($CH_3$), 33.78 (C-2'), 52.99 ($OCH_3$), 63.47 (C-5'), 74.31 (C-3'), 80.91 (C-4'), 82.95 (C-1'), 96.45 (C-4a), 98.65 (C-5), 118.12 (C-6), 153.69 (C-7a), 159.25 (C-2), 162.16 (C-4), 169.98 (C=O), 170.09 (C=O).

Beispiel 26:

2-Amino-5,6-dichlor-7[2-desoxy-3,5-di-O-acetyl-β-D-erythropentofuranosyl]-4-methoxy-7H-pyrrolo[2,3-d]pyrimidin.

Die schneller wanderne Zone liefert einen farblosen Schaum. 6 mg (9.9 %).
$^1$H-NMR (DMSO): 1.98 (s, 3H, $CH_3$), 2.07 (s, 3H, $CH_3$), 2.24 (m, 1H, H-2'b), 2.73 (m, 1H, H-2'a), 3.94 (s, 3H, $OCH_3$), 4.14 (m, 2H, H-5'), 4.39 (m, 1H, H-4), 5.40 (m, 1H, H-3'), 6.39 (m, 1H, H-1'), 6.59 (s, 2H, $NH_2$).
$^{13}$C-NMR (DMSO): 20.47 ($CH_3$), 20.75 ($CH_3$), 34.06 (C-2'), 53.34 ($OCH_3$), 63.41 (C-5'), 74.09 (C-3'), 81.01 (C-4'), 83.26 (C-1'), 94.59 (C-4a), 102.08 (C-5), 115.16 (C-6), 151.94 (C-7a), 159.59 (C-2), 162.08 (C-4), 169.97 (C=O), 170.07 (C=O).
UV (MeOH): $\lambda_{max}$ (ε): 290 nm (8500), 264 nm (13100), 226 nm (22700).

Beispiel 27:

7-(2-Desoxy-β-D-erythro-pentofuranosyl)-4[(dimethylamino)methyliden]amino-5-lod-7H-pyrrolo[2,3-d]pyrimidin (26)

Eine Lösung von 5-Iod-2'-Desoxytubercidin (14) (400 mg, 1.06 mmol) in Methanol (20 ml) wird mit N,N-Dimethylformamid Dimethylacetal (2.0 g, 16.8 mmol) für 2h bei 40°C gerührt. Nach Abdampfen des Lösungsmittels wird der Rest über Flashchromatographie (FC) (Säule: 20 x 5 cm, $CH_2Cl_2$/MeOH 9 : 1) aufgereinigt. Die Hauptzone liefert einen

farblosen Schaum (389 mg, 85 %). TLC (Dünnschichtchromatographie, Silicagel, $CH_2Cl_2$/MeOH 9 :1): $R_f$ 0.46. UV (MeOH): max = 229 nm (17400), 277 nm (10400), 323 nm (19000). $^1$H-NMR ($D_6$)DMSO): 2.18 (m, 1H, $H_a$-2'); 2.47 (m, 1H, $H_\beta$-2', überlagert von DMSO); 3.18, 3.22 (2s, 6H, $N(CH_3)_2$); 3.54 (m, 2H, H-5'); 3.81 (m, 1H, H-4'); 4.32 (m, 1H, H-3'); 5.00 (t, J = 5.4 Hz, 1H, 5'-OH); 5.23 (d, J = 3.9 Hz, 1H, 3'-OH); 6.52 ("t", J = 7.0 Hz, 1H, H-1'); 7.70 (s, 1H, H-6); 8.30 (s, 1H, H-2); 8.82 (s, 1H, N=CH). Anal. berechnet für $C_{14}H_{18}N_sO_3I$: C 38.99, H 4.21, N 16.24; gefunden: C 39.09, H 4.27, N 16.10.

Beispiel 28:

7-(2-Desoxy-β-D-erythro-pentofuranosyl)-4-[(dimethylamino)methyliden]amino-5-hexinyl-7H-pyrrolo[2,3-d]pyrimidin (27)

Eine Lösung von 5-Hexinyl-2'-desoxytubercidin (25) (400 mg, 1.21 mmol) in Methanol (20 ml) wird mit N,N-Dimethylformamid Dimethylacetal (2.0 g, 16.8 mmol) für 2h bei 40°C gerührt. Nach Abdampfen des Lösungsmittels wird der Rest über Flash-Chromatographie (FC) (Säule: 20 x 5 cm, $CH_2Cl_2$/MeOH 9 : 1) aufgereinigt. Die Hauptzone liefert einen farblosen Schaum (373 mg, 80 %). TLC (Dünnschichtchromatographie, Silicagel, $CH_2Cl_2$/MeOH 9 :1): $R_f$ 0.51. UV (MeOH): max = 278 (12100), 321 (14300). $^1$H-NMR ($D_6$-DMSO): δ = 0.91 (t, J = 7.3 Hz, 3H, $CH_3$); 1.45 (Sextett, J = 7.2 Hz, 2H, $CH_2$-$CH_3$); 1.53 (Quintett, J = 7.3 Hz, 2H, $CH_2CH_2$-$CH_3$); 2.18 (m, 1H, $H_\alpha$-2'); 2.47 (m, 3H, $H_\beta$-2', C≡C-$CH_2$, überlagert von DMSO); 3.16, 3.18 (2s, 6H, $N(CH_3)_2$); 3.56 (m, 2H, H-5'); 3.83 (m, 1H, H-4'); 4.35 (m, 1H, H-3'); 5.02 (t, J = 5.5 Hz, 1H, 5'-OH); 5.26 (d, J = 3.9 Hz, 1H, 3'-OH); 6.50 ("t", J = 7.0 Hz, 1H, H-1'); 7.64 (s, 1H, H-6); 8.32 (s, 1H, H-2); 8.76 (s, 1H, N =CH).

Beispiel 29:

7-[2-Desoxy-5-O-(4,4'-dimethoxytriphenylmethyl)-β-D-erythro-pentofuranosyl]-4-[(dimethylamino)methyliden]amino-5-Iod-7H-pyrrolo[2,3-d]pyrimidin (28)

Zu einer Lösung von Verbindung (26) (300 mg, 0.70 mmol) in getrockneten Pyridin (3 ml) wird 4,4'-Dimethoxytriphenylmethylchlorid (256 mg, 0.76 mmol) unter Argon-Atmosphäre hinzugegeben. Nach Rühren bei 50°C für 2 h wird eine 5% wässrige $NaHCO_3$-Lösung (15 ml) hinzugegeben. Die wässrige Phase wird mit $CH_2Cl_2$ extrahiert (3 mal, jeweils 50 ml). Die vereinten organischen Phasen werden über $Na_2SO_4$ getrocknet und anschließend eingedampft. Nach Flash-Chromatographie (FC) (Säule: 20 x 5 cm, B) des Restes wird ein farbloser Schaum erhalten (360 mg, 70 %). TLC (Silicagel, B): $R_f$ 0.60. UV (MeOH) max = 236 (38400), 275 (14200), 322 (18900). $^1$H-NMR ($D_6$)DMSO): 2.24 (m, 1H, $H_a$-2'); 2.47 (m, 1H, $H_\beta$-2', überlagert durch DMSO); 3.18 (m, 2H, H-5'), überlagert durch $NaCH_3$); 3.18, 3.22 (2s, 6H, $N(CH_3)_2$); 3.72 (s, 6H, 2 $OCH_3$); 3.92 (m, 1H, H-4'); 4.37 (m, 1H, H-3'); 5.30 (d, J = 4.0 Hz, 1H, 3'-OH); 6.54 ("t", J = 6.6 Hz, 1H,H-1'); 6.84 (m, 4H, aromat. H); 7.22 - 7.38 (m, 9H, aromat. H); 7.56 (s, 1H, H-6); 8.31 (s, 1H, H-2); 8.82 (s, 1H, N =CH). Anal. berechnet für $C_{35}H_{36}N_5O_5I$: C 57.30, H 4.95, N 9.55;
gefunden: C 57.48, H 5.12, N 9.44.

Beispiel 30:

7-[2-Desoxy-5-O-(4,4'-dimethoxytriphenylmethyl)-β-D-erythro-pentofuranosyl]-4-[(dimethylamino)methyliden]amino-5-hexinyl-7H-pyrrolo[2,3-d]pyrimidin (29)

Zu einer Lösung von Verbindung (27) (300 mg, 0.78 mmol) in getrockneten Pyridin (3 ml) wird 4,4'-Dimethoxytriphenylmethylchlorid (290 mg, 0.86 mmol) unter Argon-Atmosphäre hinzugegeben. Nach Rühren bei 50°C für 2 h wird eine 5% wässrige $NaHCO_3$-Lösung (15 ml) hinzugegeben. Die wässrige Phase wird mit $CH_2Cl_2$ extrahiert (3 mal, jeweils 50 ml). Die vereinten organischen Phasen werden über $Na_2SO_4$ getrocknet und anschließend eingedampft. Nach Flash-Chromatographie (FC) (Säule: 20 x 5 cm, B) wird ein farbloser Schaum erhalten (360 mg, 62 %). TLC (Silicagel, B): $R_f$ 0.60. UV (MeOH) max = 276 (17500), 320 (12900). $^1$H-NMR ($D_6$-DMSO): δ = 0.91 (t, J = 7.3 Hz, 3H, $CH_3$); 1.45 (Sextett, J = 7.2 Hz, 2H, $CH_2$-$CH_3$); 1.53 (Quintett, J = 7.3 Hz, 2H, $CH_2$-$CH_2$-$CH_3$); 2.18 (m, 1H, $H_\alpha$-2'); 2.47 (m, 3H, $H_\beta$-2', C ≡ C-$CH_2$, überlagert durch DMSO); 3.16, 3.18 (2s, 6H, $N(CH_3)_2$); 3.18 (m, 2H, H-5', überlagert durch $N(CH_3)_2$); 3.71 (s, 6H, 2 $OCH_3$); 3.91 (m, 1H, H-4'); 4.34 (m, 1H, H-3'); 5.28 (d, J = 3.9 Hz, 1H, 3'-OH); 6.53 ("t", J = 7.0 Hz, 1H, H-1'); 6.82 (m, 4H, aromat. H); 7.20 - 7.36 (m, 9H, aromat. H); 7.56 (s, 1H, H-6); 8.30 (s, 1H, H-2); 8.97 (s, 1H, N =CH).

Beispiel 31:

7-[2-Desoxy-5-0-(4,4'-dimethoxytriphenylmethyl)-β-D-erythro-pentofuranosyl)-4-[(dimethylamino)methyliden]amino-5-Iod-7H-pyrrolo[2,3-d]pyrimidin-3'-[(2-cyanoethyl)-N,N-diisopropyl-phosphoramidit (30)

Zu einer gerührten Lösung des getrockneten Nukleosids (28) (300 mg, 0.41 mmol) und wasserfreien N,N-Diisopropylethylamin (212 mg, 1.64 mmol) in getrockneten THF (2 ml) werden unter Argon-Atmosphäre Chlor-(2-cyanethoxy)-N,N-Diisopropylamino-phosphin(194 mg, 0.82 mmol) hinzugegeben. Der Ansatz wird 30 Minuten gerührt und anschließend filtriert. Das Filtrat wird mit Ethylacetat (30 ml) versetzt und zweimal mit eiskalter wässriger 10% $Na_2CO_3$-Lösung (2 x 10 ml) und 10 ml Wasser extrahiert. Die organischen Phasen werden über wasserfreien $Na_2SO_4$ getrocknet und anschließend eingedampft. Nach Flash-Chromatographie (FC) (Säule: 10 x 3 cm, Petrolether/Aceton) wird ein farbloser Schaum erhalten (222 mg, 60 %). TLC (Silica Gel, Petrolether/Aceton 1 : 1): $R_f$ 0.38, 0.45. $^{31}$P-NMR $CDCl_3$: 149.0, 149.2.

Beispiel 32:

7-[2-Desoxy-5-O-(4,4'-dimethoxytriphenylmethyl)-β-D-erythro-pentofuranosyl]-4-[(dimethylamino)methyliden]amino-5-hexinyl-7H-pyrrolo[2,3-d]pyrimidine-3'-[(2-cyanoethyl)-N,N-diisopropyl-phosphoramidit (31)

Zu einer gerührten Lösung des getrockneten Nukleosids 29 (300 mg, 0.44 mmol) und wasserfreien N,N-diisopropylethylamin (212 mg, 1.64 mmol) in getrockneten THF (2 ml) werden unter Argon-Atmosphäre Chlor-(2-cyanethoxy)-N,N-diisopropylamino-phosphin (194 mg, 0.82 mmol) hinzugegeben. Der Ansatz wird 30 Minuten gerührt und anschließend filtriert. Das Filtrat wird mit Ethylacetat (30 ml) versetzt und zweimal mit eiskalter wässriger 10% $Na_2CO_3$-Lösung (2 x 10 ml) und 10 ml Wasser extrahiert. Die organischen Phasen werden über wasserfreien $Na_2SO_4$ getrocknet und anschließend eingedampft. Nach Flash-Chromatographie (FC) (Säule: 10 x 3 cm, Petrolether/Aceton) wird ein gelber Schaum erhalten (229 mg, 60 %). $R_f$ 0.45, 0.53. $^{31}$P-NMR $CDCl_3$ : 149.0, 149.3.

Beispiel 33:

4-Amino-7-(2-desoxy-β-D-erythro-pentofuranosyl)-5-methylthio-7H-pyrrolo[2,3-d]pyrimidin (32)

Ausgehend von 2',3',5'-tri-O-acetyl-7-desazaadenosin wird durch Umsetzung dieser Verbindung mit Thiocyanogenchlorid in Essigsäure das entsprechende 5-Thiocyanat-Derivat gebildet. Reduktion mit 2-Mercaptoethanol und anschließende Methylierung liefert dass 5-Methylthio-Derivat mit einem ungeschützten Zuckerrest (Watanabe et al., Nucleosides & Nucleotides, 1 (2), 191-203, 1982). Die Methylthioverbindung (32) erhält man durch selektive Silylierung der 3',5'-OH-Gruppen und anschließender Barton-Desoxygenierung über den entsprechenden Thionoester.

Beispiel 34:

4-Amino-7-(2-deoxy-β-D-erythro-pentofuranosyl)-5-morpholinomethyl-7H-pyrrolo-[2,3-d]pyrimidine (33)

Die Herstellung von (33) ist möglich durch Anwendung der Mannich Reaktion. Durch Erhitzen von Tubercidin zusammen mit Paraformaldehyd und Morpholin in DMF erhält man das 5-Morpholinomethyl-Derivat (Watanabe et al., Nucleosides & Nucleotides, 1 (2), 191-203, 1982). Das 5-Morpholin Derivat (33) wird entsprechend der in Bsp. 33 beschrieben Silylierung und Desoxigenierung erhalten.

Beispiel 35:

4-Amino-7-(2-deoxy-β-D-erythro-pentofuranosyl)-5-trifluoromethyl-7H-pyrrolo[2,3-d]pyrimidin (34)

Verbindung 34 wird durch Reaktion von Verbindung (14) mit $CF_3Cu$ entsprechend der Vorschrift von Nair et al. (J. Am. Chem. Soc., 111, 8502-8504, 1989) erhalten.

Beispiel 36:

4-Amino-7-(2-deoxy-β-D-erythro-pentofuranosyl)-5-nitro-7H-pyrrolo[2,3-d]pyrimidin (35) und 4-Amino-7-(2-deoxy-β-D-erythro-pentofuranosyl)-6-nitro-7H-pyrrolo[2,3-d]pyrimidin (36)

Durch Behandlung von 2',3',5'-tri-O-acetyl-7-desazaadenin mit $HNO_3/H_2SO_4$ in Methylenchlorid erhält man eine Mischung aus 5- bzw. 6-substituierten Nitroderivate. Als Hauptprodukt entstehen 5-Nitro Derivate (Watanabe et al., Nucleosides & Nucleotides, 1 (2), 191-203, 1982). Deacylierung, 3',5'-OH Silylierung und 2'-Deoxygenierung führt zu den Verbindungen (35) und (36).

Beispiel 37:

4-Amino-7-(2-deoxy-β-D-erythro-pentofuranosyl)-6-cyano-7H-pyrrolo[2,3-d]pyrimidin (37)

Oxidation von Verbindung (32) führt zum 5-Methylsulfon-Derivat. Behandlung mit NaCN in DMF liefert das 6-Cyano Derivat (37), ein Regioisomer des Toyocamycins (Watanabe et al., Nucleosides & Nucleotides, 1 (2), 191-203, 1982). Silylierung und Deoxygenierung erfolgt gemäß Bsp. 33.

Beispiel 38:

4-Amino-7-(2-deoxy-β-D-erythro-pentofuranosyl)-5-carboxy-7H-pyrrolo[2,3-d]pyrimidin (38)

Verbindung (38) wird durch Hydrolyse von Verbindung (37) erhalten.

Beispiel 39:

4-Amino-5,6-dibrom-7-(2-deoxy-β-D-erythro-pentofuranosyl)-7H-pyrrolo[2,3-d]pyrimidin (66)

Methode A: Zu einer Lösung von 2'-Desoxytubercidin (1.0 g, 4.0 mmol) und NaOAc (0.78 g, 8.0 mmol) in getrocknetem DMF (10 ml) wird bei Raumtemperatur NBS (1.42 g, 8.0 mmol, gelöst in 4 ml in getrocknetem DMF) gegeben. Die rote Lösung wird 10 Minuten gerührt und anschließend eingedampft. Nach Flash-Chromatographie (Säule: 20 x 5 cm, $CH_2Cl_2$/MeOH 9 : 1), Eindampfen der schneller wandernden Zone und anschließender Umkristallisation aus Isopropanol erhält man Titelverbindung (38) als farblose Kristalle (400 mg, 25%). Isolierung der langsamer wandernden Zone liefert 5-Brom-2'-deoxytubercidin (130 mg, 10 %). Methode B: Zu einer Lösung von 5-Brom-2'-deoxytubercidin (1.3 g, 4.0 mmol) und NaOAc (0.785 g, 8.0 mmol) in getrocknetem DMF (10 ml) wird eine Lösung von NBS (1.42 g, 8.0 mmol, in 4 ml getrocknetem DMF gelöst) gegeben. Aufreinigung, siehe Methode A. Ausbeute: 490 mg, 30 % of 21. Schmelzpunkt: 181˚C. TLC (Silicagel, $CH_22Cl_2$/MeOH 9 : 1): $R_f$ 0.40. $^1H$-NMR ($D_6$-DMSO): $\delta$ = 2.12 (m, $H_a$-2'); 2.50 (m, $H_\beta$-2'); 3.51 (m, 2H, H-5'); 3.84 (m, 1H, H-4'); 4.44 (m, 1H, H-3'); 5.20, (br, 2H, 5'-OH, 3'-OH); 6.42 ("t", J = 6.2 Hz, 1H, H-1'); 6.93 (br, 2H, $NH_2$); 8.10 (s, 1H, H-2).

Beispiel 40:

4-Amino-5,6-dichlor-7-(2-deoxy-β-D-erythro-pentofuranosyl)-7H-pyrrolo[2,3-d]pyrimidin (39)

Die Titelverbindung (39) erhält man aus dem 5-Chlornucleosid (1) durch Chlorierung mit N-Chlorsuccinimid.

Beispiel 41:

4-Amino-5-iod-6-chlor-7-(2-deoxy-β-D-erythro-pentofuranosyl)-7H-pyrrolo[2,3-d]-pyrimidin (40)

Die Titelverbindung (40) erhält man aus dem 5-Iodnucleosid (14) durch Chlorierung mit N-Chlorsuccinimid.

Beispiel 42:

4-Amino-5-iod-6-brom-7-(2-deoxy-β-D-erythro-pentofuranosyl)-7H-pyrrolo-[2,3-d]pyrimidin (41)

Die Titelverbindung (41) erhält man aus dem 5-Iodnucleosid (14) durch Bromierung mit N-Bromsuccinimid.

Beispiel 43:

4-Amino-5-brom-6-iod-7-(2-deoxy-β-D-erythro-pentofuranosyl)-7H-pyrrolo-[2,3d]pyrimidin (42)

Die Titelverbindung (42) erhält man aus dem 5-Bromnucleosid (2) durch lodierung mit N-Iodsuccinimid.

Beispiel 44:

4-Amino-5-chlor-6-brom-7-(2-deoxy-β-D-erythro-pentofuranosyl)-7H-pyrrolo-[2,3-d]pyrimidin (43)

Die Titelverbindung (43) erhält man aus dem 5-Chlornucleosid (1) durch Bromierung mit N-Bromsuccinimid.

Beispiel 45:

4-Amino-5-chlor-6-iod-7-(2-deoxy-β-D-erythro-pentofuranosyl)-7H-pyrrolo-[2,3-d]pyrimidin (44)

Die Titelverbindung (44) erhält man aus dem 5-Chlornucleosid (2) durch Iodierung mit N-Iodsuccinimid.

Beispiel 46:

5-Chlor-7-[2-deoxy-3,5-di-O-(2-methylpropionyl)-β-D-erythro-pentofuranosyl]-2-[(formyl)amino]-4-methoxy-7H-pyrrolo-[2,3-d]pyrimidin (45)

Zu einer Lösung von Verbindung (18) (300 mg, 0.67 mmol in 5ml DMF) wird N-chlorsuccinimid (87 mg, 0.67 mmol) gegeben. Nach Rühren (20 h bei Raumtemperatur) wird die Lösung zu einer Mischung von $CH_2Cl_2$/5 % aq. $NaHCO_3$ (50 ml, 9 : 1) zugegeben. Die organische Schicht wird abgetrennt, mit Wasser gewaschen, über $Na_2SO_4$ getrocknet, filtriert und eingedampft. Der eingedampfte Rest wird in $CH_2Cl_2$ gelöst und über Silikagel chromatographiert (Säule: 5 x 20 cm, $CH_2Cl_2$/Aceton 95 : 5). Die Hauptzone wird eingeengt und mit n-Hexan versetzt. Die präzipitierten farblosen Kristalle (230 mg, 71 %) werden isoliert. Schmelzpunkt: 119-120°C. $^1H$-NMR ([$D_6$]DMSO): $\delta$ = 1.09 (d, J = 7.0, $CH_3$), 1.15 (d, J = 6.9, $CH_3$), 2.45, 2.60, 2.63, 2.89 (4m, CH and $H_{a,\beta}$-C(2')), 4.06 (s, $OCH_3$), 4.18 (m, H-C(5')), 4.28 (m, H-C(4')), 5.35 (m, H-C(3')), 6.47 (m, H-C(1')), 7.58 (s, H-C(6)), 9.45 (d, J = 8.9, NH), 10.84 (d, J = 9.6, HCO). Anal. berechnet für $C_{21}H_{27}ClN_4O_7$ (482.9): C 52.23, H 5.64, N 11.60; gefunden: C 52.51, H 5.69, N 11.65.

Beispiel 47:

7-[2-Deoxy-3,5-di-O-(2-methylpropionyl)-ß-D-erythro-pentofuranosyl]-2-[(formyl)aminol-5-iod-4-methoxy-7H-pyrrolo-[2,3-d]pyrimidine (46)

Verbindung (46) wird ausgehend von Verbindung (18) (500 mg, 1.11 mmol) und N-Iodsuccinimid (264 mg, 1.16 mmol) wie für Verbindung (45) beschrieben hergestellt. Die Reaktiosdauer beträgt 23 h. Farblose Kristalle (590 mg, 92%) werden aus Cyclohexan erhalten. $^1H$-NMR ([$D_6$]DMSO): $\delta$ = 1.9 (d, J = 7.0, $CH_3$), 1.15 (d, J = 7.0, $CH_3$), 2.46, 2.60, 2.89 (3 m, CH and $H_{\alpha,\beta}$-C(2')), 4.06 (s, $OCH_3$), 4.17 (m, H-C(5')), 4.27 (m, H-C(4')), 5.35 (m, H-C(3')), 6.46 (m, H-C(1')), 7.62 (s, H-C(6)), 9.55 (d, J = 9.7, NH), 10.81 (d, J = 9.9 HCO). Anal. berechnet für $C_{21}H_{27}JN_4O_7$(574.4): C 43.91, H 4.74, N 9.75; gefunden: C 43.98, H 4.75, N 9.82.

Beispiel 48:

6-Chlor-7-[2-deoxy-3,5-di-O-(2-methylpropionyl)-β-D-erythro-pentofuranosyl]-2-[(formyl)amino]-5-iod-4-methoxy-7H-pyrrolo-[2,3-d]pyrimidin (47)

Die Titelverbindung (47) erhält man aus dem 5-lodnucleosid (46) durch Chlorierung mit N-Chlorsuccinimid.

Beispiel 49:

5-Brom-6-chlor-7-[2-deoxy-3,5-di-O-(2-methylpropionyl)-β-D-erythro-pentofuranosyl]-2-[(formyl)amino-4-methoxy-7H-pyrrolo-[2,3-d]pyrimidin (48)

Die Titelverbindung (48) erhält man aus dem 5-Bromnucleosid (19) durch Chlorierung mit N-Chlorsuccinimid.

Beispiel 50:

7-[2-Deoxy-3,5-di-O-(2-methylpropionyl)-β-D-erythro-pentofuranosyl]-5,6-dichlor-2-[(formyl)amino]-4-methoxy-7H-pyrrolo-[2,3-d]pyrimidin (49)

Zu einer Lösung von Verbindung (45) (200 mg, 0.4 mmol in 5ml DMF) wird N-chlorsuccinimid (117 mg, 0.9 mmol) gegeben. Nach Rühren (16 h bei Raumtemperatur) wird die Lösung zu einer Mischung von $CH_2Cl_2$/5 % aq. $NaHCO_3$ (50 ml, 9 : 1) zugegeben. Die organische Phase wird abgetrennt, mit Wasser gewaschen, über $Na_2SO_4$ getrocknet, filtriert und eingedampft. Der eingedampfte Rest wird in $CH_2Cl_2$ gelöst und über Silikagel chromatographiert (Säule: 5 x 20 cm, $CH_2Cl_2$/Aceton 95 : 5) Die Hauptzone wird abgetrennt. Nach Abdampfen des Lösungsmittel und Auskristallisation aus Cyclohexan werden farblose Kristalle erhalten (149 mg, 72%). Schmelzpunkt: 127-128°C. $^1$H-NMR ([$D_6$]DMSO): δ = 1.01 (dd, $CH_3$), 1.14 (d, J = 7.0, $CH_3$), 2.44, 2.47, 2.60, 3.53 (4 m, CH and $H_{\alpha,\beta}$-C(2')), 4.05 (s, $OCH_3$), 4.17 (m, H-C(5')), 4.30 (m, H-C(4')), 5.56 (m, H-C(3')), 6.41 (m, H-C(1')), 9.44 (d, J = 9.1, NH), 10.94 (d, J = 9.8, HCO). Anal. berechnet für $C_{21}H_{26}Cl_2N_4O_7$ (517.4): C 48.75, H 5.07, N 10.83; gefunden: C 49.04, H 5.09, N 10.66.

Beispiel 51:

5-Chlor-6-iod-7-[2-deoxy-3,5-di-O-(2-methylpropionyl)-β-D-erythro-pentofuranosyl]-2-[(formyl)amino]-4-methoxy-7H-pyrrolo-[2,3-d]pyrimidin (50)

Die Titelverbindung (50) erhält man aus dem 5-Chlornucleosid (45) durch Iodierung mit N-Iodsuccinimid.

Beispiel 52:

2-Amino-5,6-dichlor-7-(2-deoxy-β-D-erythro-pentofuranosyl)-3,7-dihydro-4H-pyrrolo-[2,3-d]pyrimidin-4-on (51)

Eine Suspension von Verbindung (49) (200 mg, 0.4 mmol) in 2N aq. NaOH (8 ml) wird für 3 h unter Rückfluß gekocht. Nach Neutralisierung der Lösung mit konz. AcOH wird das Reaktionsprodukt filtriert, mit Wasser gewaschen und getrocknet. Nach Auskristallisation aus $CH_3CN$ erhält man farblose Kristalle (128 mg, 96 %). $^1$H-NMR ([$D_6$]DMSO): δ = 2.22 (m, $H_\alpha$-C(2')), 2.92 (m, $H_\beta$-C(2')), 3.52 (m, H-C(5')). 3.72 (m, H-C(4')), 4.33 (m, H-C(3')), 4.81 (t, 5'-OH), 5.22 (d, 3'-OH), 6.35 (dd, H-C(1')), 6.46 (br., $NH_2$), 10.75 (s, NH).

Beispiel 53:

2-Amino-5-iod-6-chlor-7-(2-deoxy-β-D-erythro-pentofuranosyl)-3,7-dihydro-4H-pyrrolo[2,3-dlpyrimidin-4-on (52)

Titelverbindung (52) wird ausgehend von Verbindung (47) wie unter Beispiel 52 beschrieben hergestellt.

Beispiel 54:

2-Amino-5-brom-6-chlor-7-(2-deoxy-β-D-erythro-pentofuranosyl)-3,7-dihydro-4H-pyrrolo[2,3-d]pyrimidin-4-on (53)

Titelverbindung (53) wird ausgehend von Verbindung (48) wie unter Beispiel 52 beschrieben hergestellt.

Beispiel 55:

2-Amino-5-chlor-6-iod-7-(2-deoxy-β-D-erythro-pentofuranosyl)-3,7-dihydro-4H-pyrrolo[2,3-d]pyrimidin-4-on (54)

Titelverbindung (54) wird ausgehend von Verbindung (50) wie unter Beispiel 52 beschrieben hergestellt.

Beispiel 56:

2-Amino-7-(2-deoxy-β-D-erythro-pentofuranosyl)-5-methyl-3,7-dihydro-4H-pyrrolo[2,3-d]pyrimidine-4-on (55)

Titelverbindung (55) wird nach der in Winkeler et al. (Liebigs Ann. Chem. 1984, 708) beschriebenen Methode hergestellt.

Beispiel 57:

2-Amino-7-(2-deoxy-β-D-erythro-pentofuränosyl)-6-methyl-3,7-dihydro-4H-pyrrolo[2,3-d]pyrimidin-4-on (56)

Titelverbindung (56) wird analog zu Beispiel 55 hergestellt.

Beispiel 58:

2-Amino-7-(2-deoxy-β-D-erythro-pentofuranosyl)-5,6-dimethyl-3,7-dihydro-4H-pyrrolo[2,3-d]pyrimidin-4-on (57)

Titelverbindung (57) wird analog zu Beispiel 55 hergestellt.

Beispiel 59:

2-Amino-7-(2-deoxy-β-D-erythro-pentofuranosyl)-5-iod-3,7-dihydro-4H-pyrrolo-[2,3-d]pyrimidin-4-on (58)

Titelverbindung (58) wird ausgehend von Verbindung (46) (200 mg, 0.35 mmol) analog zu Beispiel 51 hergestellt. Es werden farblose Kristalle (126 mg, 92%) aus MeCN erhalten. Schmelzpunkt: 218 - 220˚C. UV (MeOH): $\lambda_{max}$ 266 (12.000), 285 (sh, 8.400). $^1$H-NMR ([$D_6$]DMSO):δ = 2.08 (m, $H_a$-C(2')), 2.30 (m, $H_\beta$-C(2')), 3.48 (m, H-C(5')), 3.74 (m, H-C(4')), 4.26 (m, H-C(3')), 4.89 (t, 5'-OH), 5.18 (d, 3'-OH), 6.25 (dd, H-C(1')), 6.34 (br., $NH_2$), 7.09 (s, H-C(6)), 10.51 (br., NH). Anal. berechnet für $C_{11}H_{13}IN_4O_4$ (392.2): C 33.69, H 3.34, N 14.29; gefunden: C 33.78, H 3.42, N 14.29.

Beispiel 60:

7-(2-Deoxy-β-D-erythro-pentofuranosyl)-5-iod-2-isobutyrylamino-3,7-dihydro-4H-pyrrolo[2,3-d]pyrimidin-4-on (59)

Nach dreimaligem Trocknen unter Abdampfen von Pyridin wird Verbindung (58) (300 mg, 0.76 mmol; gelöst in 4 ml getrocknetem Pyridin) mit 0.48 ml (3.78 mmol) Trimethylchlorsilan versetzt. Die Lösung wird 15 min gerührt. Nach Zugabe von 0.62 ml (3.78 mmol) Isobutyrilanhydrid wird die Lösung 3 h bei Raumtemperatur stehen gelassen. Nach Abkühlung des Reaktionsgemisches im Eisbad wird 1 ml Wasser hinzugegeben. Nach 5 min wird 1 ml einer 25%igen wässrigen Ammoniaklösung hinzugeben und 15 min gerührt. Die Lösung wird dann fast bis zur Trockenheit eingedampft. Nach Auskristallisation aus Wasser werden farblose Kristalle erhalten (312 mg, 89%). $^1$H-NMR ([$D_6$]DMSO): δ = 1.10 (2 $CH_3$), 2.12 (m, $H_\beta$-C(2')), 2,37 (m, $H_\alpha$-C(2')), 2.73 (m, CH), 3.50 (m, H-C(5')), 3.78 (m, H-C(4')), 4.30 (m, H-C(3')), 4.89 (t, 5'-OH), 5.20 (d, 3'-OH), 6.35 (dd, H-C(l')), 7.43 (s, H-C(6)), 11.49, 11,76 (2s, 2 NH). Anal. berechnet für $C_{15}H_{19}IN_4O_5$ (462.2): C 38.98, H 4.14, N 12.12; gefunden: C 39.11, H 4.37, N 11.96.

Beispiel 61:

7-(2-deoxy-β-D-erythro-pentofuranosyl)-5-methyl-2-isobutyryl-amino-3,7-dihydro-4H-pyrrolo[2,3-d]pyrimidin-4-on (60)

Nach dreimaligem Trocknen unter Abdampfen von Pyridin wird Verbindung (55) (500 mg, 1.78 mmol; gelöst in 9 ml getrocknetem Pyridin) mit 1.2 ml (9 mmol) Trimethylchlorsilan versetzt. Die Lösung wird 15 min gerührt. Nach Zugabe von 1.2 ml (9 mmol) Isobutyrilanhydrid wird die Lösung 3 h bei Raumtemperatur stehen gelassen. Nach Abkühlung des Reaktionsgemisches im Eisbad werden 1.8 ml Wasser hinzugegeben. Nach 5 min werden 1.8 ml einer 25%igen wässrigen Ammoniaklösung hinzugeben und die Reaktion wird für weitere 15 min fortgesetzt. Das Gemisch wird dann fast bis zur Trockenheit eingedampft und in 10 ml Wasser aufgenommen, worauf sehr schnell farblose Kristalle auskristallisieren (555 mg, 89%). Dünnschichtchromatographie (Silicagel, $CH_2Cl_2$/MeOH 9 : 1): $R_f$ = 0.7. $^1$H-NMR [$D_6$-DMSO] 1.10 (d, J = 6.5 Hz, 2$CH_3$-C), 2.11, 2.28 (2m, 2H-C(2')), 2.23 (s, $CH_3$), 2.73 (q, J = 6.6 Hz, CH), 3.48 (m, 2H-C(5')), 3.75 (m, H.C(4')), 4.29 (m, H-C(3')), 4.85 (m, OH-C(5')), 5.20 (m, OH-C83')), 6.36 (t, J = 6.7 Hz, H-C(1')), 6.94 (s, H-C (6)), 11.42, 11.67 (2s, 2NH). Anal. berechnet für $C_{16}H_{22}N_4O_5$ (350.37): C 54.84, H 6.33, N 15.99; gefunden: C 54.76, H 6.46, N 16.01.

Beispiel 62:

7-[2-Deoxy-5-O-(4,4'-dimethoxytrityl)-β-D-erythro-pentofuranosyl]-5-iod-2-isobutyrylamino-3,7-dihydro-4H-pyrrolo [2,3-d]pyrimidin-4-on (61)

Verbindung (59) wird wiederholt durch Abdampfen von wasserfreiem Pyridin getrocknet. 400 mg (0.87 mmol)

der so getrockneten Verbindung (59) werden in 5 ml wasserfreien Pyridin gelöst. Nach Zugabe von 4,4'-Dimethoxytritylchlorid (328 mg, 0.95 mmol) bei Raumtemperatur wird das Reaktionsgemisch übernacht gerührt. Anschließend werden MeOH (3 ml) und 5% aq. $NaHCO_3$ (30 ml) hinzugegeben. Die wässrige Phase wird 3 mal mit $CH_2Cl_2$ extrahiert. Die organische Phase wird getrocknet ($Na_2SO_4$), eingedampft und einer Flashchromatographie unterworfen (Säule: 10 x 5 cm, Lösungsmittel $CH_2Cl_2$/Aceton, 9 : 1). Isolierung des Materials aus der Hauptzone liefert die farblose, amorphe Titelverbindung (61). (600 mg, 91 %). $^1$H-NMR ([$D_6$]DMSO): $\delta$ = 1.13 (m, 4 $CH_3$), 2.24 (m, H-C(2')), 2.77 (m, CH), 3.12 (m, H-C(5')), 3.75 (s, 2 $CH_3O$), 3.93 (m, H-C(4')), 4.35 (m, H-C(3')), 5.30 (d, 3'-OH), 6.39 (dd, H-C(1')), 6.86 - 7.39 (m, aromatic H + H-C(6)), 11.54, 11,82 (2s, 2 NH). Anal. berechnet für $C_{36}H_{37}JN_4O_7$ (764.6): C 56.55, H 4.88, N 7.33: gefunden C 56.42, H 4.82, N 7.30.

Beispiel 63:

7-[2-deoxy-5-O-(4,4-dimethoxytrityl)-β-D-erythro-pentofuanosyl]-5-methyl-2-isobutyryl-amino-3,7-dihydro-4H-pyrrolo [2,3-d]pyrimidin-4-on (62)

Verbindung (60) (390 mg, 1.1 mmol; getrocknet durch Abdampfen aus trockenem Pyridine, suspendiert in 8 ml getrocknetem Pyridin) wird mit $(MeO)_2$TrCl (448 mg, 1.3 mmol) versetzt und für 4 h bei Raumtemperatur gerührt. Nach Zugabe von MeOH (5 ml) wird das Reaktionsgemisch mit 5% aq. $NaHCO_3$ (80 ml) versetzt. Nach Extraktion mit $CH_2Cl_2$ (3 x 50 ml) werden die organischen Phasen kombiniert, getrocknet (wasserfreies $NaSO_4$) und eingedampft. Der verbleibende Rest wird in $CH_2Cl_2$ gelöst und einer Flashchromatographie unterworfen. (Silikagel-Säule: 4 x 8 cm, Lösungsmittel $CH_2Cl_2$/MeOH 95:5 enthaltend Spuren von $Et_3N$). Die Hauptzone wird isoliert und Titelverbindung (62) wird als farbloses Puder erhalten (654 mg, 90 %). Dünnschichtchromatographie (Silikagel, $CH_2Cl_2$/MeOH 95 : 5): $R_f$ = 0.3. $^1$H-NMR ($d_6$-DMSO: 1.10 (d, J = 6.7 Hz, 2$CH_3$-C); 2.16 (s, $CH_3$); 2.20, 2.40 (2m, 2H-C(2')); 2.74 (q, J = 6.8 Hz, CH); 3.12 (m, H-C(5')); 3.72 (s, 2$CH_3O$); 3.89 (m, H-C(4')); 4.34 (m, H-C(3')); 5.30 (d, J = 3.7 Hz, OH-C(3')); 6.38 (t, J = 6.7 Hz, H-C(1')); 6.7 - 7.4 (m, aromatic H and HC(6)); 11.46, 11.71 (2s, 2NH). Anal. berechnet für $C_{37}H_{40}N_4O_5$ (652.72): C 68.08, H 6.18, N 8.58; gefunden: C 68.25, H 6.29, 8.50.

Beispiel 64:

7-[2-Deoxy-5-O-(4,4-dimethoxytrityl)-β-D-erythro-pentofuranosyl]-5-iod-2-isobutyrylamino-3,7-dihydro-4H-pyrrolo [2,3-d]pyrimidin-4-on 3'-triethylammonium-phosphonat (63)

Zu einer Lösung von $PCl_3$ (180 µl, 2 mmol) und N-Methylmorpholin (2.2 ml) in $CH_2Cl_2$ (12 ml) wird 1,2,4-Triazol (480 mg, 6.8 mmol) gegeben. Die Lösung wird 30 min gerührt und dann langsam auf 0°C abgekühlt. 306 mg (0.4 mmol) der Verbindung (61) (gelöst in 12 ml $CH_2Cl_2$) werden langsam hinzugegeben und 30 min bei 0°C gerührt. Danach wird die Mischung in 1 M $(Et_3NH)HCO_3$ (TBK, pH 8.0, 25 ml) gegeben, geschüttelt und die Phasen getrennt. Die wässrige Phase wird mit $CH_2Cl_2$ (3 x 30 ml) extrahiert. Die vereinigten organischen Extrakte werden getrocknet ($Na_2S0_4$) und eingedampft. Nach Chromatographie (Säulen: 10 x 5 cm, Lösungsmittel $CH_2Cl_2/Et_3N$, 98 : 2, dann $CH_2C_2$/MeOH/$Et_3N$ (88 : 10 : 2) erhält man nach Extraktion mit 0.1 M TBK (8 x 20 ml), Trocknen mit $NCl_2SO_4$ und Abdampfen Titelverbindung (63) (320 mg, 86%) als farblosen Schaum. $^1$H-NMR ([$D_6$]DMSO): $\delta$ = 1.15 (m, 5 $CH_3$), 2.36, - 2.37 (m, H-C(2')), 2.76 (m, CH), 2.98 (m, 3 $CH_2$), 3.20 (m, H-C(5')), 3.75 (s, 2 MeO), 4.11 (m, H-C(4')), 4.80 (m, H-C(3')), 6.44 (dd, H-C(1')), 6.09 (s, pH), 6.87 - 7.39 (m, aromatic H + H-C(6)), 11.79 (br., 2 NH). $^{31}$p-NMR ([$D_6$]DMSO): 1.05 ('J(P,H) = 587, $^3$J(p, H-C(3')) = 8.3 Hz.

Beispiel 65:

7-[2-Deoxy-5-O-(4,4-dimethoxytrityl)-$_\beta$-D-erythro-pentofuranosyl]-5-methyl-2-isobutyryl-amino-3,7-dihydro-4H-pyrrolo [2,3-d]pyrimidin-4-on, 3'-triethylammonium phosphonat (64)

Zu einer Lösung von $PC1_3$ (200 µl, 2.26 mmol) und N-Methylmorpholin (2.5 ml) in $CH_2Cl_2$ (14 ml) wird 1,2,4-Triazol (523 mg, 7.3 mmol) gegeben. Die Lösung wird 30 min gerührt und dann langsam auf 0°C abgekühlt. 300 mg (0.46 mmol) der Verbindung (62) (gelöst in 14 ml $CH_2Cl_2$) werden langsam hinzugegeben und 30 min bei Raumtemperatur gerührt. Danach wird die Mischung in 1 M $(Et_3NH)HC0_3$ (30 ml) gegeben, geschüttelt und die Phasen getrennt. Die wässrige Phase wird mit $CH_2Cl_2$ (3 x 40 ml) extrahiert. Die vereinigten organischen Extrakte werden mit wasserfreiem $Na_2SO_4$ getrocknet und eingeengt. Der eingengte Rest wird einer Flashchromatographie unterworfen (Silikagel, Säule 3 x 7 cm, $CH_2Cl_2$/MeOH/$Et_3NH$ 88 : 10 : 2). Die Fraktionen der Hauptzone werden gesammelt, eingedampft, in $CH_2Cl_2$ gelöst und mit 0.1 M $(Et_3N)HCO_3$ (5 x 15 ml) gewaschen. Die organische Phase wird mit wasserfreien $Na_2S0_4$ getrocknet und eingedampft. Man erhalt die Titelverbindung (64) als farblosen Schaum (270 mg, 72 %). Dünnschichtchromatogra-

phie (Silicagel, $CH_2C1l$/MeOH /$Et_3N$ 88 : 10 : 2): $R_f$ = 0.5. $^1$H-NMR ($D_6$-DMSO): 1.16 (m, $5CH_3$); 2.19 (s, $CH_3$); 2.30, (m, H-C(2'1); 2.74 (q, J = 6.3 Hz, CH); 3.00 (q, J 6.4 Hz, $3CH_2$); 3.13, 3.18 (2m, 2H-C(5')); 3.75 (s, $CH_3O$); 4.01 (m, H-C(4')); 4.77 (m, H-C(3')); 6.43 (d, J (P,H) = 346 Hz, PH); 6.45 (t, J = 6.7 Hz H-C(1')); 6.8 - 7.4 (m, aromatic H and H-C (6)); 11.67, 11.69 (2s, 2NH). $^{31}$P-NMR ($d_6$-DMSO): 0.94 ($^1$J (P,H) = 354 Hz, $^3$J (P,H-C(3')) = 8.1 Hz). Anal. berechnet für $C_{43}H_{5S}N_5O_9P$ (817.89): C 63.14, H 6.90, N 8.56; gefunden: C 63.06, H 6.88, N 8.51.

Beispiel 66:

2-Amino-7-(2-deoxy-β-D-erythro-pentofuranosyl)-5-(1-hexinyl)-3,7-dihydro-4H-pyrrolo[2,3-d]pyrimidin-4-on (65)

Zu einer mit Argon durchspülten Lösung von Verbindung (58) (390 mg, 1 mmol in 5 ml getrocknetem DMF) werden Kupferiodid (38.1 mg, 0.2 mmol), Triethylamin (2.8 ml, 2 mmol), Tetrakis(triphenylphosphin)palladium(0) (40.5 mg, 0.1 mmol) und 1-Hexin (492 mg, 6 mmol) hinzugegeben und 24 h bei Raumtemperatur gerührt. Anschließend wird die Lösung eingedampft und auf eine Silicagelsäule (5 x 25 cm) gegeben. Nach schrittweiser Elution mit 5 %, 10 % und 20 % MeOH in $CH_2Cl_2$ erhält man Titelverbindung (65). Umkristallisation aus MeCN ergibt einen farblosen Feststoff (120 mg, 35 %). $^1$H-NMR ([$D_6$]DMSO): δ = 0.94 (m, $CH_3$), 1.49, 2.38 (m, $CH_2$). 2.0 (M, $H_\alpha$-C(2')), 2.35 (m, $H_\beta$-C(2')), 3.50 (m, $H_2$-C(5')), 3.76 (m, H-C(4')), 4. (m, H-C(3')), 4.88 (t, 5'-OH), 5.18 (d, J = 3.5, 3'-OH), 6.27 (m, H-C(1') + $NH_2$), 7.13 (s, H-C(6)), 10.34 (br., NH). MS: m/e 346 ($M^+$).

Beispiel 67:

Festphasensynthese der Oligodesoxyribonucleotide nach der Phosphonat-Methode.

Die Oligodesoxyribonucleotidsynthesen wurden im 1 μmol Maßstab mittels der Phosphonat-Technik an einem automatisierten DNA-Synthesizer Modell 380 B (Applied Biosystems, Weiterstadt) an fester Phase (CPG: ®Controlled Pore Glass), wobei das DNA-Fragment in 3'-5'-Richtung synthetisiert wurde, durchgeführt. Der Reaktionsszyklus (Detritylierung, Kupplung, Cappen und Oxidation) folgte dabei einem für die Phosphonat-Chemie entwickeltem Programm [H. Köster, K. Kulikowsky, T. Liese, W. Heikens, V. Kohli, Tetrahedron 1981, 37, 363.]. Das basen- und an der 5'-Hydroxylgruppe Dmt geschützte Oligonucleotid wurde durch 25% wässerigem Ammoniak innerhalb von 30 min vom Träger abgespalten. Die Schutzgruppen an den Heterozyklen wurden im gleichen Medium innerhalb von 48h bei 60˚C entfernt. Die Proben wurden unter Zugabe eines Tropfens Triethylamin (Vermeidung der vorzeitigen Abspaltung der 5'-OH Schutzgruppe) in einem Speed-Vac ®Konzentrator auf etwa 200 μl eingengt. Sie sind so bei -25˚C einige Monate haltbar.

Beispiel 68:

Festphasensynthese der Oligodesoxyribonucleotide nach der Phosphoramidit-Methode.

Die Oligodesoxyribonucleotidsynthesen wurden im 1 μmol Maßstab mittels der Festphasen-Phosphoramidit-Technik an einem automatisierten DNA-Synthesizer Modell 380 B (Applied Biosystems, Weiterstadt) mit Hilfe von ®CPG (Controlled Pore Glass) oder ®Fractosil, das die erste Nucleosid-Einheit über das 3'-Ende gebunden hält, durchgeführt. Dabei wurden folgende Schritte durchgeführt:

1. Waschen mit Acetonitril abs.
2. Behandeln mit 3 % Trichloressigsäure in Dichlormethan
3. Waschen mit Acetonitril abs.
4. Kondensation mit 10 μmol 5'-O-Dimethoxytritynucleosid-3'-phosphorigsäure-β-cyanoethylest er-diisopropylamidit und 50 μmol Tetrazol in 0.3 ml Acetonitril abs.
5. Waschen mit Acetonitril
6. Capping mit 20 % Acetanhydrid in THF mit 40% Lutidin und 10 % Dimethylaminopyridin
7. Waschen mit Acetonitril
8. Oxidation mit Jod (1.3 gr in THF/Wasser/Pyridin; 70:20:5 =v:v:v)

Die Schritte 1 bis 8 , nachfolgend ein DNA-Reaktionszyklus genannt, wurden zum Aufbau des Oligonucleotids entsprechend der zu synthetisierenden Sequenz wiederholt, wobei in Schritt 4 jeweils das der Sequenz entsprechende 5'-O-Dimethoxytrityl(nucleobase)-3'-phosphorigsäure-β-cyanoethylester-diisopropylamidit eingesetzt wurde. Nach abgeschlossener Synthese erfolgt die Aufarbeitung wie in Beispiel 67 beschrieben.

Beispiel 69:

HPLC-Aufreinigung der tritylgeschützten sowie entschützten Oligonucleotide

Im ersten Reinigungsschritt wurden die DMT-geschützten Oligomere über HPLC an RP-18-Kieselgel aufgereinigt (Laufmittelsystem I, s.u.), bis zur Trockene eingedampft, mehrmals mit trockenem Methanol nachgedampft und anschließend unter Eiskühlung durch 10-minütiges Einwirken von 80%-iger Essigsäure detrityliert. Daraufhin wurde die Säure tropfenweise bei 0˚C mit Triethylamin (1-2 ml) neutralisiert, bis fast zur Trockene eingeengt und zweimal mit absolutem Methanol nachgedampft. Nach Aufnahme des Rückstands in 500 µl bidestilliertem Wasser wurden die vollständig entschützten Oligonucleotide erneut über RP-18-Kieselgel per HPLC aufgereinigt (Laufmittelsystem II, s.u.). Die vereinigten Hauptzonen wurden abgedampft, der Rückstand in 500 µl bidestilliertem Wasser gelöst und über eine kurze RP-18-Säule entsalzt (Laufmittelsystem III, s.u.). Nach Lyophilisierung im Speed-Vac-Konzentrator wurden die Oligonucleotide in 100 µl bidestilliertem Wasser aufgenommen und bei -25˚C gelagert.
Es wurden folgende HPLC-Laufmittel verwendet:

A: 0.1 M Triethylammoniumacetat pH 7.0/ 5%-ig an Acetonitril
B: bidestilliertes Wasser
C: Acetonitril
D: Methanol/ Wasser 3:2

Folgende Gradienten-Systeme bestehend aus den obigen Laufmitteln wurden eingesetzt:

I: 3 min. 15% C in A, 7 min. 15-40% C in A, 5 min. 40% C in A, 5 min. 40-15%C in A
II: 20 min. 0-20% C in A, 5 min. 20% C in A
III: 100% A
IV: 30 min. B, 10 min. D
V: 12 min. 100% A, 8 min. 0-40% C in A, 5 min. 40-0% C in A

Folgende Retentionszeiten der Oligomere wurden beobachtet:

HPLC-Retentionszeiten der synthetisierten Oligonucleotide:

| Oligomer (5'→3'-Richtung) | Retentionszeiten [min] | |
|---|---|---|
| | mit Trityl | ohne Trityl |
| d($A_{12}$) | 11.6 | 15.5 |
| d($T_{12}$) | 11.5 | 13.7 |
| d($c^7A_{11}A$) | 12.3 | 15.3 |
| d($Br^7c^7A^{11}A$) | 12.7 | 19.1 |
| d($Me^7c^7A_{11}A$) | 12.2 | 18.1 |
| d(A-T)$_6$ | 13.5 | 20.1 |
| d($c^7$A-T)$_6$ | 13.6 | 20.5 |
| d($Cl^7C^7$A-T)$_6$ | 12.8 | 19.9 |
| d($Br^7C^7$A-T)$_6$ | 12.3 | 17.8 |
| d($Me^7C^7$A-T)$_6$ | 12.6 | 17.9 |

Beispiel 70:

Charakterisierung der Oligodesoxyribonukleotide durch enzymatische Hydrolyse.

Die Oligonucleotide (je 0.5 $A_{260}$-Einheiten) werden in 0.1 M Tris-HCl-Puffer (pH = 8.3, 200 µl) gelöst und mit Schlangengift-Phosphodiesterase (3 µg) 45 min bei 37˚C inkubiert. Anschließend wird mit alkalischer Phosphatase (3 µg) versetzt und weitere 30 min die Temperatur bei 37˚C gehalten. Die entstehende Nucleosid-Mischung wird mit Hilfe der Reversed-Phase-HPLC (Laufmittelsystem V) UV-spektrophotometrisch analysiert. Basierend auf den Peakflächen

und den Extinktionskoeffizienten der Nucleoside bei 260 nm (dA: 15400, dC: 7300, dG: 11700, dT: 8800, Brdc$^7$A: 5300, Medc$^7$A: 4900, Cldc$^7$A: 6300) kann die Nucleosidzusammensetzung des entsprechenden Oligonucleotids quantifiziert werden.

Beispiel 71:

Bestimmung der Spalthypochromizität durch enzymatische Hydrolyse der Oligonucleotide

0.2 $A_{260}$-Einheiten des Oligonucleotids werden in 0.1 M Tris-HCl-Puffer (pH = 8.3, 200 µl) mit Schlangengift-Phosphodiesterase hydrolysiert. Aus der UV-Absorption bei 260 nm vor und nach der Spaltung läßt sich die Hypochromizität in % unter Berücksichtigung der Enzymabsorption nach folgender Gleichung berechnen:

$$H_{enzym} = [(\epsilon_{monomer} - \epsilon_{polymer})\bullet(\epsilon_{monomer})^{-1}]\bullet 100\%$$

Beispiel 72:

UV- und CD-spektroskopische Bestimmung der $T_m$-Werte und Berechnung der thermodynamischen Daten.

Die Ermittlung der $T_m$-Werte der Oligomere erfolgte an einem Cary 1 UV-Vis Spektrophotometer (Varian, Melbourne, Australien). Die Temperatur wurde mit 0.5°C bzw. 1.0°C pro Minute linear variiert. Zur Untersuchung der Schmelztemperatur wurden Oligomerkonzentrationen zwischen 0.2-0.8 $A_{260}$-Einheiten in 1 ml 60 mM Natrium-Cacodylat-Puffer (pH 7.5, 1 M NaCl, 100 mM $MgCl_2$) verwendet. Die Einzelstrang-Konzentration betrug bei den Experimenten an den nicht selbstkomplementären Oligonucleotiden 0.2-0.6 OD. Die Schmelzhypochromizität in % ergibt sich aus der Absorptionsänderung vor und nach dem Aufschmelzen nach folgender Gleichung:

$$H_{Schm.} = [(A_e - A_t)A_e^{-1}] \times 100$$

Die Analyse der Schmelzkurven erfolgte mit einem Programm ausgehend von einem Zweizustandsmodell ("stacked/unstacked") entsprechend der Gleichung:

$$\ln K = \ln [(E^S - E)/(E^U - E)] = S/R - H/RT$$

mit E = Absorption bei der entsprechenden Wellenlänge, S = "stacked" und U = "unstacked"). Die temperaturabhängigen CD-Spektren wurden an einem Jasco 600 Spektropolarimeter mit temperierbarer Quarz-Küvette in einem Wellenlängenbereich von 200-350 nm aufgenommen. Die Temperaturerhöhung wurde in Intervallen von 5-10°C in einem Bereich von 5 - 80°C in Konzentrationen von 3 - 15 µm in 60 mM Na-Cacodylat-Puffer sowie bei 0.1 M, 1 und 4 M NaCl durchgeführt .

Beispiel 73:

$T_m$-Werte der Oligonucleotide $^{\alpha}$

| Oligomer | $T_m$ [°C] |
|---|---|
| d(A12) • d($T_{12}$) | $44^{b,f}$ |
| d($c^7A_{11}A$) • d($T_{12}$) | $30^{b,e}$ |
| d($Br^7c^7A_{11}A$) • d($T_{12}$) | $53^{b,e}$ |
| d($Me^7c^7A_{11}A$) • d($T_{12}$) | $48^{b,e}$ |

(fortgesetzt)

| Oligomer | $T_m$ [˚C] |
|---|---|
| $[d(A\text{-}T)_6]_2$ | 33[c,f] |
| $[d(c^7A\text{-}T)_6]_2$ | 36[c,e] |
| $[d(Br^7c^7A\text{-}T)_6]_2$ | 55[c,e] |
| $[d(Cl^7c^7A\text{-}T)_6]_2$ | 59[c,e] |
| $[d(Me^7c^7A\text{-}T)_6]_2$ | 41[c,e] |
| $[d(Hexinyl^7c^7A\text{-}T)_6]_2$ | 50[d] |
| $[d(I^7c^7A\text{-}T)_6]_2$ | 60[d,e] |
| $(d(G\text{-}C)_4]_2$ | 60[d,f] |
| $[d(c^7G\text{-}C)_4]_2$ | 53[d,e] |
| $[d(Me^7c^7G\text{-}C)_4]_2$ | 58[d,e] |
| $(d(I^7c^7G\text{-}C)_4]_2$ | 70[d,e] |

[a]) bestimmt in 1 M NaCl mit 60mM Na-Cacodylat, 100 mM $MgCl_2$ pH 7.1
[b]) Oligomerkonzentration: 7.5 µM Einzelstrang,
[c]) Oligomerkonzentration: 15 µM Einzelstrang
[d]) Oligomerkonzentration: 10 µM Einzelstrang
[e]) nicht erfindungsgemäß, Vergleich
[f]) Referenzoligonucleotid

Beispiel 74:

Phosphodiester-Hydrolyse von selbstkomplementären Oligonucleotide mit der Endodesoxyribonuclease EcoRI

0.5 $A_{260}$-Einheiten des entsprechenden Oligonucleotids werden in 100 µl Puffer (bestehend aus 50 µM Tris-HCl-Puffer pH 7.5, 100 mM NaCl, 10 mM Magnesiumchlorid und 1 mM Dithioerythrol) gelöst und mit Endodesoxyribonuclease *Eco*RI (high concentration, 5 µl ≡ 250 Einheiten) versetzt. Anschließend wurde bei 37˚C inkubiert und in Zeitabständen von 30 min. Proben von jeweils 10 µl Volumen entnommen und mit Hilfe der Reverse-Phase-HPLC analysiert (Laufmittelsystem II).

Beispiel 75:

Überprüfung auf Nuclease-Stabilität

10 nmol des zu untersuchenden Oligonucleotids werden in 450 µl 20%-igem fötalem Kälberserum in RPMI-Medium und 50 µl bidestilliertem Wasser gelöst und bei 37˚C inkubiert. Dann werden sofort und nach 1, 2, 4, 7 und 24 Stunden 10 µl Proben für die Gelelektrophorese bzw. 20 µl Proben für die HPLC entnommen, zum Abbruch der Reaktion mit 5 µl bzw. 10 µl Formamid versetzt und 5 Minuten auf 95˚C erhitzt. Für die Gelelektrophorese werden die Proben auf ein 15% Polyacrylamid-Gel (2% BIS) aufgetragen und dieses bei etwa 3000 Voltstunden entwickelt. Die Banden werden durch die Silberfärbung sichtbar gemacht. Zur HPLC-Analyse werden die Proben auf eine Gen-Pak Fax HPLC Säule (Waters/Millipore) gespritzt und bei 1 ml/min mit 5 bis 50 % Puffer A in B chromatographiert (Puffer A: 10mM Natrium-dihydrogenphosphat, 0,1 M NaCl in Acetonitril/Wasser 1:4 (v:v) pH 6,8; Puffer B: wie A, jedoch 1,5 M NaCl).

Beispiel 76:

Testung auf antivirale Aktivität:

Die antivirale Aktivität der Prüfsubstanzen gegen verschiedene humanpathogene Herpesviren wird im Zellkulturtestsystem untersucht. Für den Versuch werden Affennierenzellen (Vero, $2x10^5$/ml) in serumhaltigem Dulbecco's MEM (5 % Fötales Kälberserum FCS) in 96-Napf-Mikrotiterplatten ausgesät und 24 h bei 37˚C und 5 % $CO_2$ inkubiert. Das serumhaltige Medium wird dann abgesaugt und die Zellen werden zweimal mit serumfreiem Dulbecco's MEM (-FCS) überspült. Die Testsubstanzen werden in $H_2O$ auf eine Konzentration von 600 μM vorverdünnt und bei -18˚C aufbewahrt. Für den Test erfolgen weitere Verdünnungsschritte in Dulbecco's Minimal Essential Medium (MEM). Je 100 μl der einzelnen Prüfsubstanzverdünnungen werden zusammen mit 100 μl) serumfreiem Dulbecco's MEM (-FCS) zu den gespülten Zellen gegeben. Nach 3 h Inkubation bei 37˚C und 5% $CO_2$ werden die Zellen mit Herpes simplex Virus Typ 1 (ATCC VR733, HSV-1 F-strain) oder mit Herpes simplex Virus Typ 2 (ATCC VR734, HSV-2 G-Strain) infiziert in Konzentrationen, bei denen der Zellrasen innerhalb von 3 Tagen vollkommen zerstört wird. Bei HSV-1 beträgt die Infektionsstärke 500 Plaque-bildende Einheiten (PFU) pro Napf, bei HSV-2 350 PFU/Napf. Die Versuchsansätze enthalten dann Prüfsubstanz in Konzentrationen von 80 μm bis 0,04 μM in MEM, ergänzt durch 100 U/ml Penicillin G und 100 mg/l Streptomycin. Alle Versuche werden als Doppelbestimmung durchgeführt mit Ausnahme der Kontrollen, die achtfach je Platte durchgeführt werden. Die Versuchsansätze werden 17 h bei 37˚C und 5 % $CO_2$ inkubiert. Die Cytotoxizität der Prüfsubstanzen wird nach 20 h Gesamtinkubationszeit durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft. Es erfolgt daraufhin die Zugabe von FCS auf eine Endkonzentration von 4 % mit weiterer Inkubation für 55 h bei 37˚C und 5% $CO_2$. Die unbehandelten Infektionskontrollen zeigen dann einen kompletten cytopathischen Effekt (CPE). Nach mikroskopischer Begutachtung der Zellkulturen werden diese dann mit Neutralrot entsprechend dem Vitalfärbungsverfahren nach Finter (1966) angefärbt. Die antivirale Aktivität einer Testsubstanz wird definiert als minimale Hemmkonzentration (MHK), die benötigt wird, um 30-60 % der Zellen vor dem virusbedingten cytopathogenen Effekt zu schützen.

Abkürzungen:

| | |
|---|---|
| Bz | Benzoyl |
| br. | breit |
| CD | Circulardichroismus |
| d | Duplett |
| DC | Dünnschichtchromatographie |
| dG | 2'-Desoxyguanosin |
| dA | 2'-Desoxyadenosin |
| dC | 2'-Desoxycytidin |
| dT | 2'-Desoxythymidin |
| $(D_6)$DMSO | Dimethylsulfoxid, 6-fach deuteriert |
| DMF | Dimethylformamid |
| DNA | Desoxyribonucleinsäure |
| Dmt | 4,4'-Dimethoxytrityl, (4,4'-Dimethoxytriphenylmethyl) |
| EDTA | Ethylendiamintetraacetat |
| EtOAc | Ethylacetat |
| $Et_3N$ | Triethylamin |
| FC | Flashchromatographie |
| G | Freie Enthalpie |

| | |
|---|---|
| Gef. | gefunden |
| getr. | getrocknet |
| h | Stunde |
| H | Enthalpie der Duplexbildung |
| HPLC | Hochdruckflüssigkeitschromatographie |
| Hyp. | Hypochromizität |
| ibu | Isobuturyl |
| J | Kopplungskonstante |
| $K_m$ | Michaelis-Menten-Konstante |
| NMR | Kernmagnetische Resonanz |
| PAGE | Polyacrylamidgelelektrophorese |
| PCR | Polymerase chain reaction |
| ppm | parts per million |
| 2-PrOH | Isopropanol |
| $R_f$ | Retention bei der DC relativ zur Laufmittelfront |
| RNA | Ribonucleinsäure |
| RP | Reverse-Phase |
| s | Singulett |
| S | Entropie der Duplexbildung |
| Smp. | Schmelzpunkt |
| SVPD | Schlangengift-Phosphodiesterase |
| t | Triplett |
| TBK | Triethylammoniumbicarbonat |
| $T_m$ | Schmelztemperatur bei Oligomeren |
| UV | Ultraviolett |
| $v_{max}$ | maximale Reaktionsgeschwindigkeit |
| $c^7A$ | 7-Desazaadenosin |
| $Br^7C^7A$ | 7-Brom-7-Desazaadenosin |
| $Cl^7c^7A$ | 7-Chlor-7-Desazaadenosin |

| | |
|---|---|
| $I^7c^7A$ | 7-Iod-7-Desazaadenosin |
| $Me^7c^7A$ | 7-Methyl-7-Desazaadenosin |
| $c^7G$ | 7-Desazaguanosin |
| $I^7c^7G$ | 7-Iod-7-Desazaguanosin |
| $Me^7C^7$ | 7-Methyl-7-Desazaguanosin |
| λ | Wellenlänge |
| ε | molarer Extinktionskoeffizient |

**Patentansprüche**

**1.** Oligonucleotid der Formel I

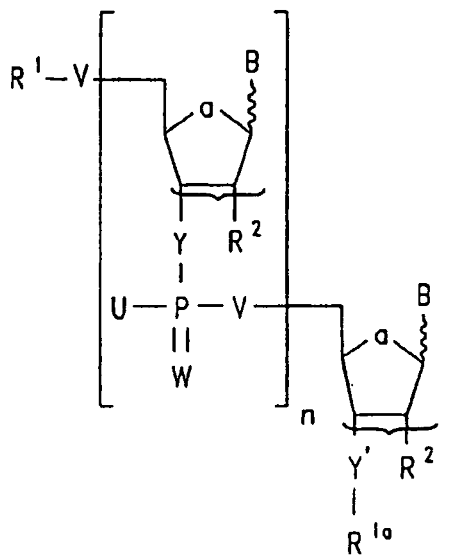

(I)

sowie dessen physiologisch verträglichen Salze worin

B unabhängig voneinander eine in der Nukleotidchemie übliche Base bedeutet und mindestens ein B eine Base der Formel II bedeutet

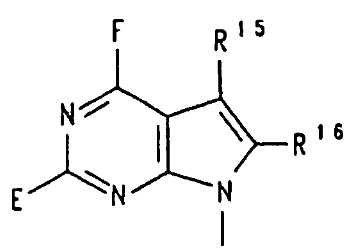

(II)

worin $R^{16}$ Wasserstoff bedeutet und $R^{15}$ $(C_2-C_{10})$-Alkinyl,
bedeutet. E=H oder $NH_2$ und F=$NH_2$ oder OH bedeutet, wobei wenn E=H ist, F=$NH_2$ bedeutet und wenn E=NH2 ist, F=OH bedeutet;

$R^1$ Wasserstoff, einen Rest der Formel IIIa

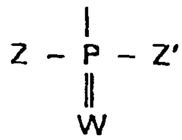

(IIIa)

bedeutet;

$R^{1a}$ Wasserstoff, oder einen Rest der Formel IIIb

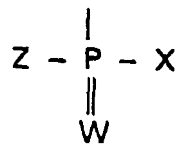

(IIIb)

bedeutet;

$R^2$ Wasserstoff, Hydroxy, bedeutet;

a für Oxy, steht;

n eine ganze Zahl ≥ 1 bedeutet;

W Oxo, bedeutet;

V Oxy, bedeutet;

Y Oxy, bedeutet;

Y' Oxy, Sulfandiyl, ist,

X Hydroxy bedeutet;

U Hydroxy bedeutet,

Z und Z' Hydroxy, bedeuten,

2. Verfahren zur Herstellung der Oligonucleotide der Formel I sowie dessen physiologisch verträglichen Salze Anspruch 1, **dadurch gekennzeichnet, daß** man sukzessive ein Nucleotid mit jeweils einer Nucleobase an einen entsprechenden derivatisierten Träger oder an eine wachsende Oligomerkette ankondensiert.

3. Verwendung der Oligonucleotide nach Anspruch 1 zur Herstellung eines Arzneimittels oder Diagnostikums.

4. Verfahren zur Herstellung eines Arzneimittels oder Diagnostikums, **dadurch gekennzeichnet, daß** mindestens ein Oligonucleotid nach Anspruch 1 mit einem physiologisch annehmbaren Träger sowie gegebenenfalls geeigneten Zusatzstoffen und/oder Hilfsstoffen vermischt wird.

5. Arzneimittel oder Diagostikum, enthaltend eine oder mehrere der Verbindungen nach Anspruch 1 gegebenenfalls zusammen mit physiologisch annehmbaren Träger und/oder Hilfsstoffen.

**Claims**

1. Oligonucleotide of formula I

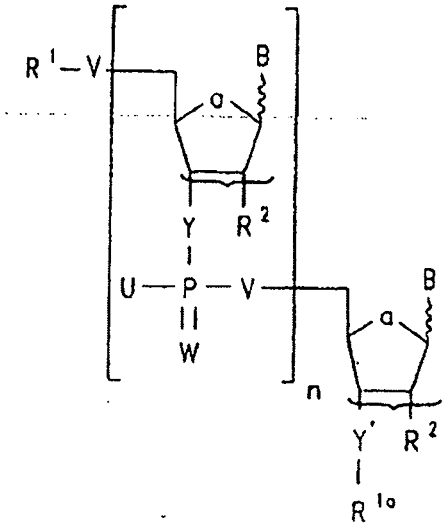

(I)

as well as physiologically acceptable salts thereof, wherein

B independently of one another denotes a usual base in nucleotide chemistry and at least one B denotes a base of formula II

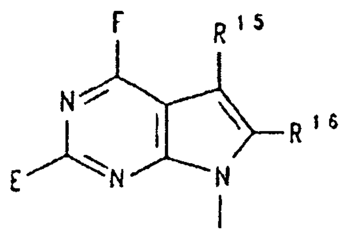

(II)

wherein

$R^{16}$ denotes hydrogen and
$R^{15}$ denotes $(C_2-C_{10})$ alkinyl,
E = H or $NH_2$ and F = $NH_2$ or OH, and if E = H, F denotes $NH_2$ and if E = $NH_2$, F denotes OH;
$R^1$ denotes hydrogen or a residue of formula IIIa

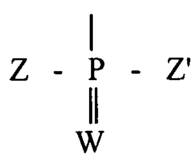

(IIIa)

$R^{1a}$ denotes hydrogen or a residue of formula IIIb

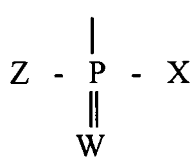

(IIIb)

$R^2$ denotes hydrogen, hydroxy,
a represents oxy,
n denotes an integer ≥ 1,
w denotes oxo,
V denotes oxy,
Y denotes oxy,
Y' denotes oxy, sulfanediyl
X denotes hydroxy,
U denotes hydroxy
Z and Z' denote hydroxy.

**2.** Process for the production of the oligonucleotides of formula I as well as physiologically acceptable salts thereof according to claim 1, **characterized in that** a nucleotide with in each case one nucleobase is successively condensed onto an appropriately derivatized support or onto a growing oligomer chain.

**3.** Use of the oligonucleotides according to claim 1 for producing a pharmaceutical preparation or diagnostic agent.

**4.** Process for the production of a pharmaceutical preparation or diagnostic agent, **characterized in that** at least one oligonucleotide according to claim 1 is mixed with a physiologically acceptable carrier as well as optionally with suitable additives and/or auxiliary substances.

**5.** Pharmaceutical preparation or diagnostic agent containing one or more of the compounds according to claim 1 optionally together with physiologically acceptable carriers and/or auxiliary substances.

**Revendications**

**1.** Oligonucléotide répondant à la formule I

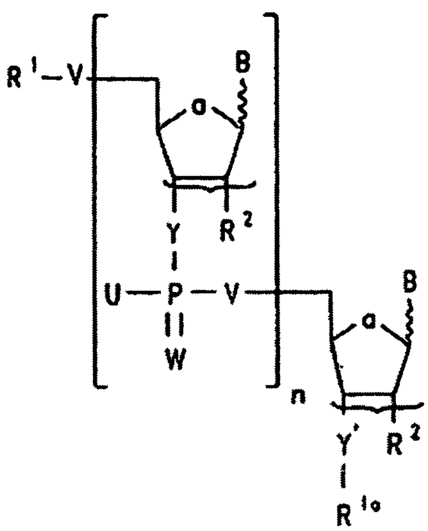

(I)

ainsi que ses sels physiologiquement acceptables, dans lequel

B représente, de manière respectivement indépendante, une des bases habituelles dans la chimie des nucléotides, et au moins un B représente une base répondant à la formule II

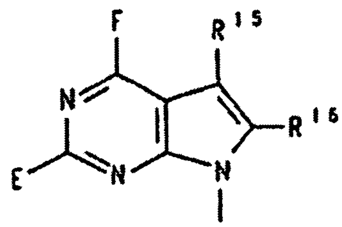

(II)

dans laquelle

$R^{16}$ représente un atome d'hydrogène et un groupe alcynyle en $C_2$-$C_{10}$:
E représente un atome d'hydrogène ou un groupe $NH_2$; et
F représente un groupe $NH_2$ ou un groupe OH,
dans laquelle, lorsque E représente un atome d'hydrogène, F représente un groupe $NH_2$, et lorsque E représente un groupe $NH_2$, F représente un groupe OH ;
$R^1$ représente un atome d'hydrogène ou un radical répondant à la formule IIIa

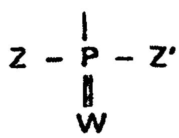

(IIIa)

$R^{1a}$ représente un atome d'hydrogène ou un radical répondant à la formule IIIb

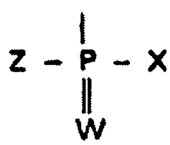

(IIIb)

$R^2$ représente un atome d'hydrogène, un groupe hydroxyle;
a représente un groupe oxy ;
n représente un nombre entier ≥ 1 ;
W représente un groupe oxo ;
V représente un groupe oxy ;
Y représente un groupe oxy ;
Y' représente un groupe oxy, un groupe sulfanediyle ;
X représente un groupe hydroxyle ;
U représente un groupe hydroxyle ;
Z et Z' représentent un groupe hydroxyle ;

2. Procédé pour la préparation des oligonucléotides répondant à la formule I et de leurs sels physiologiquement acceptables selon la revendication 1, **caractérisé en ce qu'**on soumet, de manière successive, un nucléotide à une condensation avec respectivement une base nucléique sur un support dérivé correspondant ou sur une chaîne oligomère en train de croître.

3. Utilisation des oligonucléotides selon la revendication 1, pour la préparation d'un médicament ou d'un agent diagnostique.

4. Procédé pour la préparation d'un médicament ou d'un agent diagnostique, **caractérisé en ce qu'**on mélange au moins un oligonucléotide selon la revendication 1 avec un support physiologiquement acceptable et, de manière facultative, avec des additifs et/ou des substances auxiliaires qui sont appropriés.

**5.** Médicament ou agent diagnostique contenant un ou plusieurs des composés selon la revendication 1, de manière facultative conjointement avec des supports et/ou des substances auxiliaires physiologiquement acceptables.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9310820 A
- DE P4415370
- WO 9309127 A
- WO 9422892 A
- WO 9424144 A
- WO 92002258 A
- DE P4408531
- DE P4408528
- EP 251786 A
- WO 9309217 A
- EP 63879 A
- US 5241060 A
- EP 212536 A
- EP 0552766 A


### In der Beschreibung aufgeführte Nicht-Patentliteratur


- **Uhlmann ; Peyman.** *Chem. Rev.,* 1990, vol. 90, 543
- **Milligan et al.** *J. Med. Chem.,* 1993, vol. 36, 1923
- CRC Handbook of Biochemistry and Molecular Biology. **G.D. Fasman.** Nucleic Acids. 1975, vol. 1, 58-585
- **Chollet et al.** *Nucleic Acid Research,* 1988, vol. 16, 305-317
- **E. N. Kanaya et al.** *Biochemistry,* 1987, vol. 26, 7159
- *Biochemistry,* 1984, vol. 23, 4219
- **Seela et al.** *Nucleic Acid Research,* 1982, vol. 10, 1389
- **J.S. Cohen.** Topics in Molecular and Structural Biology. Macmillan Press, 1989, vol. 12
- **E.Sonveaux.** *Bioorganic Chemistry,* 1986, vol. 14, 274-325
- **S.L. Beaucage et al.** *Tetrahedron,* 1992, vol. 48, 2223-2311
- Remington’s Pharmaceutical Sciences. 1985, 1418
- **M. Koga et al.** *J. Org. Chem.,* 1991, vol. 56, 3757
- **Beaucage et al.** *Tetrah.,* 1993, vol. 49 (10), 1925-1963
- **J. Ludwig et al.** *Acta Biochem. Biophys. Sci. Hung.,* 1981, vol. 16, 131
- **S. Beaucage et al.** *Tetrah.,* 1992, vol. 48 (12), 2223-2311
- *Tetrah.,* 1993, vol. 48 (28), 6123-6194
- **E. Sonveaux.** *Bioorganic Chemistry,* 1986, vol. 14, 274-325
- **S.L. Beaucage et al.** *Tetrathedron,* 1992, vol. 48, 2223-2311
- **Seela et al.** *Helvetica Chimica Acta,* 1994, vol. 77, 897-9031
- **N. Turro et al.** *Tetrahydron Letters,* 1994, vol. 35, 8089
- **L.J. Mc Bride et al.** *Tetrahedron Lett.,* 1983, vol. 24, 2953
- **G.S. Ti et al.** *J. Am. Chem. Soc.,* 1982, vol. 104, 1316
- **H. Schaller et al.** *J. Am. Chem. Soc.,* 1963, vol. 85, 3821
- **C.B. Reese.** *Tetrahedron,* 1978, vol. 34, 3143
- **D. Flockerzi et al.** Liebigs Ann. Chem. 1981, 1568
- **B. C. Froehler et al.** *Nucl. Acid Res.,* 1986, vol. 14, 5399
- **N. D. Sinha et al.** *Nucl. Acid Res.,* 1984, vol. 12, 4539
- **Seela et al.** *Helvetica Chimica Acta,* 1994, vol. 77, 897-903
- **F. Seela ; H. Driller.** *Nucleosides, Nucleotides,* 1989, vol. 8, 1-21
- **F.Seela ; B. Westermann ; U. Bindig.** *J. Chem. Soc. Perkin Trans I,* 1988, 699
- **Watanabe et al.** *Nucleosides & Nucleotides,* 1982, vol. 1 (2), 191-203
- **Nair et al.** *J. Am. Chem. Soc.,* 1989, vol. 111, 8502-8504
- **Winkeler et al.** *Liebigs Ann. Chem.,* 1984, 708
- **H. Köster ; K. Kulikowsky ; T. Liese ; W. Heikens ; V. Kohli.** *Tetrahedron,* 1981, vol. 37, 363